(19) Europäisches Patentamt / European Patent Office / Office européen des brevets

(11) **EP 4 609 885 A1**

(12) **EUROPEAN PATENT APPLICATION**
published in accordance with Art. 153(4) EPC

(43) Date of publication:
03.09.2025 Bulletin 2025/36

(21) Application number: 23893837.7

(22) Date of filing: 21.11.2023

(51) International Patent Classification (IPC):
*A61L 27/36* (2006.01)   *A61L 15/40* (2006.01)
*A61L 17/08* (2006.01)   *A61L 26/00* (2006.01)
*A61L 31/00* (2006.01)   *A61L 27/58* (2006.01)
*A61L 27/60* (2006.01)   *A61L 15/42* (2006.01)
*A61L 27/56* (2006.01)   *A61L 27/50* (2006.01)

(52) Cooperative Patent Classification (CPC):
A61L 15/40; A61L 15/42; A61L 17/08; A61L 26/00;
A61L 27/36; A61L 27/50; A61L 27/56; A61L 27/58;
A61L 27/60; A61L 31/00

(86) International application number:
PCT/CN2023/132986

(87) International publication number:
WO 2024/109747 (30.05.2024 Gazette 2024/22)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 22.11.2022  CN 202211464040
08.12.2022  CN 202211570908
06.07.2023  CN 202310826737

(71) Applicant: Suzhou Microport Regenerative
Medicine
Technology Co. Ltd
Suzhou, Jiangsu 215024 (CN)

(72) Inventors:
• GUO, Jingshu
  Suzhou, Jiangsu 215024 (CN)
• ZHANG, Qiang
  Suzhou, Jiangsu 215024 (CN)
• LUO, Jingwan
  Suzhou, Jiangsu 215024 (CN)
• LIU, Shusen
  Suzhou, Jiangsu 215024 (CN)
• FAN, Dongmei
  Suzhou, Jiangsu 215024 (CN)
• SHI, Lei
  Suzhou, Jiangsu 215024 (CN)

(74) Representative: Arnold & Siedsma
Bezuidenhoutseweg 57
2594 AC The Hague (NL)

(54) **BIOLOGICAL PATCH, PREPARATION METHOD THEREFOR, AND USE THEREOF**

(57) Provided are a biological patch, a preparation method therefor, and use thereof. The biological patch is a fish skin-based biological patch, and contains a first fatty acid and a second fatty acid. A mass percentage of the first fatty acid in the biological patch is greater than or equal to 1.4%, and a mass percentage of the second fatty acid in the biological patch is less than or equal to 2.0%. The first fatty acid includes at least two of butyric acid, caprylic acid, lauric acid, myristic acid, pentadecanoic acid, etc. The second fatty acid includes an $\omega$-6 unsaturated fatty acid. The biological patch includes collagen fibers with a certain diameter, and the biological patch includes micropores with a certain pore size and a certain porosity. The preparation method for the biological patch includes degreasing, pigment removal, and decellularization. The obtained biological patch is used for repairing soft tissue or used as a skin repair dressing. The biological patch can reduce or eliminate inflammatory response of an administration site, and exhibit good mechanical performance and degradation performance.

EP 4 609 885 A1

FIG. 1

**Description**

RELATED APPLICATIONS

The present application claims priority to Chinese patent application No. CN2022114640402, filed on November 22, 2022, and entitled "DECELLULARIZED FISH SKIN MATRIX, PREPARATION METHOD THEREFOR AND USE THEREOF", Chinese patent application No. CN 2022115709087, filed on December 8 , 2022 , and entitled "BIOLOGICAL PATCH, PREPARATION METHOD THEREFOR AND USE THEREOF IN SOFT TISSUE REPAIR", and Chinese patent application No. CN2023108267378, filed on July 6, 2023 , and entitled "FISH SKIN-BASED BIOLOGICAL PATCH, PREPARATION METHOD THEREFOR AND MEDICAL MATERIAL". The contents of the above three Chinese patent applications are hereby incorporated herein in their entireties by reference.

TECHNICAL FIELD

The present application relates to the technical field of biomedical materials, further to the technical field of biomedical repair materials, and more particularly to a biological patch, a preparation method thereof, and use thereof.

BACKGROUND

Soft tissue injuries often result from incidents such as traffic collisions, occupational hazards, athletic activities, and surgical procedures. However, the repair and regeneration of damaged soft tissues still remain clinically challenging. In current clinical practice, biological patches are commonly used as materials for soft tissue replacement and repair.
A biological patch is a novel medical material enabling in-situ regeneration of damaged or lost tissue, prepared based on principles from regenerative medicine, molecular biology, and immunology. Upon implantation in the human body, the biological patch functions as a "cellular scaffold", occupying the damaged site for the lost tissue. Induced by the implanted patch and combined with the body's own repair mechanism, new tissue gradually grows at the implantation site. Concurrently, the biological patch degrades, progressively, achieving regeneration of tissue and organs.
However, clinically available biological patches often trigger inflammatory responses upon implantation, causing significant risks to the host. These inflammatory responses manifest as: 1) excessive local exudation, impairing organ function and compressing adjacent organs, such as alveolar exudate impairing pulmonary gas exchange, pericardial effusion compressing the heart, laryngeal edema inducing asphyxia, etc.; 2) massive plasma exudation, leading to intravascular hypovolemia and subsequent tissue hypoxia; 3) overproduction of inflammatory mediators, causing microcirculatory disturbances and altering coagulation status, leading to tissue damage and disseminated intravascular coagulation; 4) systemic dissemination of tissue degradation byproducts, damaging other organs through blood circulation, resulting in multiple organ failure.
During the body's self-repair process, cytokines, which function as signaling molecules between cells, play critical roles in inflammatory pathologies. Cytokines can be categorized into pro-inflammatory and anti-inflammatory cytokines, which regulate and influence each other, forming a complex network. Their balance facilitates the body in eliminating pathogenic microorganisms and they participate the repair process of the body. Certain pro-inflammatory cytokines (e.g., IL-1, TNF-$\alpha$, IFN-$\gamma$) can activate monocytes/macrophages and neutrophils, upregulate adhesion molecule expression on vascular endothelial cells and leukocytes, increase capillary permeability, enhance the chemotaxis of inflammatory cells, promote extravascular extravasation of leukocyte, strengthen their phagocytic and cytotoxic functions, thereby improving the anti-infection capacity of the body. However, in cases of severe trauma or infection, abundant blood components and leukocytes accumulate at the inflammatory site, activating inflammatory signaling pathways such as NF-$\kappa$B, Jak/Stat in cells and releasing a variety of pro-inflammatory cytokines. Excessive release of pro-inflammatory cytokines and inflammatory mediators induces and amplifies each other, resulting in a cytokine storm.
In addition, biological patches are primarily composed of allogeneic or xenogeneic tissues, and thus carry risks such as immune rejection and viral infections. To mitigate these concerns, decellularization and viral inactivation protocols are often required to remove antigens from the tissues so as to reduce infection risks. However, on the one hand, conventional decellularization methodologies tend to compromise the structural integrity of patches, severely reducing their mechanical performance, which hinders the application of the patches in clinical repair; on the other hand, existing decellularization techniques for biological patches are highly complex, typically requiring strong cross-linking agents to enhance the mechanical performance of the patches, and the residual reagents may easily cause significant cytotoxicity of the material and exacerbate tissue inflammatory responses.
Consequently, mitigating inflammatory responses while improving the mechanical performance and degradation performance of the biological patches used in vivo is a critical technical problem that needs to be addressed promptly.

SUMMARY

In view of the above, an objective of the present disclosure includes providing a biological patch, a preparation method thereof, and use thereof. The inflammatory responses at the administration site induced by the biological patch can be reduced or eliminated, and the biological patch can exhibit good mechanical performance suitable for soft tissue repair. In addition, the biological patch can also exhibit good degradation performance when applied in vivo.

**In a first aspect of the present disclosure,** a biological patch is provided, which can be, but is not limited to, a fish skin-based biological patch.

In some embodiments, the biological patch includes a sheet-shaped body, wherein the sheet-shaped body includes a collagen matrix;

the biological patch contains a first fatty acid and a second fatty acid, wherein the first fatty acid includes at least two selected from butyric acid, caprylic acid, lauric acid, myristic acid, pentadecanoic acid, palmitic acid, stearic acid, arachidic acid, palmitoleic acid, oleic acid, $\gamma$-linolenic acid, linolenic acid ALA, eicosapentaenoic acid EPA, erucic acid, docosahexaenoic acid DHA, and tetracosenoic acid; the second fatty acid includes an $\omega$-6 unsaturated fatty acid; wherein based on a dry weight of the biological patch, a mass percentage of the first fatty acid in the biological patch is greater than or equal to 1.4%, and a mass percentage of the second fatty acid in the biological patch is less than or equal to 2.0%; the dry weight of the biological patch refers to a weight of the biological patch with a water content less than or equal to 5 wt% by mass;
the biological patch includes collagen fibers with a diameter in a range from 2 $\mu$m to 30 $\mu$m; the biological patch includes micropores with a pore size in a range from 2 $\mu$m to 5 $\mu$m; and a porosity of the biological patch is in a range from 55% to 75%.

In some embodiments, the first fatty acid satisfies one or more of the following characteristics:

based on the dry weight of the biological patch, a mass percentage of butyric acid in the biological patch is in a range from 0% to 0.1%, optionally a range from 0.03% to 0.1%;
based on the dry weight of the biological patch, a mass proportion of caprylic acid in the biological patch is in a range from 0 ppm to 40 ppm, optionally in a range from 0 ppm to 20 ppm;
based on the dry weight of the biological patch, a mass proportion of lauric acid in the biological patch is in a range from 0 ppm to 55 ppm, optionally in a range from 20 ppm to 50 ppm;
based on the dry weight of the biological patch, a mass percentage of myristic acid in the biological patch is in a range from 0% to 0.06%, optionally in a range from 0.01% to 0.05%;
based on the dry weight of the biological patch, a mass proportion of pentadecanoic acid in the biological patch is in a range from 0 ppm to 120 ppm, optionally in a range from 0 ppm to 50 ppm, further optionally 0 ppm to 40 ppm;
based on the dry weight of the biological patch, a mass percentage of palmitic acid in the biological patch is in a range from 0% to 1.45%, optionally in a range from 0.28% to 1.45%, further optionally in a range from 0.4% to 0.8%;
based on the dry weight of the biological patch, a mass percentage of stearic acid in the biological patch is in a range from 0% to 0.35%, optionally in a range from 0.05% to 0.35%, further optionally in a range from 0.05% to 0.1%;
based on the dry weight of the biological patch, a mass proportion of arachidic acid in the biological patch is in a range from 0 ppm to 240 ppm, optionally in a range from 30 ppm to 240 ppm, further optionally in a range from 100 ppm to 240 ppm;
based on the dry weight of the biological patch, a mass percentage of palmitoleic acid in the biological patch is in a range from 0% to 0.15%, optionally in a range from 0.1% to 0.15%, further optionally in a range from 0.01% to 0.05%;
based on the dry weight of the biological patch, a mass percentage of oleic acid in the biological patch is in a range from 0% to 5%, optionally in a range from 0.5% to 3%, further optionally in a range from 1% to 2%;
based on the dry weight of the biological patch, a mass percentage of $\gamma$-linolenic acid in the biological patch is in a range from 0% to 0.085%, optionally in a range from 0.01% to 0.085%, further optionally in a range from 0.01% to 0.05%;
based on the dry weight of the biological patch, a mass percentage of linolenic acid ALA in the biological patch is in a range from 0% to 0.28%, optionally in a range from 0.04% to 0.28%, further optionally in a range from 0.04% to 0.1%;
based on the dry weight of the biological patch, a mass proportion of eicosapentaenoic acid EPA in the biological patch is greater than or equal to 80 ppm, optionally greater than or equal to 150 ppm, further optionally greater than or equal to 300 ppm, even further optionally greater than or equal to 400 ppm, or still optionally in a range from 300 ppm to 600 ppm;
based on the dry weight of the biological patch, a mass percentage of erucic acid in the biological patch is in a range from 0% to 0.05%, optionally in a range from 0% to 0.04%, further optionally in a range from 0.01% to 0.04%;
based on the dry weight of the biological patch, a mass percentage of docosahexaenoic acid DHA in the biological patch is in a range from 0% to 0.08%, optionally in a range from 0.01% to 0.07%, further optionally in a range from 0.02% to 0.06%; and

based on the dry weight of the biological patch, a mass proportion of tetracosenoic acid in the biological patch is greater than or equal to 40 ppm, optionally greater than or equal to 70 ppm, further optionally greater than or equal to 80 ppm, even further optionally in a range from 80 ppm to 100 ppm.

In some embodiments, the second fatty acid includes linoleic acid, arachidonic acid, or a combination thereof; the second fatty acid satisfies one or more of the following characteristics:

based on the dry weight of the biological patch, a mass percentage of linoleic acid in the biological patch is in a range from 0% to 2.5%, optionally in a range from 0% to 1.5%, further optionally in a range from 0% to 1%, even further optionally in a range from 0.5% to 1%; and
based on the dry weight of the biological patch, a mass percentage of arachidonic acid in the biological patch is in a range from 0% to 0.090%, optionally in a range from 0% to 0.06%, further optionally in a range from 0.01% to 0.05%.

**In a second aspect of the present disclosure,** a method for preparing a biological patch is provided, which can be used to prepare the biological patch provided in the first aspect of the present disclosure.

In some embodiments, the method for preparing the biological patch includes following steps: performing a degreasing treatment, a pigment removal treatment, and a decellularization treatment on fish skin, and performing a freeze-drying treatment to obtain the biological patch described in the first aspect of the present disclosure; wherein the fish skin has undergone a surface mucus removal treatment and a blood removal treatment;

the degreasing treatment is performed by using a method including following steps: washing the fish skin with first treatment liquids to remove grease impurities from inner and outer surfaces of the fish skin, the washing methods including flushing and rinsing;
the pigment removal treatment is performed by using a method including following steps: soaking the degreased fish skin in a second treatment liquid, taking the degreased fish skin out from the second treatment liquid, removing pigment, and washing the degreased fish skin with a second washing liquid to obtain a depigmented fish skin matrix;
the decellularization treatment is performed by using a method including following steps: soaking the depigmented fish skin matrix in a third treatment liquid, taking the depigmented fish skin matrix out from the third treatment liquid, and washing the depigmented fish skin matrix with a third washing liquid to obtain a decellularized fish skin matrix; wherein:

the first treatment liquids include a first running washing liquid and a second washing liquid, and optionally include a surfactant solution; the running washing liquid is water; the second washing liquid is a sodium carbonate aqueous solution or a starch aqueous solution, wherein a pH value of the sodium carbonate aqueous solution is in a range from 8.4 to 11.5, a mass percentage of starch in the starch aqueous solution is in a range from 1% to 5%; the surfactant solution is a sodium dodecyl sulfate aqueous solution, and a mass percentage of sodium dodecyl sulfate in the sodium dodecyl sulfate aqueous solution is in a range from 0.3% to 0.5%;
the second treatment liquid is an aqueous solution containing a penetration enhancer, or an aqueous solution containing an acidic reagent, or an aqueous solution containing an acidic regulator;
the penetration enhancer includes sodium dodecyl sulfate, and a mass percentage of the penetration enhancer in the aqueous solution containing the penetration enhancer is in a range from 0.1% to 0.5%;
the acidic reagent is one or more selected from acetic acid, citric acid, hydrochloric acid, formic acid, sulfuric acid, and nitric acid, and a concentration of the acidic reagent in the aqueous solution containing the acidic reagent is in a range from 1 mmol/L to 500 mmol/L, a pH value of the acidic solution is in a range from 1 to 5; on a condition that the aqueous solution containing the acidic reagent is an acetic acid aqueous solution, a mass percentage of the acetic acid is in a range from 0.3 wt% to 3 wt%;
the acidic regulator includes citric acid, acetic acid, or a combination thereof, and a mass proportion of the acidic regulator in the aqueous solution containing the acidic regulator is in a range from 0.1% to 0.3%;
the third treatment liquid is a Triton X-100 aqueous solution or an aqueous solution containing an alkaline reagent; the third washing liquid is water;
a mass percentage of Triton X-100 in the Triton X-100 aqueous solution is in a range from 0.1% to 2%;
the alkaline reagent is one or more selected from sodium carbonate, sodium bicarbonate, trisodium phosphate, sodium monohydrogen phosphate, disodium hydrogen phosphate, sodium hydroxide, potassium carbonate, potassium bicarbonate, tripotassium phosphate, potassium monohydrogen phosphate, dipotassium hydrogen phosphate, and potassium hydroxide; the alkaline reagent has a concentration in a range from 0.01 mol/L to 2 mol/L in the aqueous solution containing the alkaline reagent, and a pH value of the alkaline solution is in a range from 9 to 13.

**In a third aspect of the present disclosure,** a method for preparing a single-layer biological patch is provided, including following steps:

performing an acid treatment on fish skin in an acidic solution, removing residual acidic solution, removing pigment, and washing the pigment-removed material to obtain a depigmented tissue material; wherein the fish skin has undergone a blood removal treatment and a degreasing treatment, and the acidic solution is an aqueous solution containing an acidic reagent;

performing an alkali treatment on the depigmented tissue material in an alkaline solution to obtain an alkali-treated material, and washing the alkali-treated material to obtain a decellularized tissue material; wherein the alkaline solution is an aqueous solution containing an alkaline reagent;

pre-freezing the decellularized tissue material and freeze-drying the material under vacuum to obtain the single-layer biological patch.

**In a fourth aspect of the present disclosure,** a fish skin-based biological patch is provided, including a sheet-shaped body, wherein the sheet-shaped body includes a collagen matrix; the fish skin-based biological patch is prepared from fish skin through treatments including depigmentation and decellularization; the fish skin-based biological patch has a degradation period in a range from 7 days (d) to 180 d, a fat content in a range from 1% to 19%, and a residual DNA content less than 50 ng/mg;

optionally, the fish skin-based biological patch is prepared by a method including following steps:

performing a degreasing pretreatment on the fish skin to prepare pretreated fish skin;

placing the pretreated fish skin in an aqueous solution, performing a depigmentation treatment, and optionally a partial decellularization treatment, on the pretreated fish skin to obtain a depigmented fish skin;

performing a decellularization treatment on the depigmented fish skin to obtain the fish skin-based biological patch; wherein in the depigmentation treatment, the aqueous solution is replaced every 1 hour (h) to 4 h, and optionally 2 h to 3 h.

**In a fifth aspect of the present disclosure,** a method for preparing a decellularized fish skin matrix is provided, including:

wetting a decellularized fish skin with a moisture-retaining agent; and

performing a microwave treatment on the wetted fish skin to puff the fish skin; wherein the moisture-retaining agent is vegetable oil; the microwave treatment is performed at a power of 500 watts (W) to 900 W for 5 seconds (s) to 60 s.

**In a sixth aspect of the present disclosure,** a multi-layer biological patch and a method for preparing the multi-layer biological patch are provided.

**In a seventh aspect of the present disclosure,** use of the biological patch described in the first aspect of the present disclosure, or the biological patch prepared by the method described in the second aspect of the present disclosure, or the single-layer biological patch prepared by the method described in the third aspect of the present disclosure, or the fish skin-based biological patch described in the fourth aspect of the present disclosure, or the decellularized fish skin matrix prepared by the method described in the fifth aspect of the present disclosure, in preparation of a soft tissue repair implant material is provided.

In some embodiments, the soft tissue repair for which the soft tissue repair implant material is applicable includes one or more selected from dura mater defect repair, sports tendon tear repair, hernia and abdominal wall defect repair, burn plastic surgery repair, and oral membrane defect repair.

The details of one or more embodiments of the present disclosure are set forth in the following accompanying drawings and description. Other features, objects, and advantages of the present disclosure will become apparent from the specification, accompanying drawings, and claims.

BRIEF DESCRIPTION OF THE DRAWINGS

In order to clearly illustrate the technical solutions in the embodiments and examples of the present disclosure and to provide a more comprehensive understanding of the present disclosure and beneficial effects thereof, the accompanying drawings, which are to be referred to in the description of the embodiments or examples, are briefly described below. Apparently, the drawings in the following descriptions are merely for some embodiments or examples of the present disclosure, and those skilled in the art can obtain other drawings according to the following drawings without any creative work. It should also be noted that the drawings are drawn in a simplified form only to facilitate and clearly assist in the description of the present disclosure. Various sizes of each component shown in the drawings are arbitrarily illustrated, and may be precise or may not be drawn to scale. For example, the sizes of the components may be exaggerated appropriately in some places in the drawings for clarity of illustration. Various components of the drawings are not drawn to scale unless specifically indicated. The present disclosure does not limit the specific sizes of each component.

FIG. 1 is an image showing surface morphology of a biological patch in an embodiment of the present disclosure, obtained through scanning electron microscopy (SEM), with a magnification of 100X.

FIG. 2 is an image showing surface morphology of the biological patch shown in FIG. 1, obtained through SEM, with a magnification of 3000X.

FIG. 3 is a hematoxylin and eosin (H&E) stained image of a decellularized fish skin matrix prepared in an embodiment of the present disclosure.

FIG. 4 is an SEM image of a decellularized fish skin matrix prepared in an embodiment of the present disclosure.

FIG. 5 is a graph showing in vitro degradation of decellularized fish skin matrices prepared in an example and a comparative example of the present disclosure, wherein Series 1 refers to the in vitro degradation of the decellularized fish skin matrix prepared in Comparative Example C1, and Series 2 refers to the in vitro degradation of the decellularized fish skin matrix prepared in Example C1.

FIG. 6 shows H&E stained images of samples of Comparative Example 4 (completely non-degreased), Comparative Example 5 (completely degreased) and Example 5 (partially degreased) observed at Week 2 to Week 4; specifically, a-0 and a-1 respectively correspond to the H&E staining result and its locally enlarged view of Comparative Example 4 at Week 2; b-0 and b1 respectively correspond to the H&E staining result and its locally enlarged view of Comparative Example 5 at Week 2; and c-0 and c1 respectively correspond to the H&E staining result and its locally enlarged view of Example 5 at Week 2.

DETAILED DESCRIPTION

The present disclosure will be described in detail with reference to the accompanying drawings, examples and embodiments. It should be understood that the specific embodiments and examples are only for explaining the present disclosure, and not intended to limit the scope of the present disclosure. The purpose of providing these embodiments and examples is to facilitate a more thorough and comprehensive understanding of the present disclosure. It should also be understood that the present disclosure can be implemented in many different forms and is not limited to the embodiments and examples described herein. Those of ordinary skill in the art can make various changes or modifications to the present disclosure without departing from the concept of the present disclosure, and the equivalent forms obtained also fall within the protection scope of the present disclosure. For example, the technical features illustrated or described as a part of one embodiment can be used in another embodiment in an appropriate manner to form a further embodiment. Additionally, in the following description, numerous specific details are given to facilitate a comprehensive understanding of the present disclosure. However, it should be understood that the present disclosure can be implemented without one or more of these details.

Unless otherwise defined, all technical and scientific terms used herein have the same meaning as commonly understood by those skilled in the art of the present disclosure. The terms used herein in the specification of the present disclosure are used only for the purpose of describing embodiments and examples and are not intended to limit the present disclosure.

In the present disclosure, "plurality", "multiple", "multiple times", etc., unless otherwise specified, refer to a quantity of greater than two or equal to two. For example, "one or more" means a quantity of one or greater than or equal to two.

In the present disclosure, an open-ended description for the technical features includes not only a closed-ended technical solution consisting of the listed features, but also an open-ended technical solution including the listed features.

In the present disclosure, the terms "first", "second", "third", "fourth", etc., such as "first aspect", "second aspect", "third aspect", "fourth aspect", etc., are used for descriptive purposes only and are not to be construed as indicating or implying relative importance or quantity, nor as implicitly specifying the importance or quantity of the technical features indicated. Moreover, "first", "second", "third", "fourth" and the like only serve a purpose of non-exhaustive enumeration, and it should be understood that they do not constitute a closed-ended limitation of quantity.

In the present disclosure, for unit of a numerical range, if the unit only appears after the right endpoint, it means that the units of the left endpoint and the right endpoint are the same. For example, 3 to 5 h denotes that the units of both the left endpoint of "3" and the right endpoint of "5" are hours (h). In addition, the units of other parameters such as temperature and size can be understood in a similar way.

In the present disclosure, if there are multiple steps involved in a method flow, unless otherwise specified herein, the execution sequence of the steps is not strictly limited, and the steps can be executed in other orders than described. Moreover, any step can include multiple sub-steps or multiple stages. These sub-steps or stages are not necessarily executed at the same time, but can be executed at different times, and they are not necessarily executed in a sequential order, but can be executed in turn, alternately or simultaneously with some of other steps, or sub-steps or stages of other steps.

All documents mentioned in the present disclosure are incorporated herein by reference as if each document is individually incorporated by reference. Unless there is a conflict with the objects and/or technical solutions of the present disclosure, the referenced documents related to the present disclosure are incorporated in their entireties and for all purposes. When referencing a document in the present disclosure, the definitions of relevant technical features, terms, nouns, phrases, etc. in the document are also referenced. When referencing a document in the present disclosure, the examples and preferred modes of the related technical features that are referenced can also be incorporated herein by reference, but only to the extent that they enable to carry out the present disclosure. It should be understood that when the referenced content conflicts with the description of the present disclosure, the present disclosure shall prevail or the referenced content shall be adaptively modified according to the description of the present disclosure.

In the present disclosure, exemplary descriptions such as "in some embodiments (or examples)" and "in an embodiment (or example)" may encompass but are not limited to the meaning that these solutions may be combined with other solutions in an appropriate manner to form new technical solutions.

In the present disclosure, parts per million (ppm) is a dimensionless unit of proportion. 1 ppm means one part per million. It can also be converted as follows: 1 ppm = 0.0001%, 200 ppm = 0.02%. wt% means mass percentage or weight percentage. In the present disclosure, when referring to mass ratio, mass proportion, mass content, weight ratio, weight proportion, or weight content expressed in percentage form, "%" following the numerical values refers to wt% unless otherwise specified.

Traditional decellularized matrices or biological patches are mostly derived from pig skin, cow skin, or even small intestinal submucosa. After processing, their fat content is often less than 1%, with almost unsaturated fatty acids existent. In some studies, fish skin has been used as a matrix material for decellularized matrices or biological patches, where degreasing with strong alkalis completely removes fat, which can easily damage the fish skin tissue, and, moreover, induce inflammatory responses during use of these biological patches.

The inventors of the present disclosure have found that the fatty acid content of the biological patches is related to the inflammatory responses. When no degreasing is performed, the inflammatory responses induced by the biological patches are severe; for the completely degreased biological patches, the degree of the inflammatory responses is reduced. Studies have found that compared to the completely degreased or non-degreased biological patches, the partially degreased biological patches can inhibit or reduce the occurrence of inflammatory responses.

The inventors of the present disclosure have also found that the surface roughness and water contact angle of the biological patches are related to their degradation performance. Thus, in order to achieve the goal of slowly regenerating defective tissue while slowly degrading the biological patch in the body, the surface roughness and water contact angle of the biological patch can be controlled. Preferably, the roughness of the two surfaces of the biological patch can respectively satisfy Ra $\leq$ 20 $\mu$m (optionally in a range from 8 $\mu$m to 20 $\mu$m) and Ra $\geq$ 25 $\mu$m (optionally in a range from 25 $\mu$m to 40 $\mu$m); and the water contact angle can be controlled in a range from 50° to 90°.

In a first aspect of the present disclosure, a biological patch is provided. The biological patch can reduce or eliminate occurrence or development of inflammatory responses at the administration site of the biological patch. The biological patch exhibits good mechanical performance, making it suitable for soft tissue repair. Additionally, the biological patch exhibits good degradation performance when used in vivo.

The biological patch provided in the present disclosure includes a relatively high content of a first fatty acid and a relatively low content of a second fatty acid. The first fatty acid generally has an anti-inflammatory effect, and the second fatty acid promotes inflammation. In the biological patch, collagen fibers have a certain diameter, forming micropores with a certain size and a certain porosity, which endow the biological patch with good mechanical performance. In addition, the degradation behavior of the biological patch generated upon contact with tissue fluids in the body can be regulated, allowing the biological patch to have an appropriate degradation rate.

In the present disclosure, the provided "biological patch" has a first surface and a second surface that are opposite to each other, and the second surface has a greater surface roughness than the first surface. Taking a fish skin-based biological patch as an example, the first surface is derived from the outer surface of the fish skin that contacts the water environment, while the second surface is derived from the inner surface of the fish skin that contacts the internal tissues of the fish, where the surface roughness of the second surface is greater than the surface roughness of the first surface. In the present disclosure, when applying the biological patch, the rougher surface usually faces the site to be repaired, while the smoother surface faces away from the site to be repaired. More specifically, the second surface with higher roughness is used to contact the site to be repaired of the body, facilitating the recruitment of cells required for tissue repair, nutrient exchange, and the migration, adhesion, and proliferation of cells required for tissue repair. Conversely, the smoother first surface faces away from the site to be repaired, minimizing the ingrowth of fibroblasts and other cells, serving as a barrier.

Unless otherwise specified, the biological patch provided in the present disclosure can be, but is not limited to, a fish skin-based biological patch, wherein the fish skin-based biological patch is prepared using fish skin as a raw material.

Unless otherwise specified, the fish skin is scaleless fish skin or scale-removed fish skin. The fish skin can be selected from, but is not limited to, the group consisting of longsnout catfish skin, catfish skin, eel skin, and rice eel skin. The fish skin can preferably be the scaleless skin of a Chinese longsnout catfish.

In some embodiments, the biological patch contains ≥1.4 wt% of the first fatty acid and ≤2 wt% of the second fatty acid, based on a dry weight of the biological patch. The first fatty acid includes at least two fatty acids with anti-inflammatory effects, such as butyric acid, caprylic acid, lauric acid, myristic acid, pentadecanoic acid, palmitic acid, stearic acid, arachidic acid, palmitoleic acid, oleic acid, γ-linolenic acid, linolenic acid ALA, eicosapentaenoic acid EPA, erucic acid, docosahexaenoic acid DHA, and tetracosenoic acid. The second fatty acid includes an ω-6 unsaturated fatty acid. The biological patch includes collagen fibers of a certain diameter, forming micropores of a certain pore size and a certain porosity.

In some embodiments, a biological patch is provided, including a sheet-shaped body, wherein the sheet-shaped body includes a collagen matrix. Further, the biological patch contains a first fatty acid and a second fatty acid, wherein the first fatty acid includes at least two selected from butyric acid, caprylic acid, lauric acid, myristic acid, pentadecanoic acid, palmitic acid, stearic acid, arachidic acid, palmitoleic acid, oleic acid, γ-linolenic acid, linolenic acid ALA, eicosapentaenoic acid EPA, erucic acid, docosahexaenoic acid DHA, and tetracosenoic acid; the second fatty acid includes an ω-6 unsaturated fatty acid.

Furthermore, the biological patch includes collagen fibers with a diameter in a range from 2 μm to 30 μm; the biological patch includes micropores with a pore size in a range from 2 μm to 5 μm; and a porosity of the biological patch is in a range from 55% to 75%.

In some embodiments, the biological patch provided in the first aspect of the present disclosure is a fish skin-based biological patch.

In the present disclosure, "fatty acid" refers to a type of aliphatic carboxylic acid molecule with a carbon chain and a carboxyl group, unless otherwise specified. The fatty acids can serve as components of cell membranes and can store and provide energy for muscles, the heart, and other organs.

In the present disclosure, "the dry weight of the biological patch" refers to the weight of the biological patch with a water content ≤5 wt%. The water content is calculated as a weight percentage or mass percentage in the biological patch. For a biological patch with a water content greater than 5 wt%, the weight when the water content ≤5 wt% is calculated and used as the dry weight of the biological patch. The phrase "based on the dry weight of the biological patch" means that the biological patch in the dry weight state is used as the basis. For example, "a mass percentage of material A in the biological patch based on the dry weight of the biological patch" can also be expressed as "a mass percentage of material A in the dry weight of the biological patch", indicating, when the biological patch is in the dry weight state (where the water content ≤5 wt%), the percentage of the mass of material A relative to the mass of the biological patch, which numerically equals to the percentage of the weight of material A relative to the weight of the biological patch.

In the present disclosure, unless otherwise specified, the first fatty acid and the second fatty acid are both fatty acid species existing in nature. Unless otherwise specified, butyric acid refers to normal butyric acid, caprylic acid refers to normal caprylic acid (also denoted as n-caprylic acid), pentadecanoic acid refers to normal pentadecanoic acid (also denoted as n-pentadecanoic acid), and tetracosenoic acid, also known as nervonic acid, refers to cis-15-tetracosenoic acid.

In some embodiments, the first fatty acid and the second fatty acid are respectively selected from the group consisting of the following 37 fatty acids, denoted as Group G1: butyric acid, caproic acid, caprylic acid, capric acid, undecanoic acid, lauric acid, tridecanoic acid, myristic acid, myristoleic acid, pentadecanoic acid, pentadecenoic acid, palmitic acid, palmitoleic acid, heptadecanoic acid, heptadecenoic acid, stearic acid, trans-9-octadecenoic acid, oleic acid, trans-linoleic acid, linoleic acid, arachidic acid, γ-linolenic acid, 11-eicosenoic acid, linolenic acid ALA, heneicosanoic acid, 11,14-eicosadienoic acid, behenic acid, cis-8,11,14-eicosatrienoic acid, erucic acid, methyl cis-11,14,17-eicosatrienoic acid, tricosanoic acid, arachidonic acid, cis-13,16-docosadienoic acid, lignoceric acid, eicosapentaenoic acid EPA, cis-15-tetracosenoic acid, and docosahexaenoic acid DHA.

In the present disclosure, unless otherwise specified, the content of each fatty acid in the biological patch is determined according to "GB 5009.168-2016 National Food Safety Standard - Determination of fatty acids in foods", using gas chromatography to measure the contents of different types of fatty acids in the sample. The determination can be performed by using the method of methyl esterification of fatty acids.

**First Fatty Acid**

In the present disclosure, the first fatty acid refers to a type of fatty acid that has a direct or indirect effect of inhibiting inflammatory responses, and generally has a certain anti-inflammatory effect.

In some embodiments, the first fatty acid includes at least two fatty acids with anti-inflammatory effects, such as butyric acid, caprylic acid, lauric acid, myristic acid, pentadecanoic acid, palmitic acid, stearic acid, arachidic acid, palmitoleic acid, oleic acid, γ-linolenic acid, linolenic acid ALA, eicosapentaenoic acid EPA, erucic acid, docosahexaenoic acid DHA, and tetracosenoic acid. In some embodiments, the first fatty acid is selected from the group consisting of butyric acid, lauric acid, myristic acid, palmitic acid, stearic acid, arachidic acid, palmitoleic acid, oleic acid, γ-linolenic acid, linolenic acid ALA, eicosapentaenoic acid EPA, erucic acid, docosahexaenoic acid DHA, and tetracosanoic acid.

Butyric acid is a short-chain saturated fatty acid with four carbon atoms, classified as a weak acid that exists in body fluids as an ionized form with free mobility. About 95% of butyrate is rapidly oxidized in the body to produce adenosine triphosphate (ATP), which provides energy for epithelial cells, thereby significantly reducing epithelial damage and accelerating the repair of histological injuries. Meanwhile, butyric acid can inhibit the release of cytokines and immunological markers, such as interferon-γ, and can also reduce mitochondrial respiratory deficits, organize mitochondrial autophagy, and regulate energy balance as an energy resource to promote tissue regeneration. In addition, butyric acid can also weaken the inflammatory response caused by lipopolysaccharide (LPS) without affecting the activation of NF-κB. Therefore, increasing the content of butyric acid in the biological patch is conducive to inhibiting the occurrence of inflammatory responses.

Caprylic acid is a medium-chain saturated fatty acid with eight carbon atoms. Nutritional studies have shown that caprylic acid can inhibit inflammatory responses through the TLR-4/NF-κB signaling pathway.

Pentadecanoic acid, also known as pentadecylic acid, is a long-chain saturated fatty acid with no carbon-carbon double bonds in the carbon chain. Nutritional studies have shown that pentadecanoic acid is usually used to provide greater energy storage in the form of fatty acid glycerides, which are broken down into fatty acids and glycerol for use by the body during energy deficiency. Pentadecanoic acid also participates in the construction of cell membranes, combining with other lipid molecules to form stable cell membrane structures as a protective barrier for cells. In addition, pentadecanoic acid can also act as a signal molecule in cellular signal transduction for transmitting information inside and outside cells and regulating the activity of intracellular signaling pathways. Therefore, pentadecanoic acid is, in traditional concepts, beneficial to cell growth and tissue regeneration. It has been reported in the literature that pentadecanoic acid can inhibit the levels of pro-inflammatory cytokines.

The inventors unexpectedly discovered during their research that reducing or even eliminating the content of caprylic acid and pentadecanoic acid in the final biological patch product can actually suppress the occurrence of inflammatory responses.

Lauric acid is a medium-chain saturated fatty acid with 12 carbon atoms, which has an inflammation reducing effect.

Myristic acid is a medium-chain saturated fatty acid with 14 carbon atoms, which also exhibits an anti-inflammatory effect.

Palmitic acid is a saturated fatty acid with 16 carbon atoms, which has an anti-inflammatory effect.

Stearic acid is a long-chain saturated fatty acid with 18 carbon atoms. Stearic acid can inhibit the occurrence and development of inflammatory responses and thus has an anti-inflammatory effect.

Arachidic acid is a long-chain saturated fatty acid with 20 carbon atoms. Arachidic acid can effectively inhibit inflammatory responses and reduce the production of inflammatory factors.

Palmitoleic acid, also known as hexadecenoic acid, is a 16-carbon monounsaturated fatty acid with the initial double bond located at the 7th carbon position counting from the terminal methyl group. It has been reported that palmitoleic acid can regulate body metabolism, affect inflammatory markers and PPAR metabolic pathways, and has a therapeutic effect on inflammation.

Oleic acid is a monounsaturated omega-9 fatty acid with 18 carbon atoms. It has been reported that oleic acid can inhibit the formation of free radicals, resist oxidation, protect the human body from damage caused by external stimuli, inhibit inflammatory responses, reduce inflammatory symptoms, relieve pain, and promote skin metabolism.

In the present disclosure, linolenic acid ALA refers to α-linolenic acid, also denoted as ALA. γ-linolenic acid can also be denoted as GLA. α-linolenic acid and γ-linolenic acid are two isomers of octadecatrienoic acid, which differ in the position of the carbon-carbon double bond. Eicosapentaenoic acid EPA can also be denoted as EPA. Docosahexaenoic acid DHA can also be denoted as DHA.

γ-linolenic acid can inhibit the synthesis and release of inflammatory mediators, thereby alleviating inflammatory responses.

Linolenic acid ALA, eicosapentaenoic acid EPA, and docosahexaenoic acid DHA are all ω-3 unsaturated fatty acids.

ω-3 unsaturated fatty acids, also referred to as omega-3 unsaturated fatty acids, are a class of essential polyunsaturated fatty acids with the initial double bond located at the third carbon position counting from the methyl end. It is generally believed that omega-3 unsaturated fatty acids have significant anti-inflammatory effects. For example, α-linolenic acid has an inhibitory effect on inflammatory mediators and cytokines. DHA and EPA can inhibit the production of pro-inflammatory cytokines by inflammatory cells, competitively reduce the production of inflammatory mediators, regulate immune responses, and thus alleviate excessive inflammatory responses. The anti-inflammatory effects of EPA and DHA are manifested in two ways: indirectly hindering the occurrence of inflammation, and being transformed into mediators that directly inhibit inflammation.

Erucic acid is a docosadecenoic acid, also known as docos-13-enoic acid. It has been reported that erucic acid can reduce peroxide levels, reduce tissue damage and inflammatory responses, and has a positive effect on inflammation-related diseases such as rheumatism and inflammatory bowel disease.

Tetracosenoic acid, referring to cis-15-tetracosenoic acid, is a long-chain monounsaturated fatty acid, which has been reported to inhibit inflammatory factors.

**Second Fatty Acid**

In the present disclosure, the second fatty acid refers to a type of fatty acid that has an effect of promoting inflammation occurrence, which can directly or indirectly promote inflammatory responses. For example, the second fatty acid can be either an inflammatory mediator, such as arachidonic acid, or a substance, such as linoleic acid, to produce an inflammatory mediator through metabolization.

In some embodiments, the second fatty acid at least includes an $\omega$-6 unsaturated fatty acid. The second fatty acid can non-restrictively be an $\omega$-6 unsaturated fatty acid.

$\omega$-6 unsaturated fatty acids, also referred to as omega-6 unsaturated fatty acids, have a well-established meaning in the art, representing a class of essential polyunsaturated fatty acids with the initial double bond located at the sixth carbon position counting from the terminal methyl group. Non-limiting examples include linoleic acid, arachidonic acid, etc. $\omega$-6 unsaturated fatty acids have the effect of promoting inflammatory responses.

In some embodiments, the second fatty acid includes arachidonic acid and linoleic acid. In some embodiments, the second fatty acid is a combination of arachidonic acid and linoleic acid.

The biological patch provided in the present disclosure contains the first fatty acid. The inventors of the present disclosure have found that increasing the content of the first fatty acid is beneficial to enhance the anti-inflammatory performance of the biological patch.

Based on any suitable implementations of the present disclosure, in some embodiments, based on the dry weight of the biological patch, a mass percentage of the first fatty acid in the biological patch is $\geq$1.4%, optionally is in a range from 1.4% to 8%, further optionally is in a range from 2% to 5%. Based on the dry weight of the biological patch, the mass percentage of the first fatty acid in the biological patch can also be any one of the following percentages or be within a range between any two of the following percentages: 1.5%, 1.6%, 1.8%, 2%, 2.5%, 3%, 3.5%, 4%, 4.5%, 5%, 5.5%, 6%, 6.5%, 7%, 7.5%, 8%, etc. Based on the dry weight of the biological patch, the mass percentage of the first fatty acid in the biological patch can also be within any one of following ranges: $\geq$1.5%, from 1.5% to 8%, etc.

Further, based on any suitable implementations of the present disclosure, in some embodiments, a mass percentage of the first fatty acid in the fatty acids contained in the biological patch is $\geq$ 50%, optionally $\geq$ 65%. The mass percentage of the first fatty acid in the fatty acids contained in the biological patch can also be any one of the following percentages or be within a range between any two of the following percentages: 50%, 55%, 60%, 65%, 70%, 74%, 75%, 80%, 85%, etc. Non-limiting examples of the mass percentage of the first fatty acid in the fatty acids contained in the biological patch can also be 70% to 85%, 70% to 90%, 75% to 85%, 75% to 90%, 75% to 80%, etc. In some embodiments, the amount of "the fatty acids contained in the biological patch" can be estimated based on the above-described 37 fatty acids.

The biological patch provided in the present disclosure contains the second fatty acid. The inventors of the present disclosure have found that decreasing the content of the second fatty acid is beneficial to enhance the anti-inflammatory performance of the biological patch.

Based on any suitable implementations of the present disclosure, in some embodiments, based on the dry weight of the biological patch, a mass percentage of the second fatty acid in the biological patch is $\leq$2.0%, optionally $\leq$1.5%, further optionally $\leq$1%. Based on the dry weight of the biological patch, the mass percentage of the second fatty acid in the biological patch can also be any one of the following percentages or be within a range between any two of the following percentages: 0.01%, 0.05%, 0.1%, 0.2%, 0.3%, 0.4%, 0.5%, 0.6%, 0.8%, 1%, 1.2%, 1.5%, 1.6%, 1.8%, 2.0%, etc.

Further, based on any suitable implementations of the present disclosure, in some embodiments, a mass percentage of the second fatty acid in the fatty acids contained in the biological patch is $\leq$40%, optionally $\leq$26%. The mass percentage of the second fatty acid in the fatty acids contained in the biological patch can also be any one of the following percentages or be within a range between any two of the following percentages: 10%, 15%, 20%, 25%, 26%, 30%, 35%, 40%, etc. Non-limiting examples of the mass percentage of the second fatty acid in the fatty acids contained in the biological patch can also be 20% to 35%, 25% to 35%, 20% to 30%, 25% to 30%, etc. In some embodiments, the amount of "the fatty acids contained in the biological patch" can be estimated based on the above-described 37 fatty acids.

The inventors of the present disclosure have found that the content of the first fatty acid being greater than the content of the second fatty acid is beneficial to enhance the anti-inflammatory performance of the biological patch.

Based on any suitable implementations of the present disclosure, in some embodiments, a mass ratio of the first fatty acid to the second fatty acid in the biological patch is > 1, optionally $\geq$2, further optionally $\geq$2.5, and even further optionally in a range from 2.6 to 5. The mass ratio of the first fatty acid to the second fatty acid can also be any one of the following values or be within a range between any two of the following values: 2, 2.2, 2.4, 2.5, 2.6, 3, 3.5, 4, 4.5, 5, etc.

Based on any suitable implementations of the present disclosure, in some embodiments, the biological patch has a porosity ranging from 55% to 75%, and optionally from 55% to 70%. Unless otherwise specified, the porosity of the samples is measured according to "GB/T 21650.1-2008 Pore size distribution and porosity of solid materials by mercury porosimetry and gas adsorption - Mercury porosimetry".

Regular arrangement of fibers can facilitate regular cell adhesion and proliferation, while the presence of a porous structure helps the exchange of nutrients and oxygen for implantation and repair, promoting cell growth. The inventors of the present disclosure have found through research that the pore size of the prepared decellularized fish skin matrix is within the submicron range (e.g., 1 $\mu$m to 10 $\mu$m), which is close to the size of cells. This matrix control can affect cell behavior and thus is more conducive to the adhesion, proliferation, and diffusion of osteoblasts.

Based on any suitable implementations of the present disclosure, in some embodiments, the biological patch satisfies at least one of the following characteristics (any numerical parameters in the following characteristics can also be selected from any appropriate numerical values or ranges in the context):

based on the dry weight of the biological patch, a mass percentage of the first fatty acid in the biological patch is ≥1.4%, optionally in a range from 1.4% to 8%, further optionally in a range from 2% to 5%;
based on the dry weight of the biological patch, a mass percentage of the second fatty acid in the biological patch is ≤2%, optionally ≤1.5%, further optionally ≤1%;
a mass percentage of the first fatty acid in the fatty acids contained in the biological patch is ≥50%, optionally ≥65%;
a mass percentage of the second fatty acid in the fatty acids contained in the biological patch is ≤40%, optionally ≤26%;
a mass ratio of the first fatty acid to the second fatty acid in the biological patch is >1, optionally ≥2, further optionally ≥2.5, and even further optionally in a range from 2.5 to 5;
a porosity of the biological patch is in a range from 55% to 75%, and optionally a range from 55% to 70%.

Based on any suitable implementations of the present disclosure, in some embodiments, based on the dry weight of the biological patch, a mass percentage of butyric acid in the biological patch is in a range from 0% to 0.1%, optionally in a range from 0.03% to 0.1%. Based on the dry weight of the biological patch, the mass percentage of butyric acid in the biological patch can also be any one of the following percentages or be within a range between any two of the following percentages: 0%, 0.01%, 0.02%, 0.03%, 0.04%, 0.05%, 0.06%, 0.064%, 0.065%, 0.066%, 0.067%, 0.068%, 0.07%, 0.075%, 0.08%, 0.085%, 0.09%, 0.1%, etc. Based on the dry weight of the biological patch, the mass proportion of butyric acid in the biological patch can be any suitable range selected from the following: 300 ppm to 700 ppm (corresponding to 0.03% to 0.07%), 350 ppm to 700 ppm, 370 ppm to 690 ppm, 375 ppm to 685 ppm, 540 ppm to 1000 ppm, 550 ppm to 1000 ppm, 540 ppm to 800 ppm, 550 ppm to 800 ppm, 540 ppm to 700 ppm, 550 ppm to 700 ppm, 0.03% to 0.08%, 0.064% to 0.1%, etc.

Based on the dry weight of the biological patch, a mass proportion of caproic acid in the biological patch is in a range from 0 ppm to 40 ppm, optionally in a range from 0 ppm to 20 ppm.

Based on the dry weight of the biological patch, a mass proportion of lauric acid in the biological patch is in a range from 0 ppm to 55 ppm, optionally in a range from 20 ppm to 50 ppm. Based on the dry weight of the biological patch, the mass proportion of lauric acid in the biological patch can also be any one of the following proportions or be within a range between any two of the following proportions: 0 ppm, 1 ppm, 2 ppm, 3 ppm, 4 ppm, 5 ppm, 6 ppm, 8 ppm, 10 ppm, 12 ppm, 15 ppm, 20 ppm, 25 ppm, 30 ppm, 35 ppm, 40 ppm, 45 ppm, 50 ppm, etc.

Based on the dry weight of the biological patch, a mass percentage of myristic acid in the biological patch is in a range from 0% to 0.06%, optionally in a range from 0.01% to 0.05%. Based on the dry weight of the biological patch, the mass percentage of myristic acid in the biological patch can also be any one of the following proportions or be within a range between any two of the following proportions: 0.01%, 0.02%, 0.03%, 0.04%, 0.05%, 0.06%, etc.

Based on the dry weight of the biological patch, a mass proportion of pentadecanoic acid in the biological patch is in a range from 0 ppm to 120 ppm, optionally in a range from 0 ppm to 100 ppm, further optionally in a range from 0 ppm to 50 ppm, even further optionally in a range from 0 ppm to 40 ppm.

In some embodiments, based on the dry weight of the biological patch, a mass percentage of palmitic acid in the biological patch is in a range from 0% to 1.45%, optionally in a range from 0.28% to 1.45%, further optionally in a range from 0.4% to 0.8%. Based on the dry weight of the biological patch, the mass proportion of palmitic acid in the biological patch can be any suitable range selected from the following: 0% to 1.2%, 0.28% to 1%, 0.3% to 1%, 0.3% to 1.45%, 0.28% to 0.8%, 0.3% to 0.8%, etc.

Based on the dry weight of the biological patch, a mass percentage of stearic acid in the biological patch is in a range from 0% to 0.35%, optionally in a range from 0.05% to 0.35%, further optionally in a range from 0.05% to 0.15%, even further optionally in a range from 0.05% to 0.1%. Based on the dry weight of the biological patch, the mass proportion of stearic acid in the biological patch can be any suitable range selected from the following: 0% to 0.2%, 0% to 0.15%, etc.

Based on the dry weight of the biological patch, a mass proportion of arachidic acid in the biological patch is in a range from 0 ppm to 240 ppm, optionally in a range from 30 ppm to 240 ppm, further optionally in a range from 100 ppm to 240 ppm. Based on the dry weight of the biological patch, the mass proportion of arachidic acid in the biological patch can also be any one of the following proportions or be within a range between any two of the following proportions: 0 ppm, 30 ppm, 32 ppm, 33 ppm, 34 ppm, 40 ppm, 50 ppm, 60 ppm, 80 ppm, 100 ppm, 200 ppm, 240 ppm (0.024%), etc.

Based on the dry weight of the biological patch, a mass percentage of palmitoleic acid in the biological patch is in a range from 0% to 0.15%, optionally in a range from 0.01% to 0.15%, further optionally in a range from 0.01% to 0.05%. Based on the dry weight of the biological patch, the mass percentage of palmitoleic acid in the biological patch can also be any one of the following percentages or be within a range between any two of the following percentages: 0%, 0.01%, 0.02%, 0.03%, 0.04%, 0.05%, 0.06%, 0.07%, 0.08%, 0.09%, 0.1%, 0.12%, 0.13%, 0.14%, 0.145%, 0.149%, 0.15%, etc. Based on the dry weight of the biological patch, the mass percentage of palmitoleic acid in the biological patch can also be any suitable range selected from the following: 0% to 0.12%, 0% to 0.12%, 0.015% to 0.15%, 0.018% to 0.15%, 0.015% to 0.1%, 0.018% to 0.1%, 0.015% to 0.08%, 0.018% to 0.08%, 0.015% to 0.05%, 0.018% to 0.05%, etc.

Based on the dry weight of the biological patch, a mass percentage of oleic acid in the biological patch is in a range from 0% to 5%, optionally in a range from 0.5% to 3%, further optionally in a range from 1% to 2%, or can also be any suitable range selected from the following: 0.1% to 5%, 0.2% to 5%, 0.4% to 5%, 0.1% to 3%, 0.2% to 3%, 0.4% to 3%, 0.1% to 2%, 0.2% to 2%, 0.4% to 2%, 0.1% to 1.5%, 0.2% to 1.5%, 0.4% to 1.5%, etc. Based on the dry weight of the biological patch, the mass percentage of oleic acid in the biological patch can also be any one of the following percentages or be within a range between any two of the following percentages: 0.5%, 1%, 1.5%, 2%, 2.5%, 3%, 3.5%, 4%, 4.5%, 5%, etc.

Based on the dry weight of the biological patch, a mass percentage of $\gamma$-linolenic acid in the biological patch is in a range from 0% to 0.085%, optionally in a range from 0.01% to 0.085%, further optionally in a range from 0.01% to 0.05%. Based on the dry weight of the biological patch, the mass percentage of $\gamma$-linolenic acid in the biological patch can also be any one of the following percentages or be within a range between any two of the following percentages: 0%, 0.01%, 0.015%, 0.02%, 0.03%, 0.04%, 0.05%, 0.06%, 0.07%, 0.08%, 0.085%, etc. Based on the dry weight of the biological patch, the mass percentage of $\gamma$-linolenic acid in the biological patch can also any suitable range selected from the following: 0.015% to 0.085%, 0.016% to 0.084%, 0.015% to 0.05%, etc.

Based on the dry weight of the biological patch, a mass percentage of linolenic acid ALA in the biological patch is in a range from 0% to 0.28%, optionally in a range from 0.04% to 0.28%, further optionally in a range from 0.04% to 0.15%, even further optionally in a range from 0.04% to 0.1%. Based on the dry weight of the biological patch, the mass percentage of linolenic acid ALA in the biological patch can also be any one of the following percentages or be within a range between any two of the following percentages: 0%, 0.01%, 0.02%, 0.03%, 0.04%, 0.05%, 0.06%, 0.07%, 0.08%, 0.09%, 0.1%, 0.12%, 0.14%, 0.15%, 0.16%, 0.18%, 0.2%, 0.24%, 0.25%, 0.28%, etc. Based on the dry weight of the biological patch, the mass percentage of linolenic acid ALA in the biological patch can also be any suitable range selected from the following: 0% to 0.2%, 0.01% to 0.15%, 0.02% to 0.15%, 0.04% to 0.15%, 0.01% to 0.1%, 0.02% to 0.1%, etc.

Based on the dry weight of the biological patch, a mass proportion of eicosapentaenoic acid (EPA) in the biological patch is ≥80 ppm, optionally ≥150 ppm, further optionally ≥300 ppm, even further optionally ≥400 ppm, or can also be any suitable range selected from the following: 100 ppm to 600 ppm, 150 ppm to 600 ppm, 300 ppm to 600 ppm, 400 ppm to 600 ppm, etc. Based on the dry weight of the biological patch, the mass proportion of eicosapentaenoic acid EPA in the biological patch can also be any one of the following proportions, or greater than or equal to (≥) the following proportions, or be within a range between any two of the following proportions: 80 ppm, 90 ppm, 100 ppm, 110 ppm, 120 ppm, 130 ppm, 140 ppm, 150 ppm, 160 ppm, 180 ppm, 200 ppm, 250 ppm, 300 ppm, 350 ppm, 400 ppm, 420 ppm, 430 ppm, 440 ppm, 450 ppm, 500 ppm, 550 ppm, 600 ppm, etc.

Based on the dry weight of the biological patch, a mass percentage of erucic acid in the biological patch is in a range from 0% to 0.05%, optionally in a range from 0% to 0.04%, further optionally in a range from 0.01% to 0.04%, or can also be any suitable range selected from the following: 0 to 350 ppm, 0 to 300 ppm, 100 ppm to 350 ppm, 100 ppm to 300 ppm, 150 ppm to 350 ppm, 150 ppm to 300 ppm, etc. Based on the dry weight of the biological patch, the mass percentage of erucic acid in the biological patch can also be any one of the following percentages or be within a range between any two of the following percentages: 0%, 0.01%, 0.02%, 0.03%, 0.04%, 0.05%, etc.

Based on the dry weight of the biological patch, a mass percentage of docosahexaenoic acid DHA in the biological patch is in a range from 0% to 0.08%, optionally in a range from 0.01% to 0.07%, further optionally in a range from 0.02% to 0.06%, or can also be any suitable range selected from the following: 0.01% to 0.05%, 0.02% to 0.05%, 0.01% to 0.045%, 0.02% to 0.045%, etc. Based on the dry weight of the biological patch, the mass percentage of docosahexaenoic acid DHA in the biological patch can also any one of the following percentages or be within a range between any two of the following percentages: 0%, 0.01%, 0.02%, 0.03%, 0.04%, 0.05%, 0.06%, 0.07%, 0.08%, etc.

Based on the dry weight of the biological patch, a mass proportion of tetracosenoic acid in the biological patch is ≥40 ppm, optionally ≥70 ppm, further optionally ≥80 ppm, even further optionally in a range from 80 ppm to 100 ppm. Based on the dry weight of the biological patch, the mass proportion of tetracosenoic acid in the biological patch can also any one of the following proportions, or greater than or equal to (≥) the following proportions, or be within a range between any two of the following proportions: 44 ppm, 45 ppm, 50 ppm, 60 ppm, 65 ppm, 70 ppm, 75 ppm, 80 ppm, 85 ppm, 90 ppm, 95 ppm, 100 ppm, etc. Based on the dry weight of the biological patch, the mass proportion of tetracosenoic acid in the biological patch can also be, but is not limited to, any suitable range selected from the following: 40 ppm to 120 ppm, 40 ppm to 100 ppm, 40 ppm to 90 ppm, etc.

The above-described contents of butyric acid, caprylic acid, lauric acid, myristic acid, pentadecanoic acid, palmitic acid, stearic acid, arachidic acid, palmitoleic acid, oleic acid, γ-linolenic acid, linolenic acid ALA, eicosapentaenoic acid EPA, erucic acid, docosahexaenoic acid DHA, and tetracosenoic acid in the dry weight of the biological patch can be combined in any appropriate manners. The first fatty acid can satisfy one or more of these content characteristics.

In some embodiments, based on the dry weight of the biological patch, a mass percentage of linoleic acid in the biological patch is in a range from 0% to 2.5%, optionally in a range from 0.5% to 2.5%, further optionally in a range from 0.5% to 1. Based on the dry weight of the biological patch, the mass percentage of linoleic acid in the biological patch can also be any one of the following percentages or be within a range between any two of the following percentages: 0%, 0.1%, 0.2%, 0.3%, 0.4%, 0.5%, 0.6%, 0.8%, 1%, 1.2%, 1.5%, 1.6%, 1.8%, 2%, 2.2%, 2.4%, 2.5%, etc. Based on the dry weight of the biological patch, the mass percentage of linoleic acid in the biological patch can also be any suitable range selected from the following: 0% to 1.5%, 0% to 1%, 0.5% to 1%, 0.5% to 2.4%, 0.6% to 2.5%, 0.6% to 2.4%, 0.6% to 1.5%, 0.6% to 1%, 0.8% to 2.5%, 0.8% to 2.4%, 0.8% to 1.5%, 0.8% to 1%, etc.

Based on the dry weight of the biological patch, a mass percentage of arachidonic acid in the biological patch is in a range from 0% to 0.090%, optionally in a range from 0% to 0.085%, further optionally in a range from 0% to 0.06%, even further optionally in a range from 0.01% to 0.05%. Based on the dry weight of the biological patch, the mass percentage of arachidonic acid in the biological patch can also be any one of the following percentages or be within a range between any two of the following percentages: 0%, 0.01%, 0.02%, 0.03%, 0.04%, 0.05%, 0.06%, 0.07%, 0.08%, 0.085%, 0.090%, etc.

The above-described contents of linoleic acid and arachidonic acid in the biological patch can be combined in any appropriate manners. The second fatty acid can satisfy one or more of these content characteristics.

The above-described contents of the first fatty acid and the second fatty acid in the biological patch can be combined in any appropriate manners. The biological patch can satisfy one or more of these content characteristics.

In some embodiments, by controlling the contents of butyric acid, caprylic acid, and pentadecanoic acid in the biological patch, the inflammatory responses induced by the biological patch can be eliminated or ameliorated. In some preferred embodiments, increasing the content of butyric acid and reducing the contents of caprylic acid and pentadecanoic acid can inhibit the inflammatory responses induced by the biological patch. Based on the dry weight of the biological patch, the mass percentage of butyric acid in the biological patch can be controlled in a range from 0% to 0.1%, optionally in a range from 0.064% to 0.1%; the mass proportion of caprylic acid in the biological patch can be controlled in a range from 0 ppm to 40 ppm, optionally in a range from 0 ppm to 20 ppm; the mass proportion of pentadecanoic acid in the biological patch can be controlled in a range from 0 ppm to 100 ppm, optionally in a range from 0 ppm to 50 ppm, and further optionally in a range from 0 ppm to 40 ppm,

In some embodiments, based on the dry weight of the biological patch, a mass proportion of heptadecanoic acid (also known as heptadecylic acid, referring to n-heptadecanoic acid) in the biological patch is in a range from 0 ppm to 70 ppm, optionally in a range from 0 ppm to 40 ppm, further optionally in a range from 0 ppm to 20 ppm.

Based on the dry weight of the biological patch, a mass percentage of tetracosanoic acid (referring to lignoceric acid) in the biological patch is in a range from 0% to 0.6%, optionally in a range from 0.01% to 0.06%, further optionally in a range from 0.03% to 0.056%. Based on the dry weight of the biological patch, the mass percentage of tetracosanoic acid in the biological patch can also be any one of the following percentages or be within a range between any two of the following percentages: 0%, 0.01%, 0.02%, 0.03%, 0.04%, 0.05%, 0.055%, 0.056%, 0.06%, etc. Based on the dry weight of the biological patch, the mass percentage of tetracosanoic acid in the biological patch can also be any suitable range selected from the following: 0.04% to 0.06%, 0.045% to 0.06%, 0.04% to 0.055%, 0.045% to 0.055%, etc.

Based on the dry weight of the biological patch, a mass percentage of eicosenoic acid (referring to cis-11-eicosenoic acid) in the biological patch is in a range from 0% to 0.12%, optionally in a range from 0% to 0.1%, further optionally in a range from 0% to 0.08%, even further optionally in a range from 0% to 0.05%, or can also be any suitable range selected from the following: 0.01% to 0.12%, 0.02% to 0.12%, 0.03% to 0.12%, 0.01% to 0.1%, 0.02% to 0.1%, 0.03% to 0.1%, 0.01% to 0.08%, 0.02% to 0.08%, 0.03% to 0.08%, 0.01% to 0.06%, 0.02% to 0.06%, 0.03% to 0.06%, etc. Based on the dry weight of the biological patch, the mass percentage of eicosenoic acid in the biological patch can also be any one of the following percentages or be within a range between any two of the following percentages: 0%, 0.01%, 0.02%, 0.03%, 0.04%, 0.045%, 0.046%, 0.05%, 0.06%, 0.07%, 0.08%, 0.085%, 0.09%, 0.1%, 0.11%, 0.12%, etc.

Based on the dry weight of the biological patch, a mass percentage of eicosadienoic acid (referring to icosadienoic acid) in the biological patch is in a range from 0% to 0.095%, optionally in a range from 0% to 0.08%, further optionally in a range from 0% to 0.05%, or can also be any suitable range selected from the following: 0.01% to 0.05%, 0.01% to 0.095%, 0.02% to 0.095%, 0.03% to 0.095%, 0.035% to 0.095%, 0.01% to 0.07%, 0.02% to 0.07%, 0.03% to 0.07%, 0.035% to 0.07%, 0.01% to 0.05%, 0.05% to 0.05%, 0.03% to 0.05%, 0.035% to 0.05%, etc. Based on the dry weight of the biological patch, the mass percentage of eicosadienoic acid in the biological patch can also be any one of the following percentages or be within a range between any two of the following percentages: 0%, 0.01%, 0.02%, 0.03%, 0.04%, 0.045%, 0.046%, 0.05%, 0.06%, 0.07%, 0.08%, 0.085%, 0.09%, 0.095%, etc.

Based on the dry weight of the biological patch, a mass percentage of eicosatrienoic acid (referring to icosatrienoic acid) in the biological patch is in a range from 0% to 0.16%, optionally in a range from 0% to 0.15%, optionally in a range from 0% to 0.1%, further optionally in a range from 0% to 0.08%, or can also be any suitable range selected from the following: 0.01% to 0.08%, 0.02% to 0.08%, 0.03% to 0.08%, 0.04% to 0.08%, 0.01% to 0.1%, 0.02% to 0.1%, 0.03% to 0.1%, 0.04% to 0.1%, etc. Based on the dry weight of the biological patch, the mass percentage of eicosatrienoic acid in the biological patch can also be any one of the following percentages or be within a range between any two of the following percentages: 0%, 0.01%, 0.02%, 0.03%, 0.04%, 0.045%, 0.046%, 0.05%, 0.06%, 0.07%, 0.08%, 0.085%, 0.09%, 0.1%, 0.11%, 0.12%, 0.14%, 0.15%, 0.16%, etc.

Based on the dry weight of the biological patch, a total mass percentage of eicosenoic acid, eicosadienoic acid, eicosatrienoic acid, and arachidonic acid in the biological patch is in a range from 0% to 0.45%, optionally in a range from 0% to 0.4%, further optionally in a range from 0% to 0.3%, even further optionally in a range from 0% to 0.2%. Based on the dry weight of the biological patch, the total mass percentage of eicosenoic acid, eicosadienoic acid, eicosatrienoic acid, and arachidonic acid in the biological patch can also any one of the following percentages or be within a range between any two of the following percentages: 0%, 0.1%, 0.2%, 0.3%, 0.4%, 0.45%, 0.5%, etc.

In some embodiments, based on the dry weight of the biological patch, a mass proportion of docosadienoic acid in the biological patch is in a range from 0 ppm to 300 ppm, optionally in a range from 0 ppm to 200 ppm, further optionally in a range from 0 ppm to 50 ppm, or can also be any suitable range selected from the following: 10 ppm to 150 ppm, 10 ppm to 120 ppm, 10 ppm to 100 ppm, 10 ppm to 80 ppm, 10 ppm to 50 ppm, 20 ppm to 150 ppm, 2 ppm to 120 ppm, 20 ppm to 100 ppm, 20 ppm to 80 ppm, 20 ppm to 50 ppm, etc. Based on the dry weight of the biological patch, the mass proportion of docosadienoic acid in the biological patch can also be any one of the following percentages or be within a range between any two of the following percentages: 0 ppm, 10 ppm, 20 ppm, 30 ppm, 40 ppm, 50 ppm, 60 ppm, 80 ppm, 100 ppm, 120 ppm, 150 ppm, 200 ppm, 250 ppm, 300 ppm, etc.

In some embodiments, the biological patch contains a $C_{4-24}$ fatty acid, which may or may not include a $C_{4-24}$ saturated fatty acid. In some embodiments, a $C_{16-24}$ unsaturated fatty acid can be one or more selected from palmitoleic acid, oleic acid, linoleic acid, $\gamma$-linolenic acid, linolenic acid ALA, eicosenoic acid, eicosadienoic acid, eicosatrienoic acid, and arachidonic acid, eicosapentaenoic acid EPA, erucic acid, docosadienoic acid, docosahexaenoic acid DHA, and tetracosenoic acid.

In some embodiments, based on the dry weight of the biological patch, a mass percentage of fat components in the biological patch is <20%, optionally ≤15%. The mass percentage of the fat components in the biological patch corresponds to the total fat content in the biological patch, expressed as a mass percentage, and can be simply referred to as "fat content".

In some embodiments, based on the dry weight of the biological patch, the total fat content of the biological patch is higher than 1% and lower than 20% by mass percentage, for example, in a range from 1% to 19%, optionally in a range from 3% to 19%, and can also be any suitable range selected from the following: 3% to 18.5%, 3.4% to 18.3%, 3.4% to 19%, etc. A lower fat content is beneficial to avoid potential risks such as infection, tumorigenesis, and immune rejection. Unless otherwise specified, the total fat content is determined by using the method according to section 5, part 1, Fat Content Determination Method, of "GB 5009.168-2016 National Food Safety Standard". Different from the complete degreasing or complete non-degreasing in the prior art, the present disclosure adopts a partial degreasing method, resulting in a biological patch that is more effective in reducing or eliminating inflammatory responses.

In some embodiments, based on the dry weight of the biological patch, a mass percentage of $C_{4-24}$ fatty acids in the biological patch is ≤6%, optionally ≤5%, and further optionally ≤4%, for non-limiting example, in a range from 3% to 5%, in a range from 3% to 4%, etc.

In some embodiments, based on the dry weight of the biological patch, a mass percentage of $C_{16-24}$ unsaturated fatty acids in the biological patch is ≥1%, optionally in a range from 1% to 5%, and further optionally in a range from 2% to 4%.

In some embodiments, based on the dry weight of the biological patch, a mass percentage of the $C_{16-24}$ unsaturated fatty acids in the $C_{4-24}$ fatty acids is ≥70%, optionally ≥75%, for non-limiting example, in a range from 70% to 85%, in a range from 75% to 85%, in a range from 75% to 80%, etc.

In some embodiments, the $C_{16-24}$ unsaturated fatty acids include one or more of hexadecenoic acid, octadecenoic acid, octadecadienoic acid, octadecatrienoic acid, eicosenoic acid, eicosadienoic acid, eicosatrienoic acid, eicosatetraenoic acid, eicosapentaenoic acid, dodecenoic acid, docosadienoic acid, docosahexaenoic acid, and tetracosenoic acid.

In some embodiments, the $C_{4-24}$ fatty acids include $\omega$-3 polyunsaturated fatty acids. In some embodiments, based on the dry weight of the biological patch, a mass percentage of the $\omega$-3 polyunsaturated fatty acids in the biological patch is ≥0.05%, further optionally ≥0.07%, and even further optionally ≥0.1%, for non-limiting example, in a range from 0.05% to 0.4%, from 0.05% to 0.35%, from 0.05% to 0.3%, from 0.07% to 0.4%, from 0.07% to 0.35%, from 0.07% to 0.3%, from 0.1% to 0.4%, from 0.1% to 0.35%, from 0.1% to 0.3%, from 0.1% to 0.25%, from 0.1% to 0.2%, from 0.1% to 0.2%, etc.

The above-described contents of the fatty acids in the biological patch (based on the dry weight of the biological patch) can be combined in any appropriate manners. The biological patch can satisfy one or more of these content characteristics.

The biological patch includes a first surface and a second surface that are opposite to each other, and the second surface has a greater surface roughness than the first surface. In some embodiments, the biological patch satisfies one or more of the following characteristics (any numerical parameters in the following characteristics can also be selected from any appropriate numerical values or ranges in the context):

The surface roughness Ra of the first surface satisfies Ra ≤20 µm, optionally is in a range from 8 µm to 20 µm.

The surface roughness Ra of the second surface satisfies Ra ≥25 µm, optionally is in a range from 25 µm to 40 µm.

The two surfaces of the biological patch exhibit a significant difference in surface roughness, resulting in different cell adhesion states. Studies have shown that the surface with roughness in a range from 10 µm to 20 µm has a significant impact on cell compatibility, and surface roughness is one of the important factors controlling cell viability. Studies have found that rat osteoblasts are more suitable for growing in areas with higher surface roughness, and an increase in roughness can improve cell adhesion and growth behavior. Appropriately increasing the surface roughness can increase the contact area between the implant and blood cells, thereby improving the adhesion of platelets on the implant surface, creating favorable conditions for osteoblasts to migrate on the rough surface, and improving the contact state between osteoblasts and the implant.

When using the biological patch provided in the present disclosure, the rougher surface usually faces the site to be repaired, while the smoother surface faces away from the site to be repaired. More specifically, the second surface with higher roughness is used to contact the site to be repaired of the body, facilitating the recruitment of cells required for tissue repair and being conducive to the ingrowth of these cells. Conversely, the smoother first surface faces away from the site to be repaired, minimizing the ingrowth of fibroblasts and other cells, serving as a barrier.

In the present disclosure, unless otherwise specified, the surface roughness of the sample is evaluated using a roughness tester according to the method in "GB/T 10610-2009 Geometrical product specification (GPS) surface texture: Profile method - Rules and procedures for the assessment of surface texture".

Based on any suitable implementations of the present disclosure, in some embodiments, the first surface has a water contact angle of ≥50°, optionally in a range from 50° to 90°, and further optionally in a range from 51° to 90°. The water contact angle of the first surface can also be any one of the following angles or be within a range between any two of the following angles: 50°, 51°, 55°, 60°, 65°, 70°, 75°, 80°, 85°, 85°, etc. The water contact angle of the first surface can be smaller than 90°.

Based on any suitable implementations of the present disclosure, in some embodiments, the biological patch can absorb water weighing at least 2.5 times, optionally 3 to 5 times, the dry weight of the biological patch.

According to the water contact angle of the surface of the biological patch, the first surface of the biological patch exhibits excellent hydrophilicity. Over time, for example, within 30 s to 60 s, water can penetrate into the biological patch. Good hydrophilicity enables the biological patch to better absorb proteins upon contact, thereby promoting cell adhesion. The biological patch has good water absorption capacity. In some embodiments, the weight of water that the biological patch can absorb is about 3 to 5 times its own weight.

Based on any suitable implementations of the present disclosure, in some embodiments, a water content in the biological patch is ≤12 wt%, optionally ≤6 wt%, and further optionally in a range from 3 wt% to 6 wt%, by mass percentage.

In the present disclosure, the water content in the biological patch is usually in a range from 3 wt% to 5 wt% after freeze-drying. After standing for a period of time, the biological patch may absorb moisture from the air, potentially increasing the water content to or beyond 10 wt%. Varuious hydration conditions of the biological patch should be within the scope of protection of the present disclosure.

In the present disclosure, unless otherwise specified, the contents of various chemical components (including but not limited to the aforementioned various fatty acids) in the biological patch refer to the contents when the water content in the biological patch is ≤5 wt%. The dry weight of the biological patch that is used in describing the mass percentages or mass proportions of the chemical components in the biological patch is only used to describe the hydration condition when measuring the chemical components, and is not to be understood as a limitation on the protection scope of the present disclosure. For example, for a biological patch with a water content greater than 5 wt%, when testing the percentages of chemical components in the dry weight of the biological patch, the water in the biological patch is removed first until the water content ≤5 wt%, and then the weight or mass of the biological patch at the water content ≤5 wt% is used as the calculation basis. Howver, the biological patch in the states with a water content less than or equal to 5 wt% (≤5 wt%) and greater than 5 wt% (> 5 wt%) is all within the protection scope of the present disclosure.

The water content in the biological patch can be determined by using the method in accordance with "GB 5009.3-2016 National Food Safety Standard - Determination of Moisture in Food."

In some embodiments, the biological patch has a first surface and a second surface that are opposite to each other, and the biological patch satisfies one or more of the following characteristics (any numerical parameters in the following characteristics can also be selected from any appropriate numerical values or ranges in the context):

the first surface has a water contact angle of $\geq 50°$, optionally in a range from $50°$ to $90°$, and further optionally in a range from $51°$ to $90°$;
the biological patch can absorb water weighing at least 2.5 times, optionally 3 to 5 times, the dry weight of the biological patch;
a water content in the biological patch is $\leq 12$ wt%, optionally $\leq 6$ wt%, and further optionally in a range from 3 wt% to 6 wt% by mass percentage.

In the early stages of osseointegration, surface wettability can induce cell adhesion and enhance biocompatibility. The most favorable contact angle range for cell adhesion is believed to be $40°$ to $60°$. Improved wettability leads to reduced albumin adsorption and increased fibronectin adsorption, which is beneficial for cell adhesion. Albumin is a non-adhesive protein, while fibronectin is an adhesive protein. In terms of wettability, the oxygen content on the surface and the polar components of surface energy plays important roles in promoting cell proliferation, especially when the surface roughness is correspondingly increased. The intermolecular attractive forces that dominate the surface energy are generated by various intermolecular interactions, whose contributions to the total surface energy are additive.

In some embodiments, the biological patch provided in the present disclosure can exhibit good hydrophilicity and a large specific surface area, allowing for a large contact area with the cells and tissues, as well as rapid material exchange.

In some embodiments, based on an in vitro degradation testing method, the biological patch satisfies one or more of the following characteristics:

the biological patch has a degradation period in a range from 7 days to 180 days, and optionally in a range from 7 days to 84 days; and
after 7 days of degradation, a degradation ratio of the biological patch is in a range from 5.7% to 97%. In the present disclosure, unless otherwise specified, the term "degradation period" refers to the time required for the patch to degrade completely into fragments.

In the present disclosure, unless otherwise specified, the degradation period is determined by using the following method. The biological patch is completely immersed in a phosphate-buffered (PBS) solution at 0.1g/10mL, i.e., 10 mL of PBS solution per 0.1 g of the patch, and placed in a shaking incubator at $37\pm1$ °C for degradation experiment. The time period taken for complete degradation (i.e., the sheet-shaped body breaks down into fragments) is recorded as the "degradation period".

Unless otherwise specified, the criterion for "complete fragments" or "complete degradation" is that the sample is completely dissolved in the solution or no visible block-like sample remains.

The "degradation ratio of the biological patch after 7 days of degradation" can be obtained by using the following method. At day 7, the sample is removed, freeze-dried, and weighed. The mass loss is obtained by comparing the masses before and after degradation, and the percentage of mass loss to initial mass is recorded as the degradation ratio.

The biological patch provided in the present disclosure has good degradation performance, can provide a degradation rate and a degradation period that match tissue repair needs, and can be applied to the repair of different tissues. The degradation rate and the degradation period can be controlled by adjusting process parameters.

Residual DNA content is a critical indicator for evaluating animal-derived tissue materials, which can be determined by using the following method: 10 mg of the patch is weighed, crushed in liquid nitrogen, and placed in a test tube without DNA. DNA extraction is performed according to the instructions of a DNA extraction kit. The total amount of DNA is measured using Eppendorf BioPhotometer D30 (Germany), and the DNA content can be calculated based on the pre-weighed sample weight. In some embodiments, the mass proportion of DNA in the biological patch is less than or equal to 50 ng/mg, optionally less than or equal to 30 ng/mg, and further optionally less than or equal to 20 ng/mg.

In some embodiments, the biological patch is a single-layer biological patch with a single-layer structure, or a multi-layer biological patch formed by stacking and combining multiple single-layer biological patches. In some embodiments, the single-layer biological patch has a thickness in a range from 0.1 mm to 1 mm.

In some embodiments, the multi-layer biological patch has a thickness in a range from 0.5 mm to 2 mm. In the multi-layer biological patch, adjacent single-layer biological patches are fixed together by an adhesive layer or by a thread that penetrates the adjacent single-layer biological patches in the thickness direction.

In some embodiments, the number of the single-layer biological patches stacked in the multi-layer biological patch can be 2, 3 or 4.

The above-described biological patch can be prepared using the following method, which is simple in process and can simultaneously suppress inflammatory responses while maintaining the mechanical performance and degradation performance of the biological patch, making the patch suitable for soft tissue repair. It should be noted that the preparation method provided in the second aspect is for the single-layer biological patch. The preparation of a multi-layer biological patch will also be described below. The multi-layer biological patch can be prepared by adhering or sewing multiple single-layer biological patches together.

**In a second aspect of the present disclosure,** a method for preparing a biological patch is provided, which can be used to prepare the biological patch described in the first aspect of the present disclosure. The preparation method adopts specific reagents at specific concentrations for the degreasing treatment, pigment removal treatment, and decellularization treatment, and employs synergistic cooperation of the three steps to stably control the types and contents of fatty acids in the biological patch, as well as the diameter of collagen fibers, the pore size and porosity of the biological patch, thereby obtaining the biological patch described in the first aspect of the present disclosure.

It should also be noted that the definition of one or some of the parameters can be referred to the third, fourth, and fifth aspects below. Features from different aspects are allowed to be appropriately combined to the extent that the biological patch described in the first aspect of the present disclosure can be obtained.

In some embodiments, a method for preparing a biological patch is provided, including following steps: performing a degreasing treatment, a pigment removal treatment, and a decellularization treatment on fish skin, followed by a freeze-drying treatment, to obtain the biological patch described in the first aspect of the present disclosure; wherein the fish skin has undergone a surface mucus removal treatment and a blood removal treatment;

the degreasing treatment is performed by using a method including following steps: washing the fish skin with first treatment liquids to remove grease impurities from inner and outer surfaces of the fish skin, the washing methods including flushing and rinsing;

the pigment removal treatment is performed by using a method including following steps: soaking the degreased fish skin in a second treatment liquid, taking the degreased fish skin out from the second treatment liquid, removing pigment, and washing the degreased fish skin with a second washing liquid to obtain a depigmented fish skin matrix;

the decellularization treatment is performed by using a method including following steps: soaking the depigmented fish skin matrix in a third treatment liquid, taking the depigmented fish skin matrix out from the third treatment liquid, and washing the depigmented fish skin matrix with a third washing liquid to obtain a decellularized fish skin matrix.

The second washing liquid and the third washing liquid can be water, such as deionized water.

In some embodiment, the first treatment liquids include a first running washing liquid and a second washing liquid, and further optionally include a surfactant solution; the running washing liquid is water; the second washing liquid is a sodium carbonate aqueous solution or a starch aqueous solution, wherein a pH value of the sodium carbonate aqueous solution is in a range from 8.4 to 11.5, a mass percentage of starch in the starch aqueous solution is in a range from 1% to 5%, optionally in a range from 3% to 5%; and the surfactant solution is a sodium dodecyl sulfate aqueous solution, and a mass percentage of sodium dodecyl sulfate in the sodium dodecyl sulfate aqueous solution is in a range from 0.3% to 0.5%.

In some embodiment, the second treatment liquid is an aqueous solution containing a penetration enhancer, or an aqueous solution containing an acidic reagent, or an aqueous solution containing an acidic regulator. In some embodiment, the penetration enhancer includes sodium dodecyl sulfate, and a mass percentage of the penetration enhancer in the aqueous solution containing the penetration enhancer is in a range from 0.1% to 0.5%, optionally in a range from 0.3% to 0.5%.

In some embodiment, the acidic reagent is one or more selected from acetic acid, citric acid, hydrochloric acid, formic acid, sulfuric acid, and nitric acid, and a concentration of the acidic reagent in the aqueous solution containing the acidic reagent is in a range from 1 mmol/L to 500 mmol/L, a pH value of the acidic solution is in a range from 1 to 5. The definition of the acidic reagent can also be referred to any suitable implementations in the context. On a condition that the aqueous solution containing the acidic reagent is an acetic acid aqueous solution, a mass percentage of the acetic acid is $\geq$ 0.3 wt%, optionally in a range from 0.3 wt% to 3 wt%. For example, the acidic reagent includes one or more of citric acid and acetic acid.

In the present disclosure, for concentration units, "mol/L" can also be expressed as "M", and "mmol/L" can also be expressed as "mM".

In some embodiments, the acidic reagent is acetic acid, and the aqueous solution containing the acidic reagent is an acetic acid aqueous solution. In some embodiments, a molar concentration of acetic acid in the acetic acid aqueous solution can be in a range from 0.05 mol/L to 0.5 mol/L, optionally in a range from 0.15 mol/L to 0.5 mol/L.

In some embodiments, the aqueous solution containing the acidic reagent is an acetic acid aqueous solution, and a mass percentage of acetic acid in the acetic acid aqueous solution is in a range from 0.3 wt% to 3 wt%, optionally in a range from 1 wt% to 3 wt%.

In some embodiments, the acidic reagent is citric acid, and the aqueous solution containing the acidic reagent is a citric acid aqueous solution. In some embodiments, a molar concentration of the citric acid in the citric acid aqueous solution is in a range from 0.001 mol/L to 0.02 mol/L, optionally in a range from 0.005 mol/L to 0.015 mol/L, and further optionally in a range from 0.005 mol/L to 0.01 mol/L.

In some embodiments, the acidic regulator includes citric acid, acetic acid, or a combination thereof, and a mass proportion of the acidic regulator in the aqueous solution containing the acidic regulator is in a range from 0.1% to 0.3%.

In some embodiments, the third treatment liquid is a Triton X-100 aqueous solution or an aqueous solution containing an alkaline reagent. The third washing liquid is water, such as deionized water.

In some embodiments, a mass percentage of Triton X-100 in the Triton X-100 aqueous solution is in a range from 0.1% to 2%, optionally in a range from 0.5% to 2%.

In some embodiments, the alkaline reagent is one or more selected from sodium carbonate, sodium bicarbonate, trisodium phosphate, sodium monohydrogen phosphate, disodium hydrogen phosphate, sodium hydroxide, potassium carbonate, potassium bicarbonate, tripotassium phosphate, potassium monohydrogen phosphate, dipotassium hydrogen phosphate, and potassium hydroxide. A concentration of the alkaline reagent in the aqueous solution containing the alkaline reagent is in a range from 0.01 mol/L to 2 mol/L, and a pH value of the alkaline solution is in a range from 9 to 13. The definition of the alkaline reagent can also be referred to any suitable implementations in the context.

Based on any suitable implementations of the present disclosure, in some embodiments, the method for preparing the biological patch adopts an approach selected from the following:

Approach I: the first treatment liquids include a first running washing liquid and a second washing liquid, and may or may not include a surfactant solution; the second treatment liquid is an aqueous solution containing an acidic reagent; the third treatment liquid is an aqueous solution containing an alkaline reagent.

Approach II: the first treatment liquids include a first running washing liquid and a second washing liquid, the second washing liquid is a starch aqueous solution; the second treatment liquid is an aqueous solution containing a penetration enhancer, or an aqueous solution containing an acidic regulator; the third treatment liquid is a Triton X-100 aqueous solution.

Approach III: the first treatment liquids include a first running washing liquid and a second washing liquid, the second washing liquid is a sodium carbonate aqueous solution; the second treatment liquid is an aqueous solution containing an acidic regulator; the third treatment liquid is a Triton X- 100 aqueous solution.

Approach I employs an acid-alkali combined treatment for decellularization, which is simple with substantially no residual reagents and low cytotoxicity. This acid-alkali combined treatment method has minimal impact on the structure of the biological patch, allowing the biological patch to maintain good mechanical performance and to be formed into a product suitable for clinical use. In addition, the raw materials are widely available and cost-effective, and the process is environmentally friendly. Further details can be found in the third aspect below.

Approaches II and III adopt depigmentation treatment for the degreased fish skin, and the conditions for depigmentation are mild. Thus, the obtained fish skin exhibits good mechanical performance and degradation performance, facilitating tissue repair. The preparation method is simple, involves few process steps, requires short processing time, and causes no environmental pollution. The method can be used to form fish skin-based biological patches with various degradation periods, meeting the needs for repairing different tissues.

In some embodiments, such as but not limited to Approach II, after the degreasing treatment and before the pigment removal treatment, the degreased fish skin can be repeatedly frozen and thawed with a cryoprotectant to better remove surface moisture. The cryoprotectant can be an aqueous solution including one or both of sucrose and trehalose, such as but not limited to a sucrose aqueous solution with a concentration of 15 wt%.

In some embodiments, in the step of soaking the degreased fish skin in the second treatment liquid, the second treatment liquid is at a soaking temperature in a range from 0 °C to 60 °C, optionally from 20 °C to 35 °C, and further optionally from 20 °C to 30 °C.

In some embodiments, the second treatment liquid is not replaced or replaced at least once, and each replacement cycle is in a range from 1 h to 4 h, optionally, from 2 h to 3 h.

In some embodiments, in Approach II, in the step of soaking the degreased fish skin in the second treatment liquid, the second treatment liquid is replaced at least once, and each replacement cycle is in a range from 1 h to 4 h, optionally, from 2 h to 3 h.

In some embodiments, in Approaches II and III, the second treatment liquid may not be replaced.

In some embodiments, in the step of soaking the degreased fish skin in the second treatment liquid, the total soaking duration is in a range from 2 h to 8 h, optionally from 2 h to 4 h.

In some embodiments, the freeze-drying treatment is performed according to any one of the following methods:

Method F1: freeze-drying under vacuum at a temperature of -80 °C to -50 °C and a pressure of 15 Pa to 60 Pa for a freeze-drying period of 6 h to 24 h. Method F1 also allows for an optional step of pre-freezing at a temperature of -80 °C to -76 °C for a time period of 20 h to 24 h before freeze-drying under vacuum.

Method F2: pre-freezing at a temperature of -80 °C to -76 °C for a time period of 20 h to 24 h, and drying at a temperature of 20° C to 30 °C and a pressure of 38 Pa to 42 Pa for a time period 45 h to 50 h.

In some embodiments, after the decellularization treatment and before the freeze-drying treatment, the method further includes the following steps:

wetting the decellularized fish skin matrix obtained from the decellularization treatment with a moisture-retaining agent; and
performing a microwave treatment on the wetted decellularized fish skin matrix to puff the decellularized fish skin matrix.

The reason that the microwave treatment can cause fish skin to puff is mainly because the microwave treatment allows the moisture contained in the fish skin to quickly vaporize after uniformly absorbing heat, thereby making the fish skin swell and increase its thickness. Theoretically, besides microwaving, other heating methods, or methods involving rapid depressurization after pressurization can also cause fish skin to puff. However, these heating methods may severely damage the internal structure of the fish skin, making it unsuitable as a skin repair dressing. The present disclosure adopts microwave treatment to achieve puffing of the fish skin while minimizing the damage to the fish skin structure.

Unless otherwise specified, "wetting" refers to applying the moisture-retaining agent to the surface of the fish skin. As long as the moisture-retaining agent can be applied to the surface of the fish skin, any method can be used without special limitations, such as soaking, spraying, brushing, dipping, etc. The moisture-retaining agent is used to maintain the moisture in the fish skin during the subsequent treatment, and mainly includes oil, such as vegetable oil (e.g., soybean oil, linseed oil, castor oil, rapeseed oil), animal oil (e.g., lard, sheep oil, tallow), mineral oil, chemical synthetic oil (e.g., paraffin oil), etc. Considering the cost and safety, the moisture-retaining agent can be vegetable oil.

The biological patch prepared after microwave treatment exhibits excellent processability, can quickly absorb water, and becomes soft and docile after water absorption, avoiding breakage issues when implanted to the skin lesion site. Moreover, the decellularized fish skin matrix or biological patch does not contain cell components, offering excellent biocompatibility and cellular compatibility with low antigenicity. In addition, the fish skin matrix or biological patch puffed by microwave treatment has a greater thickness, comparable to the thickness of normal human skin, thereby preventing uneven surfaces between the biological patch and normal human skin, thus avoiding irregular scarring after healing. Furthermore, the increased thickness of the decellularized fish skin matrix or biological patch can effectively prevent fragmentation after implantation at the skin lesion site.

In some embodiments, the microwave treatment is performed at a power in a range from 500 W to 900 W, optionally from 700 W to 900 W.

In some embodiments, a time period of the microwave treatment is in a range from 5 s to 60 s, optionally from 30 s to 60 s.

**In a third aspect of the present disclosure,** a method for preparing a single-layer biological patch is provided, which can be used to prepare the biological patch described in the first aspect of the present disclosure.

In some embodiments, the method for preparing the single-layer biological patch includes:

performing an acid treatment on fish skin in an acidic solution, where the fish skin is used as an animal-derived tissue material, removing residual acidic solution, removing pigment, and washing the pigment-removed material to obtain a depigmented tissue material; wherein the fish skin has undergone a blood removal and a degreasing treatment, and the acidic solution is an aqueous solution containing an acidic reagent;
performing an alkali treatment on the depigmented tissue material in an alkaline solution to obtain an alkali-treated material; and washing the alkali-treated material to obtain a decellularized tissue material; wherein the alkaline solution is an aqueous solution containing an alkaline reagent;
pre-freezing the decellularized tissue material and freeze-drying the material under vacuum to obtain the single-layer biological patch.

This preparation method sequentially includes decellularization and drying steps. The decellularization step involves acid treatment and alkali treatment, employing an acid-alkali combined treatment approach, which is simple and effective, with minimal residual reagents and low cytotoxicity. The change in the fatty acid components of the obtained biological patch is conducive to inhibiting the occurrence of inflammatory responses. Pre-freezing followed by vacuum freeze-drying allows water in the material to sublimate directly from a solid to a gaseous state without a liquid phase, and thus the three-dimensional structure of the tissue can be better maintained. Consequently, the above preparation method has minimal impact on the tissue structure, especially as the freeze-drying method is conducive to retaining the intact three-dimensional structure of the tissue, balancing the mechanical performance and the in vivo degradation performance of the biological patch. The mechanical performance of the biological patch is critical for providing sufficient mechanical support during tissue injury repair, reducing inflammatory responses of the wound is essential for achieving rapid wound healing, and the degradation performance determines whether there is foreign matter remaining in the body after the tissue regeneration is complete.

The single-layer biological patch prepared by the above method can be used for soft tissue repair and tailored to suit different sizes of injured sites, meeting multiple requirements for biocompatibility, mechanical performance, dimensions, etc. in the repair of various tissue injuries.

In some embodiment, the acidic reagent is one or more selected from acetic acid, citric acid, hydrochloric acid, formic acid, sulfuric acid and nitric acid. A concentration of the acidic reagent in the acidic solution is in a range from 1 mmol/L to 500 mmol/L. A pH value of the acidic solution is in a range from 1 to 5.

In some embodiments, the acid treatment can be performed for 1 minute (min) to 60 min.

In some embodiments, the pigment is removed by scraping, stirring, shaking, or any combinations thereof.

In some embodiments, the pigment-removed material is washed with water under shaking, wherein for every 1 g of the pigment-removed material, the volume of water used is 10 mL to 30 mL; the washing is performed once or multiple times, with each washing lasting 5 min to 15 min.

In some embodiments, the alkaline reagent is one or more selected from sodium carbonate, sodium bicarbonate, trisodium phosphate, sodium monohydrogen phosphate, disodium hydrogen phosphate, sodium hydroxide, potassium carbonate, potassium bicarbonate, tripotassium phosphate, potassium monohydrogen phosphate, dipotassium hydrogen phosphate, and potassium hydroxide. A concentration of the alkaline reagent in the alkaline solution is in a range from 0.01 mol/L to 2 mol/L. A pH value of the alkaline solution is in a range from 9 to 13.

In some embodiments, the alkali treatment is performed at a temperature in a range from 20°C to 30°C under shaking at a speed in a range from 100 rpm to 200 rpm.

In some embodiments, the alkali treatment is performed for 6 h to 30 h.

In some embodiments, the alkali-treated material is washed with water under shaking, wherein for every 1 g of the alkali-treated material, the volume of water used is 10 mL to 50 mL; the washing is performed once or multiple times, with each washing lasting 1 min to 12 min.

In some embodiments, the pre-freezing is performed at a temperature in a range from -80 °C to -76 °C for a time period in a range from 20 h to 24 h. Unless otherwise specified, the pre-freezing is performed at normal pressure, that is, the ambient pressure is not particularly changed.

In some embodiments, the freeze-drying is performed at a temperature in a range from 20 °C to 30 °C at a pressure in a range from 38 Pa to 42 Pa, and the freeze-drying is performed for a time period in a range from 45 h to 50 h.

**Fish Skin-Derived Tissue Material**

In the present disclosure, the used fish skin-derived tissue material has undergone blood removal and degreasing unless otherwise specified. Excess blood, fat and other impurities can be removed through washing to minimize immunogenicity and the risk of viral infection.

The fish skin-derived tissue material is the dermis of fish skin, which is widely available and cost-effective. The fish skin used for the fish skin-derived tissue material is descaled fish skin or scaleless fish skin, such as but not limited to longsnout catfish skin, catfish skin, eel skin, or rice eel skin, e.g., scaleless skin of a Chinese longsnout catfish.

Before the acid treatment, the fish skin can be cut into a suitable shape and size. In some non-limiting examples, the fish skin can be cut into sheets, and further, can be cut into squares, rectangles, circles, or any other suitable sheet-shaped materials. Take squares or rectangles as examples, the dimensions can be such as (50 mm to 80 mm) × (80 mm to 120 mm), (80 mm to 100 mm) × (80 mm to 100 mm), (80 mm to 100mm) × (150 mm to 180 mm), etc. Some non-limiting examples include an 80 mm × 80 mm square, a 90 mm × 170 mm rectangle, a 60 mm × 100 mm rectangle, etc.

In the present disclosure, unless otherwise specified, the fish skin-derived tissue material is sheet-shaped, having two opposite surfaces, one surface being smooth and relatively dense (corresponding to the first surface), and the other surface being rough with a porous, loose structure (corresponding to the second surface).

Furthermore, the fish skin-derived tissue material can be a rectangular sheet with a thickness ranged from 0.5 mm to 2 mm, a dimension of 90 mm × 170 mm × 0.8 mm, 60 mm × 100 mm × 0.5 mm, etc., and a weight ranged from 10 g to 100 g.

The method for obtaining the above-described fish skin-derived tissue material can be as follows. Use a skinning machine to remove skin from a fish (e.g., scaleless skin of a Chinese longsnout catfish) weighing one kilogram or more (i.e., ≥1 kg); wash to remove excess impurities such as blood and fat; select intact fish skin with an appropriate size for cutting to ensure neat edges; and weigh the required amount of fish skin. It should be understood that the cutting step is optional.

The preparation method is described in detail below.

**Acid Treatment**

Acid treatment is performed on the fish skin-derived tissue material using an acidic solution. Non-restrictively, the fish skin-derived tissue material can be soaked and stand in the acidic solution for a period of time to achieve the acid treatment. During the acid treatment, the acidic reagent is ionized, generating hydrogen ions that can cause the protein components in the fish skin-derived tissue material to carry positive charges. Based on the principle of repulsion between like charges, the fish skin-derived tissue material swells and becomes larger in volume. During the acid treatment, the treated tissue material curls and puffs, and after the acid treatment, the tissue material becomes brittle.

In the present disclosure, unless otherwise specified, "puffing" refers to volume expansion. Taking the acid treatment of fish skin as an example, an ionization reaction occurs during this process, in which the hydrogen ions can cause the protein components in the fish skin to carry positive charges, leading to swelling and volume increase through the principle of like-charge repulsion.

The acidic reagent is a protonic acid that can release protons $H^+$ and can be in the form of molecules or ions. In some embodiment, the acidic reagent is one or more selected from acetic acid, citric acid, hydrochloric acid, formic acid, sulfuric acid, and nitric acid. A single type of acidic reagent can be used, or two or more types of acidic reagents can be combined, in which case the acidic solution can also be referred to as a composite acid solution. Using the composite acid solution for acid treatment has the following advantages: Strong and weak acids can be combined to precisely control the amount of hydrogen ions released and the release curve over time. In some embodiments, the acidic reagent in the composite acid solution is a combination of hydrochloric acid and acetic acid, wherein hydrochloric acid is a strong acid while acetic acid is a weak acid. This combination facilitates the provision of sufficient and stable hydrogen ion concentration throughout the acid treatment.

In some embodiments, the concentration of the acidic reagent in the acidic solution is in a range from 1 mM to 500 mM (i.e., from 0.001 M to 0.5 M), and for non-limiting examples, such as 10 mM to 500 mM, 50 mM to 500 mM, 100 mM to 500 mM, etc. The acid treatment is performed for 1 min to 60 min, such as 2 min to 45 min, 2 min to 30 min, etc. The appropriate acid treatment duration can be selected based on factors such as the weight of the sample to be treated, the type of acidic reagent, the concentration or pH of the acidic solution, etc. The pH value of the acidic solution can be in a range from 1 to 5, such as 2 to 4, 2.5 to 3.5. For example, the pH value of the acidic solution is 3, with reasonable deviations such as $\pm 0.2$, $\pm 0.1$, etc. If the concentration of the acidic solution is too high, or if the acid treatment duration is too long, the integrity of the fish skin may be compromised. Alternatively, if the pH value of the acidic solution is too low, the pigment may not be completely removed.

After the acid treatment, the residual acid solution needs to be removed. The method of removal is not particularly limited. For example, the residual acid solution can be poured out.

Before the acid treatment, the fish skin-derived tissue material may be pretreated, which can refer to the degreasing treatment step in the context. Features from different aspects are allowed to be appropriately combined to the extent that the biological patch described in the first aspect of the present disclosure can be obtained.

**Pigment Removal**

The fish skin-derived tissue material that has undergone blood removal and degreasing often still retains residual pigment. The residual pigment remained in the biological patch may lead to inconsistent coloration and the presence of unnecessary impurities, which are unfavorable for implantation in the body. In the present disclosure, pigment can be removed after acid treatment. Acid treatment weakens the bonding between pigment and the dermis, making the pigment easier to remove. The pigment can be removed using a physical method, such as scraping, stirring, shaking, or a combination thereof. For example, after removing the residual acidic solution, the loose pigment can be scraped off using a blunt instrument.

After pigment removal, the tissue material can be washed to remove excess impurities. The washing can be performed using an oscillatory method, i.e., the pigment-removed material can be washed under shaking. The liquid used for washing can be water, such as purified water, to avoid introduction of impurities.

In some embodiments, the pigment-removed material is washed with water under shaking. For every 1 g of the material to be washed (i.e., the pigment-removed material), the volume of water used can be 10 mL to 30 mL, such as 20 mL per gram material, 25 mL per gram material. The washing can be performed once or multiple times, such as 3 times, 4 times or 5 times. Each washing can last 5 min to 15 min, such as 5 min, 10 min or 15 min.

**Alkali Treatment**

Alkali treatment is performed on the depigmented tissue material in an alkaline solution, and the alkali-treated material is washed to obtain a decellularized tissue material. Further, the alkaline solution is an aqueous solution containing an alkaline reagent.

During the alkali treatment, the alkaline reagent reacts with the residual acidic reagent to neutralize the acid ions in the depigmented tissue material, reducing or eliminating the repulsion between like charges, thereby causing the puffed tissue material to shrink in volume and depuff. During the alkali treatment, the treated tissue material depuffs, and after the alkali treatment, the tissue material becomes soft and pliable.

The alkaline agent is a Lewis base, selected from molecules or atomic groups capable of providing electron clouds. For example, in some embodiments, the alkaline reagent is one or more selected from sodium carbonate, sodium bicarbonate, trisodium phosphate, sodium monohydrogen phosphate, disodium hydrogen phosphate, sodium hydroxide, potassium carbonate, potassium bicarbonate, tripotassium phosphate, potassium monohydrogen phosphate, dipotassium hydrogen phosphate, potassium hydroxide, etc. A single type of alkaline reagent can be used, or two or more types of alkaline reagents can also be combined, in which case the alkaline solution can also be referred to as a composite alkali solution. By using the composite alkali solution for alkali treatment, strong and weak bases can be combined to precisely control the concentration and concentration-time curve of the hydroxide ions released by the Lewis base. In some embodiments, the alkaline reagent in the composite alkali solution is a combination of sodium carbonate and sodium hydroxide, wherein sodium hydroxide is a strong base while sodium carbonate is a weak base. This combination facilitates the provision of sufficient and stable hydroxide ion concentration throughout the alkali treatment.

The concentration of the alkaline reagent is in a range from 0.01 M to 2 M (i.e., from 0.01 mol/L to 2 mol/L), and for non-limiting examples, such as 0.02 M to 2 M, 0.05 M to 2 M, 0.1 M to 2 M, 0.2 M to 2 M, 0.4 M to 2 M, 0.5 M to 2 M. The pH value of the alkaline solution is in a range from 9 to 13. For example, the pH value of the alkaline solution is 9 toll, 10 or 11, with reasonable deviations such as $\pm 0.2$, $\pm 0.1$, etc.

Unless otherwise specified, the alkali treatment is performed by soaking and standing. In an embodiment, the alkali treatment can be performed under shaking, e.g., can be performed in an oscillation box, and further can be performed in a thermostatic oscillation box. The thermostatic temperature can be set to 20°C to 30°C, for example, 20°C, 24°C, 25°C, 26°C, 28°C, 30°C, or any suitable temperatures. In other words, the alkali treatment can be performed at a temperature ranged from 20°C to 30°C under shaking. The shaking can be performed at a speed of 100 rpm to 200 rpm. The alkali treatment under shaking can accelerate the neutralization reaction and shorten the treatment time. In an embodiment, the alkali treatment can also be performed by soaking and standing in combination with shaking. The alkali treatment can be performed for 6 h to 30 h, for example, 6 h to 24 h.

If the concentration of the alkaline solution is too high, or if the alkali treatment duration is too long, or if the pH value of the alkaline solution is too high, the fish skin may be easily and severely dehydrated and wrinkled.

In some embodiments, after the alkali treatment, the tissue material is washed to remove residual alkaline solution. The washing can be performed using an oscillatory method, i.e., the alkali-treated material can be washed under shaking. In some embodiments, the liquid used for washing is water, which can avoid introduction of impurities. For every 1 g of the material to be washed (i.e., the alkali-treated material), the volume of water used can be 10 mL to 50 mL, which can also be expressed as 10 to 50 mL per gram material, such as 20 mL per gram material, 30 mL per gram material, 40 mL per gram material, etc. The alkali-treated material can be washed once or multiple times, such as 3 times, 4 times or 5 times. Each washing can last 1 min to 12 min, such as 2 min, 5 min or 10 min.

**Freeze-Drying**

The decellularized tissue material obtained after the alkali treatment is subjected to freeze-drying. The freeze-drying is conducive to retaining the porous structure of the material. The freeze-drying can be performed as follows. Pre-freeze the decellularized tissue material, followed by freeze-drying under vacuum. After freeze-drying, the tissue material appears as a white solid film. In the present disclosure, unless otherwise specified, "freeze-drying" refers to a drying technique in which, for materials containing solid water, the solid water is directly sublimated into a gaseous state under vacuum without a liquid state, thereby removing water. Therefore, the decellularized tissue material is typically pre-frozen to solidify the moisture, and the tissue structure can be better maintained during the subsequent freeze-drying.

The pre-freezing is performed at a temperature in a range from -80 °C to -76 °C for a time period in a range from 20 h to 24 h. For example, the pre-freezing is performed at -80°C for 24 h. The freeze-drying is performed under vacuum at a temperature in a range from 20 °C to 30 °C and a pressure in a range from 38 Pa to 42 Pa for a time period in a range from 45 h to 50 h, and can be performed in a freeze-dryer. For example, the freeze-drying is performed at a temperature of 25°C and a pressure of 40 Pa for 48 h.

In some embodiments, the pre-freezing is performed at a temperature of -80 °C to -76 °C for a time period of 20 h to 24 h, followed by freeze-drying at a temperature of 23 °C to 26 °C (e.g., 25 °C) and a pressure of 38 Pa to 42 Pa for a time period of 45 h to 50 h.

After freeze-drying, the material can be cut into suitable shapes and sizes for later use.

The single-layer biological patch prepared by the above preparation method can withstand a tensile force greater than 20 N (further optionally ≥25 N) without breaking (i.e., the tensile breaking force is greater than 20 N), for example, can withstand a tensile force of 25 N to 30 N.

The biological patch prepared by the above method meets multiple requirements for biocompatibility, mechanical performance, dimensions, etc. of the biological patch in the repair of various tissue injuries, including one or more of dura mater defect repair, sports tendon tear repair, hernia and abdominal wall defect repair, burn plastic surgery repair, oral membrane defect repair, etc.

**In a fourth aspect of the present disclosure,** a fish skin-based biological patch is provided, including a sheet-shaped body, wherein the sheet-shaped body includes a collagen matrix; the fish skin-based biological patch is prepared from fish skin through treatments including depigmentation and decellularization; the fish skin-based biological patch has a degradation period in a range from 7 d to 180 d, a fat content in a range from 1% to 19%, and a residual DNA content less than 50 ng/mg. The fish skin-based biological patch can be used as the biological patch described in the first aspect of the present disclosure.

In some embodiments, the fish skin-based biological patch is prepared by a preparation method including: performing a degreasing pretreatment on the fish skin to prepare pretreated fish skin; placing the pretreated fish skin in an aqueous solution, performing a depigmentation treatment, and optionally a partial decellularization treatment, on the pretreated fish skin to obtain a depigmented fish skin; performing a decellularization treatment on the depigmented fish skin to obtain the fish skin-based biological patch; wherein in the depigmentation treatment, the aqueous solution is replaced every 1 h to 4 h, and optionally 2 h to 3 h.

The method of degreasing pretreatment can refer to the description of other aspects of the present disclosure, such as the method of degreasing treatment described in the second aspect of the present disclosure.

In some embodiments, the step of performing the degreasing pretreatment on the fish skin includes: flushing the fish skin with running water; and soaking or washing the fish skin in a starch aqueous solution. The step of flushing the fish skin with running water can be performed 2 to 3 times, each time for 5 min to 10 min. The starch aqueous solution treatment can further remove fat and impurities such as oil on the surface of the fish skin.

In some embodiments, the depigmentation treatment is performed at a temperature in a range from 0 °C to 60 °C, for example but not limited to, 0 °C, 10 °C, 20 °C, 30 °C, 40 °C, 50 °C, 60 °C, or within a range between any two of the above values.

In some embodiments, the aqueous solution further includes one or more additives selected from a penetration enhancer and an acid or alkali regulator. The penetration enhancer includes sodium dodecyl sulfate, and a mass percentage of the penetration enhancer in the aqueous solution is in a range from 0.3% to 0.5%. The acid or alkali regulator includes an acidic regulator, and the acidic regulator includes one or more of citric acid or acetic acid.

In some embodiments, the step of performing the decellularization treatment on the depigmented fish skin includes: decellularizing the depigmented fish skin with a Triton solution containing 0.1% to 10% by mass of Triton X-100. The decellularization treatment can be performed by soaking at a temperature of 20°C to 37°C for 12h to 24h.

For the above-mentioned fish skin-based biological patch, its preparation method and medical material, the fish skin-based biological patch is prepared through treatments including depigmentation and decellularization without harming the fish skin structure or the performance. The resulting fish skin-based biological patch has a wide degradation period and can meet the needs for repairing different tissues.

In the method for preparing the fish skin-based biological patch, the depigmentation treatment can be performed on the pretreated fish skin in water bath, and the aqueous solution is replaced every 1 h to 4 h. The depigmentation conditions are mild, without harming the fish skin structure or the performance. Thus, good mechanical performance and degradation performance that facilitate tissue repair can be obtained. In addition, the preparation method is simple, involves few process steps, requires short processing time, and causes no environmental pollution.

**In a fifth aspect of the present disclosure,** a method for preparing a decellularized fish skin matrix is provided, particularly involving a post-treatment and a pre-treatment. The prepared decellularized fish skin matrix can be used as the biological patch described in the first aspect of the present disclosure.

In some embodiments, the method for preparing the decellularized fish skin matrix includes: wetting a decellularized fish skin with a moisture-retaining agent; and performing a microwave treatment on the wetted fish skin to puff the fish skin; wherein the microwave treatment is performed at a power of 500 W to 900 W for 5 s to 60 s.

In the above-mentioned preparation method, the "decellularized fish skin" can be the "decellularized tissue material" in the third aspect of the present disclosure, or can be a semi-finished product, such as the fish skin-based biological patch after decellularization in the fourth aspect of the present disclosure. The decellularized fish skin matrix does not contain cellular components, offering excellent biocompatibility and cellular compatibility, with low antigenicity. By adopting the microwave puffing treatment of the fifth aspect of the present disclosure, the obtained decellularized fish skin matrix exhibits excellent processability, can quickly absorb water, and becomes soft and docile after water absorption, avoiding breakage issues when implanted to the skin lesion site. In addition, the fish skin matrix puffed by microwave treatment has a greater thickness, comparable to the thickness of normal human skin, thereby preventing uneven surfaces between the biological patch and normal human skin, thus avoiding irregular scarring after healing. Furthermore, the increased thickness of the decellularized fish skin matrix can effectively prevent fragmentation after implantation at the skin lesion site.

The implementation of steps in the preparation method can refer to other aspects of the present disclosure, such as that described in the second aspect.

In some embodiments, the microwave treatment is performed at a power in a range from 500 W to 900 W, and a time period of the microwave treatment is in a range from 30 s to 60 s; alternatively, the microwave treatment is performed at a power in a range from 700 W to 900 W, and a time period of the microwave treatment is in a range from 5 s to 60 s.

In some embodiments, before performing the decellularization treatment on the fish skin, the method can further include steps of removing mucus and oil from the surfaces of the fish skin, and removing pigment cells and connective tissue from the inner and outer surfaces of the fish skin. The step of removing mucus and oil from the surfaces of the fish skin can include: rinsing the fish skin in an alkali solution with a pH value of 8.4 to 11.5, the mass percentage concentration of alkali in the alkaline solution can be in a range from 0.53% to 2.86%. The alkali can mainly include sodium carbonate. The rinsing can be performed 2 to 3 times, with each rinsing lasting 5 min to 10 min. The step of removing pigment cells and connective tissue from the inner and outer surfaces of the fish skin can include: soaking the mucus and oil removed fish skin in an acid solution with a pH value of 2.0 to 4.5 at a temperature of 20°C to 37°C. The mass percentage concentration of acid in the acidic solution can be in a range from 0.08% to 2%. During the soaking process in the acid solution, stirring or shaking can also be performed. For example, the acid solution containing the fish skin can be placed on a shaker with a rotation speed of 150 rpm to 250 rpm and shaken for 2 min to 10 min.

In some embodiments, a Triton X-100 aqueous solution with a mass percentage concentration of 2% can be used for the decellularization treatment. The decellularization treatment can be performed by soaking at a temperature of 20 °C to 37 °C for 12h to 24h or shaking on a shaker at a rotation speed of 200 rpm for 6h to 12h.

In some embodiments, after the microwave treatment, the method further includes a step of freeze-drying the decellularized fish skin matrix. The freeze-drying is performed at a temperature of -80 °C to -50 °C and a vacuum degree of 15 Pa to 60 Pa for a time period of 6 h to 24 h.

In some embodiments, the fish skin is selected from scaleless fish skin. The scaleless fish skin can be selected from longsnout catfish skin, catfish skin, eel skin, and rice eel skin.

**In a sixth aspect of the present disclosure,** a multi-layer biological patch and a preparation method thereof are provided. The biological patch has low cytotoxicity, good biocompatibility, suppressed or eliminated inflammatory responses, good mechanical performance, moderate degradation performance, and can be used for soft tissue repair.

The method for preparing the multi-layer biological patch can involve using the single-layer biological patch described in the first aspect, or the single-layer biological patches prepared by the preparation methods in the second, third, fourth, and fifth aspects. In the preparation method of the sixth aspect, a lamination technique is combined with the single-layer biological patch, employing a simple and effective multi-layer composite process (e.g., using adhesives or sewing techniques) to further improve the mechanical performance of the biological patch. For injuries such as sports tendon or ligament ruptures, which have higher requirements for mechanical performance, the multi-layer biological patch can be preferably used.

The method for preparing the multi-layer biological patch can include following steps: forming a plurality of single-layer biological patches into a multi-layer biological patch through adhering, pressing, and drying, or through sewing, wherein the single-layer biological patches can be the single-layer biological patches described in the first aspect of the present disclosure. The prepared multi-layer biological patch can have a thickness in a range from 0.5 mm to 2 mm.

Method 1: Adhesion Method

In some embodiments, a plurality of single-layer biological patches are adhered, pressed, and dried to form a multi-layer biological patch. For example, a multi-layer biological patch can be prepared from two, three, or four single-layer biological patches by using a method including the following steps:

adhering multiple single-layer biological patches into a multi-layer material with an adhesive solution, wherein the adhesive solution is filled between any two adjacent single-layer biological patches, optionally, the adhesive solution is an aqueous solution containing an adhesive;
pressing the multi-layer material; and
vacuum drying the pressed multi-layer material.

In some embodiments, the multiple single-layer biological patches used to prepare the multi-layer material can preferably have substantially the same surface shape and size, so that the surface outer contours of the multiple single-layer biological patches are aligned with each other, reducing the need for edge trimming in subsequent processing steps and saving time.

In some embodiments, the adhesive can be made of a natural polymer material or a synthetic polymer material. The adhesive is one or more selected from silk fibroin, gelatin, hydroxyethyl cellulose, hydroxymethyl cellulose, polylactic acid, polycaprolactone, and polyglycolic acid. Non-limiting examples of natural polymer materials include silk fibroin, gelatin, hydroxyethyl cellulose, etc., with silk fibroin being preferred due to its strong adhesion and high mechanical strength. Non-limiting examples of synthetic polymer materials include polylactic acid, polycaprolactone, polyglycolic acid, etc., with polylactic acid (PLA) being preferred due to its degradability and high mechanical strength.

In some embodiments, a mass-volume concentration of the adhesive in the adhesive solution is in a range from 10% to 30% (w/v), for example in a range from 15% to 25% (w/v).

In some embodiments, as a non-limiting example, the multi-layer material is prepared by a method including the following steps: selecting 3 to 4 pieces of fish skin with the same shape and size and good integrity, evenly applying an adhesive solution (with a preset coating amount and a preset adhesive concentration) to a surface of one piece of fish skin with a coating rod, and covering and adhering another piece of fish skin onto the surface of fish skin applied with the adhesive solution. The above steps are repeated to adhere the pieces of fish skin layer by layer, obtaining a three-layer or four-layer material. The adhesive solution can be a solution containing a natural polymer (such as silk fibroin, gelatin, hydroxyethyl cellulose, etc., preferably silk fibroin), or a solution containing a synthetic polymer (such as polylactic acid, polycaprolactone, polyglycolic acid, etc., preferably polylactic acid).

The adhesion strength can be tested using the following method to evaluate the effectiveness of the adhesion method:

Cut freeze-dried single-layer fish skin into strip-shaped samples sized 200 mm × 25 mm. Apply adhesive solution to a 125 mm × 25 mm area of one fish skin sample to adhere another fish skin sample, leaving an unbonded area of 75 mm × 25 mm.

Peel apart the unbonded fish skin areas to create two ends, secure both ends with clamps, and use a universal material mechanical testing machine with a gauge length of 50 mm, a width of 25 mm, and a speed of 250 mm/min until the adhered fish skin detaches.

Record the adhesion strength. The adhesion strength is calculated as follows: Adhesion strength = Maximum peel force × 2.5 cm / (2.5 cm × 2.5 cm).

The higher the adhesion strength, the stronger the adhesion between adjacent layers and the better the adhesion effect.

It should be understood that sample dimensions may vary, but when comparing the adhesion strength of different samples, comparison shall be made upon results obtained using the same testing method.

Method 2: Sewing Method

The thickness of the multi-layer biological patch prepared using the sewing method can be selected from any suitable thicknesses mentioned above.

In some embodiments, a plurality of single-layer biological patches are sewed together to form a multi-layer biological patch by using a method including the following steps: stacking multiple single-layer biological patches into a multi-layer stack; and stitching the multi-layer stack at a preset position with a thread. The thread is one or more selected from of a polylactic acid thread, a polyglycolic acid thread, a polyester thread, a silk thread, etc., with a silk thread being preferred.

In some embodiments, the sewing method can adopt a diagonal cross-stitch pattern. For example, stitches can be formed along a circle at a position 1 cm from the outer contour of the multi-layer stack, then along the diagonal lines. Alternatively, a "concentric circles" stitch pattern can be adopted. For example, stitches can be formed along a circle at a position 1 cm from the outer contour of the multi-layer stack, then along another circle at a position 3 cm from the outer contour, resulting in a 2 cm spacing between adjacent stitched threads. For larger multi-layer stacks, stitches can be formed along multiple circles with adjacent stitch trajectories spaced 2 cm apart. Alternatively, a multi-point cross-stitch pattern with multiple horizontal and vertical lines can be adopted. For example, stitches can be formed along lines at 1 cm intervals in both horizontal and vertical directions. These sewing methods are applicable to multi-layer stacks of any suitable dimensions, including but not limited to sheet-shaped multi-layer stacks in a size of 80 mm × 80 mm. In contrast, if the following sewing method is used: stitching an 80 mm × 80 mm surface along one circle at a position 0.5 cm from the outer contour and another circle at a position 3.5 cm from the outer contour to tightly bond multiple pieces of fish skin, adjacent single-layer biological patches may not be well attached, inducing low tensile breaking force and low adhesion strength.

In some embodiments, the required stitching length (denoted as LA) per square centimeter of the stack area is ranged from 0.5 cm to 2 cm, such as 0.5 cm, 0.64 cm (±0.01 cm), 1.75 cm, etc., wherein the stack area refers to the traverse overlapping portion of multiple single-layer biological patches in the multi-layer stack. In the present disclosure, the required stitching length per square centimeter of the stack area reflects the density of the stitching trajectories. The greater the LA value, the denser the stitches. An over short stitching length may lead to looseness in some areas, causing delamination. Additionally, the perforations created by the threads on the patch can cause uneven local stress, and may compromise the structural stability of the biological patches if the stitching length is too long.

In some embodiments, the minimum distance (denoted as $D_{min}$) from any point on the stitched surface of the multi-layer stack to the nearest stitching trajectory satisfies $D_{min} \leq 1.6$ cm, optionally $\leq 1.5$ cm, further optionally $\leq 1.45$ cm, furthermore optionally $\leq 1.42$ cm. In some embodiments, the maximum value of $D_{min}$ is about 1.414 cm. In some embodiments, the maximum value of $D_{min}$ is about 1.24 cm. In some embodiments, the maximum value of $D_{min}$ is about 1.17 cm. It can be understood that $D_{min}$ equals 0 for points located on the stitching trajectories. The "stitched surface" refers to the surface being stitched, which is penetrated by threads for stitching.

For all points on the stitched surface excluding the points on stitching trajectories, the maximum value among all $D_{min}$ values reflects the density of the stitching trajectory. The smaller the maximum value of $D_{min}$, the denser the stitches. It should be noted that the distance between the outer contour of the multi-layer biological patch and the stitching trajectory should also be considered, as insufficient attachment at edges of the patches may adversely affect peel performance.

If the stitching length is not long enough (e.g., LA is too small) or if there are unstitched points with large $D_{min}$ values, delamination may occur due to insufficient attachment between single-layer biological patches.

In some embodiments, the multi-layer biological patch can withstand a tensile force greater than 75 N, optionally $\geq 80$ N, further optionally $\geq 90$ N, furthermore optionally $\geq 100$ N, furthermore optionally $\geq 110$ N, furthermore optionally $\geq 120$ N, furthermore optionally $\geq 130$ N, without breaking, for example, can withstand a tensile force ranged from 75 N to 150 N, from 80 N to 150 N, or from 90 N to 150 N. The testing method for tensile strength (i.e., tensile breaking force) can be found in the example section below.

The multi-layer biological patch provided in the present disclosure exhibits good adhesion strength between the single-layer biological patches. The adhesion strength can be tested using the method shown in the examples below. In some embodiments, the adhesion strength of the multi-layer biological patch is greater than 70 J/cm$^2$, for example, in a range from 70 J/cm$^2$ to 280 J/cm$^2$. The adhesion strength of some multi-layer biological patches prepared by the adhesion method can be in a range from 70 to 100 J/cm$^2$. The adhesion strength of some multi-layer biological patches prepared by the sewing method is in a range from 180 to 280 J/cm$^2$.

If 504 glue is used as the adhesive solution, the resulting multi-layer biological patch has low adhesion and high cytotoxicity.

If catgut suture is used as the stitching thread, the resulting multi-layer biological patch has low tensile breaking force and poor adhesion strength.

**In a seventh aspect of the present disclosure,** use of the biological patch described in the first aspect of the present disclosure, or the biological patches or decellularized fish skin matrices prepared by the methods described in the second, third, fourth, fifth, and sixth aspects of the present disclosure, in preparation of a soft tissue repair implant material is provided.

In some embodiments, the soft tissue repair for which the soft tissue repair implant material is applicable includes one or more selected from dura mater defect repair, sports tendon tear repair, hernia and abdominal wall defect repair, burn plastic surgery repair, and oral membrane defect repair.

The biological patches and decellularized fish skin matrices provided in the present application exhibit low in vivo inflammatory responses and excellent mechanical performance, and can be used for soft tissue repair. In addition, these biological patches have favorable degradation performance.

**In an eighth aspect of the present disclosure,** a method for soft tissue repair or skin repair is provided, including a step of applying a biological patch to the lesion site;

wherein the biological patch can be the biological patch described in the first aspect of the present disclosure, or the biological patch prepared by the method described in the second aspect of the present disclosure, or the single-layer biological patch prepared by the method described in the third aspect of the present disclosure, or the fish skin-based biological patch described in the fourth aspect of the present disclosure, or the decellularized fish skin matrix prepared by the method described in the fifth aspect of the present disclosure. The lesion site can be in the body or on the skin.

In some embodiments, the biological patch includes a first surface and a second surface that are opposite to each other, and a surface roughness of the second surface is greater than that of the first surface. Taking the fish skin-based biological patch as an example, the first surface is derived from the outer surface of the fish skin that contacts the water environment, while the second surface is derived from the inner surface of the fish skin that contacts the internal tissues of the fish.

In the present disclosure, when using the biological patch, the rougher surface usually faces the site to be repaired, while the smoother surface faces away from the site to be repaired. More specifically, the second surface with higher roughness is used to contact the site to be repaired of the body, facilitating the recruitment of cells required for tissue repair, nutrient exchange, and the migration, adhesion, and proliferation of cells required for tissue repair. Conversely, the smoother first surface faces away from the site to be repaired, minimizing the ingrowth of fibroblasts and other cells, serving as a barrier.

The subject can be an animal, further can be a mammal, and furthermore can be a human.

The aforementioned biological patches (including the single-layer biological patches and the multi-layer biological patches) can be used for soft tissue repair and can be tailored to suit different sizes of injured sites, facilitating clinical use and meeting multiple requirements for biocompatibility (suppression or elimination of inflammatory responses), mechanical performance, dimensions, degradation performance, etc. in the repair of various tissue injuries.

The biological patches can provide a protective barrier, and the specific types and contents of fatty acids present in the biological patches can effectively reduce inflammatory responses in surrounding tissues, demonstrating excellent biocompatibility, promoting cell adhesion and growth, and facilitating the ingrowth of surrounding tissues. Additionally, the biological patches also have good mechanical performance, providing support during tissue repair. Furthermore, in certain repair sites, the degradation performance of the biological patches matches the progress of repair, which can further promote the formation of new tissue.

The following provides some examples, and the embodiments of the present disclosure will be described in detail in combination with the examples. It should be understood that these examples are only intended to explain the present disclosure rather than being construed as limitation to the scope of the present disclosure. In the following examples, experimental methods not specifying specific conditions should refer first to the guidance given in the present disclosure, or to the experimental manuals or conventional conditions in the art, or to the conditions recommended by the manufacturers, or to the experimental methods known in the art.

In the examples described below, the measurement parameters for raw material components may have minor deviations within the range of weighing accuracy unless specifically stated, and the temperature and time parameters may have acceptable deviations due to instrument testing accuracy or operational precision.

In the examples described below, scaleless skin of longsnout catfish was used as an example. It can be understood that other types of fish skin can also be used, referring to the above description. Steps without specified operating temperatures follow the temperatures of conventional methods, typically performed at room temperature.

For molar concentrations, mmol/L can be expressed as mM, and mol/L can be expressed as M.

**Examples - Part I**

I. Method for Preparing Single-Layer Biological Patch

The single-layer biological patches were prepared by a method sequentially including steps of degreasing treatment, pigment removal treatment, decellularization treatment, and freeze-drying.

Raw material: Scaleless fish skin of a Chinese longsnout catfish was taken out from a -20 °C freezer and placed in a -4 °C refrigerator for slow overnight thawing. A skinning machine was used to remove skin from a fish weighing 1 kg or more (i.e., ≥1 kg).

(1) Degreasing Treatment: the fish skin was washed with first treatment liquids to remove grease impurities on inner and outer surfaces of the fish skin, the washing included flushing and rinsing. The first treatment liquids included a first running washing liquid (water) and a second washing liquid (sodium carbonate aqueous solution or starch aqueous solution), and optionally included a surfactant solution (sodium dodecyl sulfate (SDS) aqueous solution).

Running water was used as the first running washing liquid in the first treatment liquids for flushing. The visible yellow grease can be flushed and removed from the skin surface by running tap water. During the flushing process, a certain water flow rate was maintained to flush the fish skin for a certain time period (such as 5 min to 10 min).

When the second washing liquid in the first treatment liquids was the starch aqueous solution, the fish skin was repeatedly washed three times with the concentration shown in Table 1 to remove surface fat.

When the second washing liquid in the first treatment liquids was the sodium carbonate aqueous solution, the fish skin was repeatedly rinsed three times with the concentration shown in Table 1 to remove mucus and grease from the inner and outer surfaces of the fish skin.

For the optional (may or may not be used) surfactant solution (sodium dodecyl sulfate aqueous solution) in the first treatment liquids, the fish skin was repeatedly washed three times with the concentration shown in Table 1.

The first running washing liquid can be applied prior to the second washing liquid or after the second washing liquid, or the first running washing liquid can be applied both before and after the second washing solution.

After treatment with the second washing liquid, the fish skin can be flushed repeatedly with deionized water three times to remove residual reagents.

After removing the surface fat, optionally, the undamaged degreased fish skin can be soaked in a 15 wt% sucrose solution as the cryoprotectant for 30 min, the surface moisture can be removed, and then the fish skin can be repeatedly frozen and thawed in a -20°C freezer, with the freeze-thaw process repeated three times.

(2) Pigment Removal Treatment: the degreased fish skin was soaked in a second treatment liquid (optionally under shaking on a shaker at a speed of 150 rpm to 200 rpm), and taken out from the second treatment liquid to remove the pigment. The soft pigment can be scraped off from the fish skin using a blunt instrument, and then the fish skin can be repeatedly flushed with deionized water three times or rinsed in deionized water three times under shaking to obtain a depigmented fish skin matrix.

The second treatment liquid was an aqueous solution containing a penetration enhancer, or an aqueous solution containing an acidic reagent, or an aqueous solution containing an acidic regulator.

The aqueous solution containing the acidic reagent can be the same as the aqueous solution containing the acidic regulator.

(3) Decellularization Treatment: the depigmented fish skin matrix was soaked in a third treatment liquid (optionally under shaking on a shaker at a speed of 150 rpm to 200 rpm), taken out from the third treatment liquid, and flushed with deionized water repeatedly three times to obtain a decellularized fish skin matrix.

(4) Freeze-drying.

The freeze-drying can be performed using Method F1 or F2.

Method F1: freeze-drying under vacuum at a temperature of -80 °C to -50 °C and a pressure of 15 Pa to 60 Pa for a freeze-drying period of 6 h to 24 h, and optionally, pre-freezing at a temperature of -80 °C to -76 °C for a time period of 20 h to 24 h before the freeze-drying.

Method F2: pre-freezing at a temperature of -80 °C to -76 °C for a time period of 20 h to 24 h, and drying at a temperature of 20° C to 30 °C and a pressure of 38 Pa to 42 Pa for a time period 45 h to 50 h.

Through the steps (1), (2), (3) and (4), a decellularized fish skin matrix was prepared, which can be used as a biological patch, as a single-layer biological patch. When rinsing or flushing was involved, it can be performed three times, with each session lasting 5 min to 10 min.

The operational parameters for Examples 1 to 12 can be referred to Table 1. Examples 1 to 10 all relate to single-layer biological patches. Examples 11 and 12 relate to multi-layer biological patches.

Table 1.

| No. | Degreasing | Pigment removal (decolorization) | Decellularization (with water as solvent) | Freeze-drying |
|-----|------------|----------------------------------|-------------------------------------------|---------------|
| Example 1 | Running water, 4 wt% starch aqueous solution | Acidic regulator 0.2 wt% acetic acid solution (pH 3.04) | 0.5 wt% Triton X-100 aqueous solution | Freeze-drying (-80°C to -50°C) |
| Example 2 | Running water, 2 wt% sodium carbonate aqueous solution | Acidic regulator 0.05 M acetic acid (about pH 2.96, 0.3 wt%) | 2 wt% Triton X-100 aqueous solution | Freeze-drying (-80°C to -50°C) |
| Example 3 | Running water, 3 wt% starch aqueous solution, 0.5 wt% SDS | Acidic reagent 0.5M acetic acid (pH 2.47, 3 wt%) | Alkaline reagent (0.3 wt% sodium carbonate aqueous solution) | Freeze-drying (-80°C to -50°C) |
| Example 4 | Running water, 2 wt% sodium carbonate aqueous solution, 0.5 wt% SDS aqueous solution | Acidic regulator 0.1 wt% acetic acid aqueous solution and 0.1 wt% citric acid aqueous solution in sequence | 0.5% Triton X-100 aqueous solution and alkaline reagent (0.01M sodium bicarbonate aqueous solution) in sequence | Freeze-drying (-80°C to -50°C) |
| Example 5 | Running water, 4 wt% starch aqueous solution | Penetration enhancer 0.5 wt% SDS aqueous solution | 0.5 wt% Triton X-100 aqueous solution | Freeze-drying (-80°C to -50°C) |
| Example 6 | Same as Example 2 | | | Microwave puffing, freeze-drying (-56°C) |
| Example 7 | Same as Example 3 | | | Pre-freezing (-80°C to -76°C), drying (20°C) |

(continued)

| No. | Degreasing | Pigment removal (decolorization) | Decellularization (with water as solvent) | Freeze-drying |
|---|---|---|---|---|
| Example 8 | Same as Example 3 | | | Pre-freezing (-80°C to -76°C), freeze-drying (-56°C) |
| Example 9 | Same as Example 1 | | | Microwave puffing, freeze-drying (-56°C) |
| Example 10 | Same as Example 1 | | | Pre-freezing (-80°C to -76°C), drying (-56°C) |
| Example 11 | Multi-layer patch prepared from single-layer biological patches of Example 1 using adhesive method | | | |
| Example 12 | Multi-layer patch prepared from single-layer biological patches of Example 1 using sewing method Stitching, multi-layer patch | | | |
| Comparative Example 1 | Running water, 3 wt% starch aqueous solution | Bleach (5% $H_2O_2$ and 0.1 % NaOH) | 0.5 wt% Triton X-100 aqueous solution | Freeze-drying (-80°C to -50°C) |
| Comparative Example 2 | 2 wt% sodium carbonate aqueous solution, running water | Bleach (5% $H_2O_2$ and 0.1 % NaOH) | 2 wt% Triton X-100 aqueous solution | Freeze-drying (-80°C to -50°C) |
| Comparative Example 3 | Running water, 3 wt% starch aqueous solution | Bleach (5% $H_2O_2$ and 0.1 % NaOH) | Alkaline reagent (0.09 wt% sodium carbonate aqueous solution) | Freeze-drying (-80°C to -50°C) |
| Comparative Example 4 | Washing directly with water | / | / | Freeze-drying (-80°C to -50°C) |
| Comparative Example 5 | Washing directly with water | / | / | Supercritical $CO_2$ treatment |

Example 1

Fish skin was taken out from a -20 °C freezer. After thawing, the yellow grease visible on the skin surface was flushed with running tap water until it was completely removed and invisible to the naked eye. During flushing, the water was maintained at a certain flow rate, and the flushing was performed for 10 min. Then the fish skin was washed with a 4 wt% starch aqueous solution three times to remove surface fat. The degreased fish skin was soaked in a 0.2 wt% acetic acid aqueous solution for 2 h to remove surface melanin. After decolorization, the fish skin was soaked in a 0.5 wt% Triton X-100 aqueous solution for 24 h for decellularization. Subsequently, the fish skin was freeze-dried using Method F1 at -56 °C and 28 Pa for 8 h to obtain a biological patch to be tested.

Example 2

Fish skin was thawed and flushed with running tap water using the same method as in Example 1, and then washed with a 2 wt% sodium carbonate aqueous solution to remove surface fat. The washing was repeated three times. The degreased fish skin was soaked in a 0.3 wt% acetic acid aqueous solution under shaking on a shaker for 2 min to remove surface melanin. After decolorization, the fish skin was soaked in a 2 wt% Triton X-100 aqueous solution under shaking on a shaker for 6 h for decellularization. Subsequently, the fish skin was freeze-dried using Method F1 at -56 °C and 28 Pa for 8 h to obtain a biological patch to be tested.

Example 3

Fish skin was thawed and flushed with running tap water using the same method as in Example 1, and then washed with a 3 wt% starch aqueous solution and a 0.5 wt% sodium dodecyl sulfate aqueous solution separately to remove surface fat. The washing was repeated three times. The degreased fish skin was soaked in a 3 wt% acetic acid aqueous solution under shaking on a shaker for 2 min to remove surface melanin. After decolorization, the fish skin was soaked in a 0.3 wt% sodium carbonate aqueous solution under shaking on a shaker for 24 h for decellularization. Subsequently, the fish skin was freeze-dried using Method F1 at -56 °C and 28 Pa for 8 h to obtain a biological patch to be tested.

Example 4

Fish skin was thawed and flushed with running tap water using the same method as in Example 1, and then washed with a 2 wt% sodium carbonate aqueous solution and a 0.5 wt% sodium dodecyl sulfate aqueous solution separately to remove surface fat. The washing was repeated three times. The degreased fish skin was soaked in a 0.1 wt% acetic acid aqueous solution and a 0.1 wt% citric acid aqueous solution separately for 2 min under shaking on a shaker to remove surface melanin. After decolorization, the fish skin was soaked in a 0.5 wt% Triton X-100 aqueous solution and a 0.09 wt% sodium bicarbonate aqueous solution separately for 12 h under shaking on a shaker for decellularization. Subsequently, the fish skin was freeze-dried using Method F1 at -56 °C and 28 Pa for 8 h to obtain a biological patch to be tested.

Example 5

Fish skin was thawed and flushed with running tap water using the same method as in Example 1, and then washed with a 4 wt% starch aqueous solution to remove surface fat. The washing was repeated three times. The degreased fish skin was soaked in a 0.3 wt% sodium dodecyl sulfate aqueous solution to remove surface melanin. After decolorization, the fish skin was soaked in a 0.5 wt% Triton X-100 aqueous solution for 24 h for decellularization. Subsequently, the fish skin was freeze-dried using Method F1 at -56 °C and 28 Pa for 8 h to obtain a biological patch to be tested.

Example 6

Degreasing, decolorization and decellularization were performed using the same methods as in Example 2. After the decellularization, the fish skin was soaked in castor oil, taken out and placed in a microwave generator for puffing treatment at a power of 700 W for 5 s, and then freeze-dried at -56 °C and 28 Pa for 8 h, using Method F1.

Example 7

Degreasing, decolorization and decellularization were performed using the same methods as in Example 3. After the decellularization, the fish skin was pre-frozen at -80 °C to -76 °C for 24 h, followed by drying at 20 °C and 40 Pa for 48 h, using Method F2.

Example 8

Degreasing, decolorization and decellularization were performed using the same methods as in Example 3. After the decellularization, the fish skin was pre-frozen at -80 °C to -76 °C for 24 h, followed by freeze-drying at -56 °C and 28 Pa for 8 h, using Method F1.

Example 9

Degreasing, decolorization and decellularization were performed using the same methods as in Example 1. After the decellularization, the fish skin was soaked in castor oil, taken out and placed in a microwave generator for puffing treatment at a power of 700 W for 5 s, and then freeze-dried at -56 °C and 28 Pa for 8 h, using Method F1.

Example 10

Degreasing, decolorization and decellularization were performed using the same method as in Example 1. After the decellularization, the fish skin was pre-frozen at -80 °C to -76 °C for 24 h, followed by freeze-drying at -56 °C and 28 Pa for 8 h, using Method F1.

Example 11

Three single-layer biological patches (single-layer fish skin) prepared according to the method of Example 1, with identical dimensions and good integrity, were selected. A silk fibroin aqueous solution with a mass-volume concentration of 20% (w/v) was evenly applied to a surface of the first fish skin at a coating amount of 0.05 mL/cm$^2$ with a coating rod. The second fish skin was then covered and adhered onto the surface with the adhesive solution. The third fish skin was adhered onto the second fish skin by repeating the above steps.

The adhered fish skins were placed between stainless steel plates of a pressing device, with the parameters set as follows: temperature of 37 °C, pressure of 0.3 MPa, and time of 1 h, and then dried in a vacuum drying oven, with the parameters set as follows: temperature of 25 °C, time of 2 h, and pressure of 1 Pa.

Example 12

Two single-layer biological patches (single-layer fish skin) prepared according to the method in Example 1, with identical dimensions of 50 mm × 80 mm and good integrity, were selected. Using a silk thread and an automatic sewing machine, the patches were sewn together at specific positions, which is stitching along a circle 1 cm from the outer contour of the fish skin, followed by diagonal stitching, thereby tightly bonding the two fish skin patches. The resulting product was set aside for later use.

Comparative Example 1

Fish skin was thawed and flushed with running tap water using the same method as in Example 1, and then washed with a 3 wt% starch aqueous solution to remove surface fat. The washing was repeated three times. The degreased fish skin was soaked in a mixed aqueous solution containing 5 wt% hydrogen peroxide and 0.1 wt% sodium hydroxide for bleaching to remove surface melanin. After decolorization, the fish skin was soaked in a 0.5 wt% Triton X-100 aqueous solution for 24 h for decellularization. Subsequently, the fish skin was freeze-dried using Method F1 at -56 °C and 28 Pa for 8 h.

Comparative Example 2

Fish skin was thawed and flushed with running tap water using the same method as in Example 1, and then washed with a 2 wt% sodium carbonate aqueous solution to remove surface fat. The washing was repeated three times. The degreased fish skin was soaked in a mixed aqueous solution containing 5 wt% hydrogen peroxide and 0.1 wt% sodium hydroxide for bleaching to remove surface melanin. After decolorization, the fish skin was soaked in a 2 wt% Triton X-100 aqueous solution under shaking on a shaker for 6 h for decellularization. Subsequently, the fish skin was freeze-dried using Method F1 at -56 °C and 28 Pa for 8 h.

Comparative Example 3

Fish skin was thawed and flushed with running tap water using the same method as in Example 1, and then washed with a 3 wt% starch aqueous solution to remove surface fat. The washing was repeated three times. The degreased fish skin was soaked in a mixed aqueous solution containing 5 wt% hydrogen peroxide and 0.1 wt% sodium hydroxide for bleaching to remove surface melanin. After decolorization, the fish skin was soaked in a 0.09 wt% sodium carbonate aqueous solution for decellularization. Subsequently, the fish skin was freeze-dried using Method F1 at -56 °C and 28 Pa for 8 h.

Comparative Example 4

Fish skin was taken out from a -20 °C freezer. After thawing, the yellow grease visible on the skin surface was flushed with running tap water until it was completely removed and invisible to the naked eye. During flushing, the water was maintained at a certain flow rate, and the flushing was performed for 10 min.

Subsequently, the fish skin was freeze-dried using Method F1 at -56 °C and 28 Pa for 8 h to obtain a biological patch to be tested.

Comparative Example 5

Fish skin was taken out from a -20°C freezer. After thawing, the yellow grease visible on the surface was flushed with running tap water until it was completely removed and invisible to the naked eye. During flushing, the water was maintained at a certain flow rate, and the flushing was performed for 10 min.

Using an intermittent supercritical cleaning tank to increasing pressure and temperature of liquid $CO_2$, output from a $CO_2$ storage tank, to specified operating values (pressure >7.38 MPa, temperature >31.1°C) via a high-pressure pump and a heater to form supercritical fluid, which was then introduced into a cleaning tank. The supercritical $CO_2$ fully contacted the fish skin in the material basket of the cleaning tank, where grease in the fish skin was eluted through permeation and dissolution by the supercritical $CO_2$. The mixture of supercritical $CO_2$ and grease was appropriately depressurized via a pressure relief valve and separated in a separation tank.

Other Comparative Examples

Other bleaches such as sodium hypochlorite and peracetic acid were also used for decolorization. The results showed that due to their strong oxidizing effects, the fish skin raw materials were deactivated, rendering them unsuitable for further experiments.

II. Characterization and Testing Analysis Methods

1. Chemical Composition Testing

(i) Determination of Fatty Acid Composition and Content

Testing Method: the contents of different fatty acid components in the samples can be determined by using a gas chromatography method in accordance with "GB 5009.168-2016 National Food Safety Standard - Determination of fatty acids in foods".

The test results can be found in Table 2 and Table 3.

The determination can be performed through methyl esterification of 37 fatty acids, and the corresponding tested methyl esterified components are: methyl butyrate (CAS: 623-42-7), methyl hexanoate (CAS: 106-70-7), methyl octanoate (CAS: 111-11-5), methyl decanoate (CAS: 110-42-9), methyl undecanoate (CAS: 1731-86-8), methyl laurate (CAS: 111-82-0), methyl tridecanoate (CAS: 1731-88-0), methyl myristate (CAS: 124-10-7), methyl myristoleate (CAS: 56219-06-8), methyl pentadecanoate (CAS: 7132-64-1), methyl cis-10-pentadecenoate (CAS: 90176-52-6), methyl palmitate (CAS: 112-39-0), methyl palmitioleate (CAS: 1120-25-8), methyl heptadecanoate (CAS: 1731-92-6), methyl cis-10-heptadecenoate (CAS: 75190-82-8), methyl stearate (CAS: 112-61-8), methyl elaidate (CAS:), methyl oleate (CAS: 112-62-9), methyl linolelaidate (CAS: 2566-97-4), methyl linoleate (CAS: 112-63-0), methyl arachidate (CAS: 1120-28-1), methyl-gama-linolenate (CAS: 16326-32-2), methyl 11-eicosenoate (CAS:), methyl linolenate (CAS: 301-00-8), methyl heneicosanoate (CAS: 6064-90-0), methyl cis,cis-11,14-eicosadienoate (CAS: 2463-02-7), methyl behenate (CAS: 929-77-1), methyl homogamma linolenate (CAS: 21061-10-9), methyl erucate (CAS: 1120-34-9), methyl cis-11,14,17-eicosatrienoate (CAS: 55682-88-7), methyl tricosanoate (CAS: 2433-97-8), methyl arachidonate (CAS: 2566-89-4), methyl cis-13,16-docosadienoate (CAS: 61012-47-3), methyl lignocerate (CAS: 2442-49-1), methyl cis-5,8,11,14,17-eicosapentaenoate (CAS: 2734-47-6), methyl cis-15-tetracosenoate (CAS: 2733-88-2), and methyl 4,7,10,13,16,19-docosahexaenoate (CAS: 301-01-9).

(2) Determination of Fat Content (Total Fat Content)

Testing Method: The total fat content was determined according to the "Fat Content Determination Method" specified in Section 5, Part I of "GB 5009.168-2016 National Food Safety Standard - Determination of fatty acids in foods". Specifically, the fish skin was treated with supercritical $CO_2$ using the Soxhlet extraction method, and the fat content in the fish skin before and after the treatment was determined. First, the mass of the test sample was measured. The sample was then placed in an extraction cartridge, and petroleum ether was used for the extraction experiment. After 6 h to 10 h, fat extraction was completed. The receiving flask was weighed to obtain the fat content. The fat content in the sample was calculated using the following formula:

$$X = \frac{m1 - m0}{m2} \times 100$$

where X represents the fat content of the sample (g/100g), m1 represents the total mass of the receiving flask and the extracted fat after constant weight, m0 represents the mass of the receiving flask, and m2 represents the mass of the sample.

2. Morphology and Porosity Testing

(1) Surface Morphology Testing

Instrument and Method: The surface morphology of the fish skin was observed using a scanning electron microscope (SEM). Specifically, the test samples were sputtered with gold and placed in the vacuum chamber of the SEM for surface morphology observation after vacuum evacuation.
SEM Instrument: Prisma E, probe model ETD, mode SE, working distance: 12 mm, magnification: 100X to 3000X, accelerating voltage: 15 kV, beam spot diameter: 4.5 nm.

(2) Porosity Testing

Testing Method: The porosity of the samples was measured according to "GB/T 21650.1-2008 Pore size distribution and porosity of solid materials by mercury porosimetry and gas adsorption - Mercury porosimetry".

3. Water Contact Angle

Testing Method: The water contact angle of the samples was measured using a water contact angle meter. The sample was placed under the contact angle meter, and a 20 $\mu$L water droplet was dropped from a syringe onto the sample surface, which was photographed to calculate the contact angle. This process was repeated three times, and the average value was taken.

4. Surface Roughness Testing

Testing Method: The surface roughness of the samples was evaluated using a roughness tester according to the method in "GB/T 10610-2009 Geometrical product specification (GPS) surface texture: Profile method - Rules and procedures for the assessment of surface texture".

5. Mechanical Performance Testing: Tensile Breaking Force Test Method

The sample was cut into a 100 mm $\times$ 15 mm strip specimen. The specimen was clamped with clamps and tested using a universal material mechanical testing machine, with a gauge length of 50 mm, width of 15 mm, and speed of 20 mm/min until tensile fracture occurs. The maximum tensile breaking force was recorded.

6. Cytotoxicity Testing

Test Samples: Biological patches (single-layer or multi-layer) prepared in the examples and comparative examples. Control group: Serum-free culture medium. Cell type: Mouse fibroblast cells (L929). Testing method was referred to standard GB/T 16886.5-2017.

(1) The sample was cut into a 30 mm $\times$ 30 mm specimen.
(2) The specimen was placed in serum-free culture medium at an extraction ratio of 3 cm$^2$/mL and incubated in a $CO_2$ incubator at 37 °C for 72 h.
(3) The cells were seeded at a density of $1 \times 10^4$ cells/100$\mu$L into 96-well plate and incubated for 24 h. The serum-free culture medium was removed, and 100 $\mu$L of the extract was added. The control group was only added with 100 $\mu$L of serum-free culture medium.
(4) After the 24 h incubation, the extract and serum-free culture medium were removed, and a phosphate-buffered saline (PBS) solution containing 10% CCK8 (using a CCK8 kit) was added prior to incubation in a $CO_2$ incubator for 2 h.
(5) 100 $\mu$L of the supernatant was pipetted into another 96-well plate, which was placed in a microplate reader to measure and record the absorbance (A) at 450 nm. The absorbance was normalized based on the control group A0 as 100%.

(6) The cytotoxicity index was calculated according to: Cytotoxicity Index = 100% × A/A0, which can be considered as the cell viability. A higher value indicates lower cytotoxicity.

7. Degradation Performance Testing Method

The fish skin patches obtained from the examples and comparative examples were fully soaked individually in PBS at a ratio of 0.1g/10mL and placed on a shaker at 37±1 °C for degradation test. The time required for complete degradation of the fish skin (i.e., when the sheet broke down into fragments) was observed.

The criterion for "complete fragments" or "complete degradation" was that the sample was completely dissolved in the solution or no visible block-like sample remains.

The "degradation ratio of the biological patch after 7 days of degradation" was obtained by using the following method. At day 7, the sample was removed, freeze-dried, and weighed. The mass loss was obtained by comparing the masses before and after degradation, and the percentage of mass loss to initial mass was recorded as the degradation ratio.

8. Residual DNA

The residual DNA content was a critical indicator for evaluating animal-derived tissue materials. Detailed operation was as follows: 10 mg of the patch was weighed, crushed in liquid nitrogen, and placed in a test tube without DNA. DNA extraction was performed in accordance with the instructions of a DNA extraction kit. The total amount of DNA was measured using Eppendorf BioPhotometer D30 (Germany), and the DNA content was calculated based on the pre-weighed sample weight.

9. Inflammatory Response Detection Method

Subcutaneous implantation experiments were conducted in rats. At specific implantation time points (Weeks 2, 4, 6 and 8), the degree of fibrosis and inflammation around the implant site was observed using H&E staining. The detailed steps were as follows: The tissue was placed in a 10% formaldehyde aqueous solution for at least 24 h for fixing, followed by conventional paraffin embedding. The tissue was sliced at a thickness of 4 $\mu$m. After drying, the slices were dewaxed and rehydrated, and H&E stained. The detailed HE staining procedure included: (1) Dewaxing and Rehydration: Xylene I for 20 min → Xylene II for 20 min → Absolute ethanol for 10 min → 90% ethanol for 3 min → 80% ethanol for 3 min → 70% ethanol for 3 min. (2) Hematoxylin Staining: The tissue was soaked in hematoxylin solution for 3 min, rinsed in water to remove floating hematoxylin, differentiated in 1% hydrochloric acid ethanol for 2 s until the tissue turned red, rinsed in water three times to remove hydrochloric acid ethanol, and soaked in water for 15 min. (3) Dehydration: 75% ethanol dehydration for 2 s to 3 s → 95% ethanol for 2 s to 3 s. (4) Eosin Staining: The tissue was soaked in eosin solution for 3 min and rinsed in water to remove floating eosin. (5) Dehydration: 100% ethanol dehydration for 2 s to 3 s. (6) Mounting: The tissue was sealed with neutral resin, observed and photographed.

90% ethanol, 80% ethanol, and 70% ethanol refer to mixed solvents respectively composed of ethanol at 90%, 80% and 70%, mixed with water.

III. Characterization and Testing Analysis Results

1. Fat Composition Testing

(1) Fatty acid type and content testing, and (2) Fat content testing

Using *ictalurus punctatus* as the skin source, more than 20 fatty acid components were detected in the biological patches prepared from the examples and comparative examples, primarily including butyric acid, lauric acid, myristic acid, palmitic acid, stearic acid, arachidic acid (eicosanoic acid), lignoceric acid (tetracosanoic acid), palmitoleic acid, oleic acid (octadecenoic acid), linoleic acid (octadecadienoic acid), $\gamma$-linolenic acid, linolenic acid ALA ($\alpha$-linolenic acid), arachidonic acid, eicosatrienoic acid (icosatrienoic acid), eicosadienoic acid (icosadienoic acid), eicosenoic acid (cis-11-eicosenoic acid), eicosapentaenoic acid EPA, dodecenoic acid, docosadienoic acid, docosahexaenoic acid DHA, and tetracosenoic acid, etc. The carbon atom number of these fatty acids was in a range from 4 to 24.

The fatty acid composition of Example 1 is shown in Tables 2 and 3. The mass percentages of some fatty acid components in the fish skin raw material and the prepared biological patch are shown in Table 3, based on the dry weight of the biological patch. The content of other fatty acid types can be analyzed in a similar manner.

The unsaturated fatty acids accounted for 81.9% of the raw material sample, and accounted for 77.3% of the patch sample, indicating that a relatively high proportion of unsaturated fatty acids was maintained after treatment. In the decellularized fish skin matrix, the EPA content was 429 mg/kg, and the DHA content was 449 mg/kg.

EPA and DHA are long-chain unsaturated fatty acids belonging to the ω-3 unsaturated fatty acids, which are a class of essential polyunsaturated fatty acids. Studies have found that DHA and EPA can inhibit inflammatory cells from producing pro-inflammatory cytokines, competitively reduce the production of inflammatory mediators, regulate immune responses, thereby alleviating excessive inflammatory responses

The biological patches provided in the examples had low fat content, which can prevent potential risks such as infection, tumorigenesis, and immune rejection.

The first fatty acid included at least butyric acid, caprylic acid, lauric acid, myristic acid, pentadecanoic acid, palmitic acid, stearic acid, arachidic acid, palmitoleic acid, oleic acid, γ-linolenic acid, linolenic acid ALA, eicosapentaenoic acid EPA, erucic acid, docosahexaenoic acid DHA, and tetracosenoic acid. The content of the first fatty acid can be estimated based on the total amount of the above fatty acids.

The second fatty acid includes at least linoleic acid and arachidonic acid based on ω-6 polyunsaturated fatty acids. The amount of the second fatty acid can be estimated based on the total amount of linoleic acid and arachidonic acid.

Table 2. Testing results of fatty acid types and contents

| Type of fatty acid | Name of fatty acid | Number of carbon atoms | Content in fish skin raw material (mg/kg) | Content in biological patch (mg/kg) | Mass proportion in fatty acids, for fish skin raw material | Mass proportion in fatty acids, for biological patch | Percentage of Mass loss |
|---|---|---|---|---|---|---|---|
| Saturated Fatty Acids | | | | | 18.07% | 22.69% | |
| Butyric acid | | 4 | 627.43 | 684.17 | 0.48% | 1.97% | -9.04% |
| Caprylic acid | | 8 | 56.07 | / | 0.04% | / | |
| Dodecanoic acid | Lauric acid | 12 | 58.63 | 46.86 | 0.05% | 0.13% | 20.08% |
| Tetradecanoic acid | Myristic acid | 14 | 723.8 | 219.76 | 0.56% | 0.63% | 69.64% |
| Pentadecanoic acid | | 15 | 134.93 | / | 0.10% | / | |
| Hexadecanoic acid | Palmitic acid | 16 | 16863.92 | 5221.16 | 12.98% | 15.03% | 69.04% |
| Heptadecanoic acid | | 17 | 97.96 | / | 0.08% | / | |
| Octadecanoic acid | Stearic acid | 18 | 4052.98 | 930.24 | 3.12% | 2.68% | 77.05% |
| Eicosanoic acid | Arachidic acid | 20 | 249.2 | 230.65 | 0.19% | 0.66% | 7.44% |
| Tetracosanoic acid | Lignoceric acid | 24 | 617.39 | 546.35 | 0.48% | 1.57% | 11.51% |
| Unsaturated Fatty Acids | | | | | 81.93% | 77.31% | |
| Hexadecenoic acid | Palmitoleic acid | 16 | 1732.17 | 306.98 | 1.33% | 0.88% | 82.28% |
| Octadecenoic acid | Oleic acid | 18 | 58831.04 | 14167.24 | 45.28% | 40.79% | 75.92% |
| Octadecadienoic acid | Linoleic acid (LA) | 18 | 34873.84 | 8266.31 | 26.84% | 23.80% | 76.30% |
| Octadecatrienoic acid | γ-linolenic acid | 18 | 967.69 | 310.83 | 0.74% | 0.89% | 67.88% |
| Octadecatrienoic | ALA | 18 | 3026.06 | 708.27 | 2.33% | 2.04% | 76.59% |

(continued)

| Type of fatty acid | Name of fatty acid | Number of carbon atoms | Content in fish skin raw material (mg/kg) | Content in biological patch (mg/kg) | Mass proportion in fatty acids, for fish skin raw material | Mass proportion in fatty acids, for biological patch | Percentage of Mass loss |
|---|---|---|---|---|---|---|---|
| acid (α-linolenic acid, ALA) | | | | | | | |
| Eicosenoic acid | | 20 | 1394.68 | 462.79 | 1.07% | 1.33% | 66.82% |
| Eicosadienoic acid | | 20 | 1068.62 | 346.72 | 0.82% | 1.00% | 67.55% |
| Eicosatrienoic acid | | 20 | 1777.77 | 618.6 | 1.37% | 1.78% | 65.20% |
| Eicosatetraenoic acid (arachidonic acid) | AA | 20 | 926.84 | 378.8 | 0.71% | 1.09% | 59.13% |
| Eicosapentaenoic acid (EPA) | EPA | 20 | 66.79 | 428.7 | 0.05% | 1.23% | -541.86% |
| Docosadecenoic acid | Erucic acid | 22 | 557.49 | 278.46 | 0.43% | 0.80% | *50.05%* |
| Docosadienoic acid | | 22 | 326.37 | 39.37 | 0.25% | 0.11% | 87.94% |
| Docosahexaenoic acid (DHA) | DHA | 22 | 841.22 | 449.27 | 0.65% | 1.29% | 46.59% |
| Tetracosenoic acid | | 24 | 59.63 | 89.2 | 0.05% | 0.26% | -49.59% |

Table 3. Summary of fatty acid contents (converted to mass percentage)

| Type of fatty acid | Name or characteristic of fatty acid | Number of carbon atoms | Mass proportion in fish skin raw material | Mass proportion in biological patch |
|---|---|---|---|---|
| Total fat content | / | / | 20.2% | 5.6% |
| First fatty acid | | / | 8.89% | 2.41% |
| Second fatty acid | | / | 3.58% | 0.86% |
| Mass ratio of first fatty acid to second fatty acid | | | 2.48 | 2.78 |
| Linoleic acid (LA) | Octadecadienoic acid | 18 | 3.49% | 0.83% |
| Arachidonic acid (AA) | Eicosatetraenoic acid | 20 | 927 ppm | 379 ppm |
| ω-3 polyunsaturated fatty acids | ALA, EPA, and DHA | 18, 20, 22 | 0.39% (3934 ppm) | 0.16% (1586 ppm) |
| ALA | Octadecatrienoic acid | 18 | 3026 ppm | 708 ppm |
| EPA | Eicosapentaenoic acid | 20 | 67 ppm | 429 ppm |
| DHA | Docosahexaenoic acid | 22 | 841 ppm | 450 ppm |
| Butyric acid + Caprylic acid + Pentadecanoic acid | | | 818 ppm | 684 ppm |

(continued)

| Type of fatty acid | Name or characteristic of fatty acid | Number of carbon atoms | Mass proportion in fish skin raw material | Mass proportion in biological patch |
|---|---|---|---|---|
| C$_{4-24}$ fatty acids | Saturated + Unsaturated | 4 to 24 | 12.99% | 3.47% |
| Unsaturated C$_{4-24}$ fatty acids | | 4 to 24 | 10.6% | 2.69% |
| C$_{4-15}$ fatty acids | Saturated + Unsaturated | 4 to 15 | 0.16% | 0.10% |
| C$_{16-24}$ saturated fatty acids | Saturated | 16 to 24 | 2.19% | 0.69% |
| Palmitic acid | Saturated | 16 | 1.69% | 0.52% |
| Stearic acid | Saturated | 18 | 4053 ppm | 930 ppm |
| C$_{16-24}$ unsaturated fatty acids | Unsaturated | 16 to 24 | 10.65% | 2.69% |
| C$_{16}$ unsaturated fatty acids | Palmitoleic acid | 16 | 1732 ppm | 307 ppm |
| C$_{18}$ unsaturated fatty acids | Monoenoic acids, dienoic acids, trienoic acids | 18 | 9.77% | 2.35% |
| C$_{20}$ unsaturated fatty acids | Monoenoic acids, dienoic acids, trienoic acids, tetraenoic acids, pentaenoic acids | 20 | 5234 ppm | 2236 ppm |
| C$_{22}$ unsaturated fatty acids | Monoenoic acids, dienoic acids, hexaenoic acids | 22 | 1725 ppm | 767 ppm |
| C$_{24}$ unsaturated fatty acids | Monoenoic acids | 24 | 60 ppm | 89 ppm |
| Comparison Item | | | Mass proportion in fatty acids, for fish skin raw material | Mass proportion in fatty acids, for biological patch |
| First fatty acid | | | 68.38% | 69.31% |
| Second fatty acid | | | 27.55% | 24.89% |

The fatty acid composition analysis of the examples is shown in Table 4 and Table 5.

Table 4. Summary of fatty acid composition

| Type of fatty acid | Mass content in the dry weight of biological patch (mg/kg) | | | |
|---|---|---|---|---|
| Type of fatty acid | Examples 1 to 10 (range) | Comparative Example 1 | Comparative Example 2 | Comparative Example 3 |
| Butyric acid | 376.2 to 684.17 | 501.94 | 451.74 | 533.31 |
| Caprylic acid | 0 to 39.34 | 44.86 | 40.73 | 50.23 |
| Dodecanoic acid | 25.73 to 49.67 | 46.90 | 42.79 | 48.66 |
| Tetradecanoic acid | 125.26 to 598.75 | 579.04 | 506.66 | 592.52 |
| Pentadecanoic acid | 0 to 116.07 | 107.94 | 94.45 | 115.67 |
| Hexadecanoic acid | 2976.06 to 14165.69 | 13491.93 | 12141.48 | 14332.43 |
| Heptadecanoic acid | 0 to 68.56 | 100.14 | 74.74 | 89.01 |
| Octadecanoic acid | 539.54 to 3404.50 | 3242.38 | 2958.67 | 3285.21 |

(continued)

| Type of fatty acid | Mass content in the dry weight of biological patch (mg/kg) | | | |
|---|---|---|---|---|
| Type of fatty acid | Examples 1 to 10 (range) | Comparative Example 1 | Comparative Example 2 | Comparative Example 3 |
| Eicosanoic acid | 33.70 to 230.65 | 260.10 | 180.12 | 286.16 |
| Tetracosanoic acid | 450.43 to 546.35 | 543.30 | 555.65 | 650.14 |
| Hexadecenoic acid | 184.16 to 1489.66 | 1385.74 | 1212.52 | 1405.43 |
| Octadecenoic acid | 8216.99 to 49418.55 | 48214.45 | 42946.54 | 45888.21 |
| Octadecadienoic acid (linoleic acid) | 4565.32 to 23586.69 | 28247.81 | 25109.16 | 26852.65 |
| Octadecatrienoic acid | 174.06 to 822.53 | 725.76 | 735.44 | 764.06 |
| Octadecatrienoic acid ($\alpha$-lino-lenic acid, ALA) | 403.71 to 2693.06 | 2602.41 | 2178.76 | 2330.06 |
| Eicosenoic acid | 314.79 to 1199.42 | 1200.33 | 1046.10 | 1342.97 |
| Eicosadienoic acid | 367.89 to 929.69 | 961.75 | 769.40 | 833.53 |
| Eicosatrienoic acid | 433.02 to 1545.99 | 1564.43 | 1279.99 | 1404.43 |
| Eicosatetraenoic acid (arachidonic acid) | 340.67 to 873.12 | 1264.73 | 695.13 | 829.15 |
| Eicosapentaenoic acid (EPA) | 124.32 to 564.32 | 302.15 | 435.70 | 104.53 |
| Docosadecenoic acid | 155.93 to 479.44 | 457.20 | 418.11 | 423.17 |
| Docosadienoic acid | 23.22 to 270.88 | 244.77 | 228.45 | 163.15 |
| Docosahexaenoic acid (DHA) | 247.08 to 731.86 | 672.39 | 630.91 | 670.20 |
| Tetracosenoic acid | 44.60 to 89.2 | 129.20 | 135.15 | 178.20 |

Table 5

| Summary of indicators | Mass percentage or mass proportion in the dry weight of biological patch | | | |
|---|---|---|---|---|
| | Examples 1 to 10 | Comparative Example 1 | Comparative Example 2 | Comparative Example 3 |
| Total fat content | 3.4% to 18.3% | 17.1% | 15.1% | 17.2% |
| First fatty acid | 1.4% to 7.8% | 7.3% | 6.5% | 7.1% |
| Second fatty acid | 0.49% to 1.89% | 3.0% | 2.6% | 2.8% |
| Mass ratio of first fatty acid to second fatty acid | 2.43 to 2.85 | 2.46 | 2.53 | 2.56 |
| $\omega$-3 polyunsaturated fatty acids | 775 ppm to 3989 ppm | 3576 ppm. | 3245 ppm | 3104 ppm |
| Saturated fatty acids | 0.45% to 2.0% | 1.9% | 1.7% | 2.0% |
| Unsaturated fatty acids | 1.6% to 9.2% | 8.8% | 7.8% | 8.3% |
| Butyric acid + Caprylic acid + Pentadecanoic acid | 376.2 ppm to 667.33 ppm | 654.74 ppm | 586.92 ppm | 699.21 ppm |

(continued)

| Comparison Item | Mass percentage or mass proportion in fatty acids contained in the biological patch | | | |
|---|---|---|---|---|
| | Examples 1 to 10 | Comparative Example 1 | Comparative Example 2 | Comparative Example 3 |
| First fatty acid | 68.3% to 69.6% | 68.2% | 69.4% | 68.9% |
| Second fatty acid | 23.9% to 28.6% | 28.0% | 27.4% | 27.2% |

The mass percentage or mass proportion of the specified fatty acid in fatty acids contained in the biological patch in Table 5 and the mass proportion in fatty acids in Table 2 were estimated based on using the total content of 37 fatty acids within the detection range as the amount of "fatty acids contained in the biological patch".

2. Morphology and Porosity Test

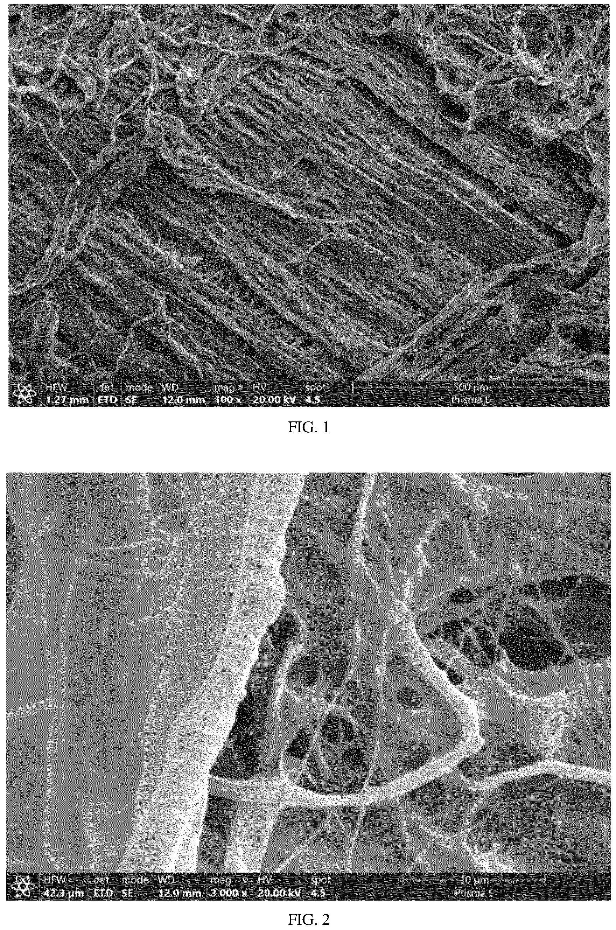

The surface morphology of the rougher inner surface of the biological patch in Example 1 is shown in FIGs. 1 and 2. Under low magnification (100X, see FIG. 1), a regular structure of interwoven fibers can be clearly observed. The fiber diameters primarily ranged from several micrometers ($\mu$m) to tens of micrometers, including collagen fibers with diameters of 2 $\mu$m to 30 $\mu$m. Under high magnification (3000X, see FIG. 2), micropores are also observable, with pore diameters predominantly ranging from 2 $\mu$m to 5 $\mu$m. The regular arrangement of fibers facilitates orderly cell adhesion and proliferation, while the porous structure facilitates the exchange of nutrients and oxygen for implantation and repair, promoting cell growth. In the decellularized fish skin matrix prepared in this example, the pore size was close to the cell dimensions, falling within the submicron range. Studies have shown that when the pore sizes are in the submicron range (e.g., 1 $\mu$m to 10 $\mu$m), the matrix can influence cell behavior, being conducive to the adhesion, proliferation, and spreading of osteoblasts.

According to testing results, the porosity was 64%, exhibiting good hydrophilicity and a large specific surface area, allowing for a large contact area with the cells and tissues, as well as rapid material exchange.

In all examples, the fiber diameters were approximately from 2 $\mu$m to 30 $\mu$m, the pore sizes were predominantly ranged from 2 $\mu$m to 5 $\mu$m, and the porosities ranged from 55% to 75%, with most concentrated in a range from 62% to 68%.

In Comparative Examples 1 to 3, after decolorization with the strong oxidizing agent hydrogen peroxide, the surface structure of the fish skin was severely damaged, with no obvious fiber structure observed, recorded as "no fiber structure". A summary of the results for the comparative examples can be found in Table 6.

Table 6.

| No. | Collagen fiber diameter | Pore size | Porosity | Water contact angles of the first surface and the second surface | Surface roughness Ra ($\mu$m) of the first surface (front, outer surface) | Surface roughness Ra ($\mu$m) of the second surface (back, inner surface) |
|---|---|---|---|---|---|---|
| Comparative Example 1 | No fiber structure | No fiber structure | 75% | 20° to 30° | 10.20$\pm$3.18 | 20.34$\pm$1.12 |
| Comparative Example 2 | No fiber structure | No fiber structure | 72% | 20° to 30° | 13.45$\pm$2.56 | 23.23$\pm$4.28 |
| Comparative Example 3 | No fiber structure | No fiber structure | 78% | 20° to 30° | 15.27$\pm$4.33 | 26.53$\pm$4.34 |

3. Surface Hydrophilicity/Hydrophobicity and Wettability

The water contact angles of the first surface and the second surface of the biological patches in the examples were approximately from 51° to 90° (and all within 55° to 65°), and both surfaces exhibited excellent hydrophilicity. Over time, within 30 s to 60 s, water can penetrate into the decellularized fish skin matrix. Good hydrophilicity enabled the decellularized fish skin matrix to better absorb proteins upon contact, thereby promoting cell adhesion. The water absorption capacity of the decellularized fish skin matrix was excellent. The decellularized fish skin matrix can absorb about 3 to 5 times its own weight of water.

In the early stages of osseointegration, surface wettability can induce cell adhesion and enhance biocompatibility. The most favorable contact angle range for cell adhesion is believed to be 40° to 60°. Improved wettability leads to reduced albumin adsorption and increased fibronectin adsorption, which is beneficial for cell adhesion. Albumin is a non-adhesive protein, while fibronectin is an adhesive protein. In terms of wettability, the oxygen content on the surface and the polar components of surface energy play important roles in promoting cell proliferation, especially when the surface roughness is correspondingly increased. The intermolecular attractive forces that dominate the surface energy are generated by various intermolecular interactions, whose contributions to the total surface energy are additive.

In Comparative Examples 1 to 3, the contact angles of the two surfaces of the samples were all ranged from 20° to 30°, resulting in excessively high hydrophilicity of the second surface, which may be detrimental to cell adhesion, proliferation, and tissue repair.

4. Surface Roughness

The first surface refers to the outer surface that contacts the water environment, and the second surface refers to the inner surface that contacts the internal tissues of the fish. The testing results of surface roughness of the inner and outer surfaces of the examples can be found in Table 6.

In the examples, the surface roughness of the first surface was in a range from 8 μm to 20 μm, and the surface roughness of the second surface was in a range from 25 μm to 40 μm.

In Example 1 as an example, the surface roughness of the first surface of the biological patch was 13.95 ± 1.27 μm, and the surface roughness of the second surface was 33.23 ± 5.58 μm. The two surfaces exhibited a large difference in surface roughness, also revealing different cell adhesion states. Studies have shown that the surface with roughness in a range from 10 μm to 20 μm has a significant impact on cell compatibility, and surface roughness is one of the important factors controlling cell viability. Studies found that rat osteoblasts are more suitable for growing in areas with large surface roughness, and an increase in roughness can improve cell adhesion and growth behavior. Appropriately increasing the surface roughness can increase the contact area between the implant and blood cells, thereby improving the adhesion of platelets on the implant surface, creating favorable conditions for osteoblasts to migrate on the rough surface, and improving the contact state between osteoblasts and implant.

In Comparative Examples 1 to 3, the difference in roughness between the inner and outer surfaces was reduced.

5. Testing Results of Tensile Breaking Force, Cytotoxicity, and Degradation Performance

The testing results of the mechanical performance, cytotoxicity, and degradation performance of the examples and comparative examples can be found in Table 7.

The biological patches prepared in Examples 1 to 12 exhibited good mechanical performance including high tensile breaking force and high adhesion strength, low cytotoxicity, good biocompatibility, and moderate degradation performance, and thus were all suitable for soft tissue repair.

In Comparative Examples 1 to 3, the mechanical performance was deteriorated, degradation rate was significantly increased, and degradation period was short.

Table 7

| No. | Mechanical performance | Biocompatibility | Degradation performance | |
|---|---|---|---|---|
| No. | Tensile Breaking Force (N) | Cytotoxicity index (%) | Degradation period for the biological patch to be completely degraded | Degradation ratio of the biological patch at Day 7 (%) |
| Example 1 | 280.14±56.2 | 97 | 56 days | 12 |
| Example 2 | 316.12±13.25 | 96 | 55 to 60 days | 13 |
| Example 3 | 279.30±24.56 | 84 | 55 to 60 days | 10 |
| Example 4 | 240.23±25.10 | 88 | 55 to 60 days | 15 |
| Example 5 | 290±31.72 | 83 | 55 to 60 days | 14 |
| Example 6 | 360.20±26.25 | 93 | 55 to 60 days | 16 |
| Example 7 | 300.47±35.53 | 81 | 55 to 60 days | 16 |
| Example 8 | 287.15±24.99 | 82 | 55 to 60 days | 17 |

(continued)

| No. | Mechanical performance | Biocompatibility | Degradation performance | |
| --- | --- | --- | --- | --- |
| No. | Tensile Breaking Force (N) | Cytotoxicity index (%) | Degradation period for the biological patch to be completely degraded | Degradation ratio of the biological patch at Day 7 (%) |
| Example 9 | 338.29±29.25 | 94 | 55 to 60 days | 16 |
| Example 10 | 241.54±31.09 | 100.4 | 55 to 60 days | 13 |
| Example 11 | 530.14±40.08 | 93 | 80 to 85 days | 5.7 |
| Example 12 | 640.1±33.73 | 93 | 80 to 85 days | 6.8 |
| Comparative Example 1 | 197.34±24.51 | 97 | 7 to 14 days | 91 |
| Comparative Example 2 | 224.25±20.60 | 95 | 7 to 14 days | 95 |
| Comparative Example 3 | 178.10±23.34 | 100 | 7 to 14 days | 97 |

DNA residue testing showed that the mass proportion of DNA in the biological patch was ≤ 50 ng/mg in Examples 1 to 10, and even ≤30 ng/mg in some of the examples. In Example 1, the mass proportion of DNA in the biological patch was ≤20 ng/mg.

6. Inflammatory Response in Animal Experimental Tissues

The samples of Example 5, Comparative Examples 4 and 5 were subjected to subcutaneous implantation experiments in rats, and the inflammatory responses to the samples in the animals were observed at Weeks 2, 4, 6 and 8, respectively.

According to the comparison of H&E staining results, when observed in Week 2 to Week 4, the inflammatory response to the sample of Comparative Example 4 (completely non-degreased) was greater than that to the sample of Comparative Example 5 (completely degreased), while the inflammatory response to the sample of Comparative Example 5 was greater than that to the sample of Example 5 (partially degreased). When observed in Week 6 to Week 8, the tissue inflammatory response to the sample of Comparative Example 4 (completely non-degreased) was greater than that to the sample of Comparative Example 5 (completely degreased), while the inflammatory response to the sample of Comparative Example 5 was basically the same as that to the sample of Example 5 (partially degreased).

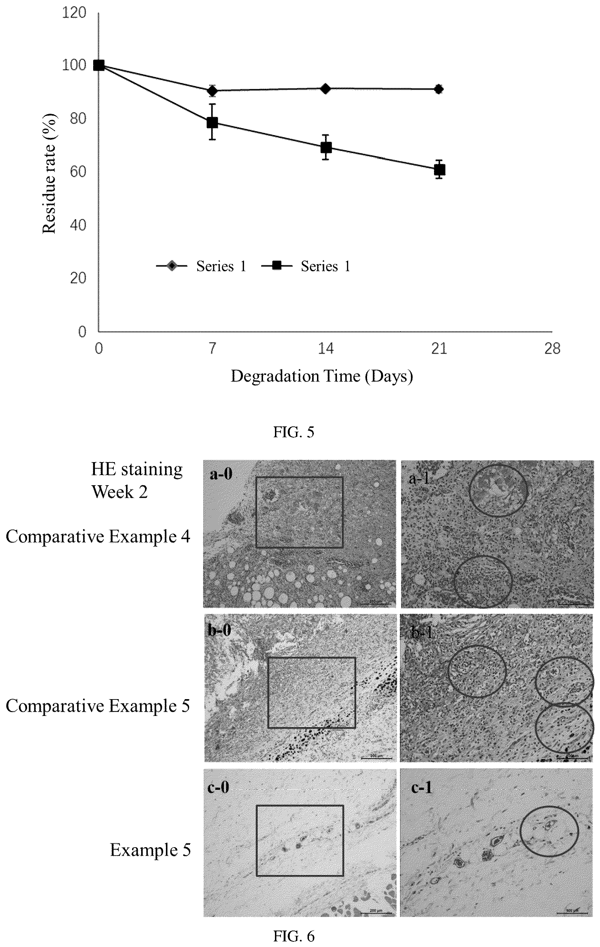

Taking the staining results at Week 2 as an example, FIG. 6 shows the H&E staining results of three samples at Week 2; a-0 and a-1 respectively correspond to the H&E staining result and it locally enlarged view of Comparative Example 4 at Week 2; b-0 and b1 respectively correspond to the H&E staining result and its locally enlarged view of Comparative Example 5 at Week 2; and c-0 and c1 respectively correspond to the H&E staining result and its locally enlarged view of Example 5 at Week 2. As shown in the enlarged view (a-1) of a local area of a-0 in FIG. 6, the upper circled region reveals tissue necrosis, while the lower circled region reveals a significant aggregation of basophilic cells in the tissue, indicating inflammatory cell infiltration. As shown in the enlarged view (b-1) of a local area of b-0 in FIG. 6, the circled region on the left reveals a significant aggregation of basophilic cells and inflammatory cell infiltration in the tissue, indicating aggravated inflammation; the upper circled region on the right reveals newly formed blood vessels containing red blood cells, and the lower circled region on the right reveals visible pigment deposition. As shown in the enlarged view (c-1) of a local area of c-0 in FIG. 6, the circled region reveals newly formed blood vessels containing red blood cells in the tissue. Based on the state of tissue necrosis and neovascularization, the inflammatory response can be ranked from highest severity to lowest severity as follows: Comparative Example 4 > Comparative Example 5 > Example 5. It can be seen that the anti-inflammatory effect of the partially degreased sample was significantly better than that of the completely degreased sample, and the anti-inflammatory effect of the completely degreased sample was better than that of the completely non-degreased sample.

**Examples - Part Two: Pigment removal treatment by acidic solution in combination with decellularization treatment by alkaline solution (Approach I)**

In the following examples for preparing biological patches, unless otherwise specified, the solvent used for prepare the acidic solution and the alkaline solution was water. Unless otherwise specified, the "bath ratio" involved in washing refers to the ratio of the volume of the washing solution to the mass of the material to be washed, with a unit of mL/g. For example, the volume of the washing solution can be in a range from 10 mL/g to 30 mL/g of the material. Unless otherwise specified, the freeze-drying was performed using a freeze-dryer under conditions including a temperature of 25 °C and a vacuum pressure of 40 Pa.

In the following Example Bi (i is 1 to 5), unless otherwise specified, in skinning step, the skin was washed by using a conventional method in the art to remove excess impurities such as blood and fat. The parameters described in Examples - Part I can be used. In an example, the fish skin was repeatedly rinsed 3 times with a 2 wt% sodium carbonate solution to remove mucus and grease from the inner and outer surfaces of the fish skin, and then repeatedly rinsed 3 times with deionized water. In another example, the fish skin was flushed with running tap water to remove the visible yellow grease from the skin surface, and then washed with a 3 wt% starch aqueous solution to remove surface fat. The washing was repeated three times. This method was used in the following Example B1.

Example B1 - Preparation of single-layer biological patch

(1) Skinning: Scaleless skin was removed from a Chinese longsnout catfish weighing more than 1 kg using a skinning machine, and washed to remove excess impurities such as blood and fat.

(2) Cutting: An appropriate size of intact fish skin was selected and cut to ensure neat edges. The fish skin weighed about 50 g (with a deviation within $\pm$ 0.5 g).

(3) Preparing acidic solution: 200 mL of 0.5 M acetic acid solution was prepared and added to a 1000 mL beaker for later use.

(4) Acid treatment and pigment removal: The fish skin weighed about 50 g obtained in step (2) was put into the 200 mL acetic acid solution prepared in step (3), and stirred for 2 min. The acidic solution was discarded, and the soft pigment was scraped off from the fish skin using a blunt instrument. The fish skin was washed with deionized water under shaking at a bath ratio of 20 mL/g three times, each lasting 10 min.

(5) Alkali treatment: The fish skin after step (4) was put into a conical flask containing 0.01 M $Na_2CO_3$ solution (alkaline solution, 400 mL). The conical flask was placed in a constant temperature shaker, shaken at a temperature of 25 °C and a speed of 160 rpm for 24 h.

(6) Washing: The fish skin after step (5) was washed with deionized water under shaking at a bath ratio of 20 mL/g three times, each lasting 10 min, to remove excess $Na_2CO_3$ solution.

(7) Freeze-drying: The fish skin after step (6) was pre-frozen at -80 °C for 24 h, and then dried in a freeze-dryer for 48 h. The freeze-dried single-layer fish skin was then taken out and cut into suitable sizes for later use.

Example B2 - Preparation of single-layer biological patch

The differences from Example B1 were as follows: In preparation of acidic solution, 200 mL of 0.001 M citric acid solution was prepared and added to a 1000 mL beaker for later use. In alkali treatment, 0.5 M $NaHCO_3$ solution (alkaline solution, 500 mL) was used. In washing, the bath ratio was 30 mL/g.

Example B3 - Preparation of single-layer biological patch

The differences from Example B1 were as follows: In preparation of acidic solution, 200 mL of 0.1 M hydrochloric acid solution was prepared and added to a 1000 mL beaker for later use. In acid treatment, the stirring period was 10 min. In alkali treatment, 600 mL of 2.0 M trisodium phosphate ($Na_3PO_4$) solution was used and the alkali treatment lasted for 12 h. In washing, the bath ratio was 40 mL/g.

Example B4 - Preparation of single-layer biological patch using composite acid solution

The differences from Example B1 were as follows: In preparation of acidic solution, 100 mL of 0.5 M acetic acid solution and 100 mL of 0.1 M hydrochloric acid solution were prepared for later use. In acid treatment, 200 mL of composite acid solution was used. In alkali treatment, 0.02 M $Na_2CO_3$ solution (alkaline solution, 400 mL) was used. In washing, the bath ratio was 30 mL/g.

Example B5 - Preparation of single-layer biological patch using composite alkali solution

The differences from Example B2 were as follows: In alkali treatment, 500 mL of alkaline composite solution including 250 mL of 0.5 M $NaHCO_3$ solution and 250 mL of 0.01 M $Na_2CO_3$ solution was used. In washing, the bath ratio was 40 mL/g.

Comparative Example B1 - Preparation of single-layer biological patch

In Comparative Example B1, the method was substantially the same as that in Example B2, except using Triton as a surfactant for decellularization. After step (6) of washing with NaHCO$_3$ solution, following steps (7), (8) and (9) were performed.

(7) Re-decellularization treatment: The fish skin after step (6) was added to a Triton X-100 solution with a concentration of 1% at a bath ratio of 20 mL/g, and placed in a constant temperature shaker, shaken at a temperature of 25 °C and a speed of 160 rpm for 48 h.

(8) Re-washing: The fish skin after step (7) was washed with deionized water under shaking at a bath ratio of 30 mL/g three times, each lasting 5 min, to remove excess Triton X100 solution.

(9) Freeze-drying: The fish skin after step (8) was pre-frozen at -80 °C for 24 h, and then dried in a freeze-dryer for 48 h. The fish skin was then taken out and cut into suitable sizes for later use.

Comparative Example B2 - Preparation of single-layer biological patch using conventional chemical reagent treatment method instead of the acid-alkali combined treatment method

(1) Skinning: Scaleless skin was removed from a Chinese longsnout catfish weighing more than 1 kg using a skinning machine, and washed to remove excess impurities such as blood and fat.

(2) Cutting: An appropriate size of intact fish skin was selected and cut to ensure neat edges. The fish skin weighed about 20 g (with a deviation within ± 0.2 g).

(3) Preparing solution: 500 mL of 0.02 M sodium dodecyl sulfate (SDS) solution was prepared and added to a 1000 mL beaker for later use.

(4) SDS treatment and pigment removal: The fish skin weighed about 20 g obtained in step (2) was put into the 200 mL SDS solution prepared in step (3), and stirred for 30 min. The SDS solution was discarded, and the soft pigment was scraped off from the fish skin using a blunt instrument. The fish skin was washed with deionized water under shaking at a bath ratio of 20 mL/g three times, each lasting 10 min.

(5) Enzyme treatment: The fish skin after step (4) was put into a conical flask containing 0.01 M DNase solution (enzyme solution, 200 mL). The conical flask was placed in a constant temperature shaker, shaken at a temperature of 25 °C and a speed of 160 rpm for 20 h.

(6) Washing: The fish skin after step (5) was washed with deionized water under shaking at a bath ratio of 30 mL/g three times, each lasting 10 min, to remove excess enzyme solution.

(7) Re-decellularization treatment: The fish skin after step (6) was added to a Triton X-100 solution with a concentration of 1% at a bath ratio of 20 mL/g, and placed in a constant temperature shaker, shaken at a temperature of 25 °C and a speed of 160 rpm for 48 h.

(8) Re-washing: The fish skin after step (7) was washed with deionized water under shaking at a bath ratio of 30 mL/g three times, each lasting 5 min, to remove excess Triton X100 solution.

(9) Freeze-drying: The fish skin after step (8) was pre-frozen at -80 °C for 24 h, and then dried in a freeze-dryer for 48 h. The fish skin was then taken out and cut into suitable sizes for later use.

Comparative Example B3 - Preparation of single-layer biological patch without acid treatment (omitting the acid treatment compared with Example B1)

The method was substantially the same as that in Example B1, except that the acid treatment was omitted, i.e., steps (3) and (4) of Example B1 were omitted.

Comparative Example B4 - Preparation of single-layer biological patch with pH value of acidic solution changed

The method was substantially the same as that in Example B1, except that the pH value of the acidic solution was changed, i.e., the concentration of the acetic acid solution was changed to 1 M.

Comparative Example B5 - Preparation of single-layer biological patch without alkali treatment

The method was substantially the same as that in Example B1, except that the alkali treatment step was omitted, i.e., step (7) was directly performed after step (4) of Example B1.

Comparative Example B6 - Preparation of single-layer biological patch with pH value of alkaline solution changed

The method was substantially the same as that in Example B1, except that the pH value of the alkaline solution was changed, i.e., the concentration of the $Na_2CO_3$ solution was changed to 4 M.

The preparation parameters of the above single-layer biological patches in the examples and comparative examples can be found in Table 8. The size of the stitched surface of each of the biological patches in Examples B1 to B13 and Comparative Examples B1 to B11 was 80 mm × 80 mm.

Table 8. Preparation parameters for single-layer decellularized fish skin (single-layer biological patch)

| No. | Description | Acidic solution | | Alkaline solution | | Other reagents for decellularization |
|---|---|---|---|---|---|---|
| | | Acidic reagent | Concentration | Alkaline reagent | Concentration | |
| Example B1 | Acid-alkali combined treatment | Acetic acid | 0.5M | $Na_2CO_3$ | 0.01M | None |
| Example B2 | Acid-alkali combined treatment | Citric acid | 0.001M | $NaHCO_3$ | 0.5M | None |
| Example B3 | Acid-alkali combined treatment | Hydrochloric acid | 0.1M | $Na_3PO_4$ | 2.0M | None |
| Example B4 | Using composite acid solution | Acetic acid, hydrochloric acid | 0.5M, 0.1M | $Na_2CO_3$ | 0.01M | None |
| Example B5 | Using composite alkali solution | Citric acid | 0.001M | $NaHCO_3$, $Na_2CO_3$ | 0.5M, 0.01M | None |
| Comparative Example B1 | Acid-alkali combined treatment in addition with using reagents other than acid and alkali for decellularization | Citric acid | 0.001M | $NaHCO_3$ | 0.5M | 1% Triton X-100 solution |
| Comparative Example B2 | Conventional chemical reagent treatment instead of acid-alkali combined treatment | Sodium dodecyl sulfate | 0.02M | DNase | 0.01M | 1% Triton X-100 solution |
| Comparative Example B3 | Omitting acid treatment | / | / | $Na_2CO_3$ | 0.01M | None |
| Comparative Example B4 | Changing pH of acidic solution | Acetic acid | 1M | $Na_2CO_3$ | 0.01M | None |
| Comparative Example B5 | Omitting alkali treatment | Acetic acid | 0.5M | / | / | None |
| Comparative Example B6 | Changing pH of alkaline solution | Acetic acid | 0.5M | $Na_2CO_3$ | 4M | None |

Testing

Samples to be tested were the biological patches (single-layer or multi-layer) of desired sizes prepared by the preparation methods of the examples and comparative examples.

Testing results and analysis

The testing results can be found in Table 9. In Table 9, each testing was performed with 3 parallel samples.

Table 9. Testing results

| Type of biological patch | No. | Tensile breaking force | Cytotoxicity index (cell viability) (%) |
|---|---|---|---|
| Single-layer biological patch | Example B1 | 29.99±4.73 | 92 |
| | Example B2 | 25.44±7.39 | 93 |
| | Example B3 | 27.38±6.17 | 90 |
| | Example B4 | 32.11±5.04 | 90 |
| | Example B5 | 28.59±8.11 | 92 |
| | Comparative Example B1 | 16.57±4.55# | 64* |
| | Comparative Example B2 | 10.34±4.72# | 53* |
| | Comparative Example B3 | 24.46±6.63 | 52* |
| | Comparative Example B4 | 15.76±9.13# | 63* |
| | Comparative Example B5 | 16.14±3.98# | 55* |
| | Comparative Example B6 | 22.55±7.46 | 64* |

In Table 9, "#" indicates that the tensile breaking force of the specified group shows a significant difference compared to Examples B1 to B5, and "*" indicates that the cytotoxicity of the specified group shows a significant difference compared to Examples B1 to B5, both at a statistically significance level of $p < 0.05$.

The biological patches prepared in Examples 1 to 12 exhibited low cytotoxicity, good biocompatibility, and good mechanical performance (including large high breaking force and high adhesion strength), and thus were all suitable for soft tissue repair.

In Comparative Example B1, the surfactant Triton X-100 was additionally employed, resulting in a sinlge-layer biological patch with low tensile breaking force and high cytotoxicity, as the control group for cell cytotoxicity was 100%, the lower values indicate greater toxicity.

In Comparative Example B2, the conventional method was performed by using chemical and enzymatic reagents throughout the whole process, which significantly damaged the structure of the fish skin material, leading to inferior mechanical performance and higher cytotoxicity due to residual surfactant.

In Comparative Example B3, the acid treatment was omitted. While the mechanical performance remained undiminished, pigments cannot be removed, and residual pigments triggered cellular reactions. Additionally, the alkaline solution cannot be neutralized, resulting in significant cytotoxicity.

In Comparative Example B4, the acidity was excessively strong, causing severe puffing and loose structure of the fish skin, reducing its mechanical performance. Additionally, residual acidity cannot be fully neutralized, resulting in significant cytotoxicity.

In Comparative Example B5, the alkali treatment was omitted, failing to alleviate fish skin puffing and loose structure. The acidic solution cannot be neutralized, thus leading to reduced mechanical performance and significant cytotoxicity.

In Comparative Example B6, the alkaline solution was excessively strong, which cannot be neutralized, leading to significant cytotoxicity.

Additionally, for Examples B1 to B5, based on supplementary testing, the contents of the fatty acids were generally consistent with the ranges shown in Tables 4 and 5, the fiber diameters ranged approximately from 2 $\mu$m to 30 $\mu$m, the pore sizes ranged approximately from 2 $\mu$m to 5 $\mu$m, and the porosity ranged approximately from 55% to 75%, with most concentrated in 55% to 70%. The water contact angles of the biological patch surfaces of the examples ranged approximately from 51° to 90°. The surface roughness of the first surface of the examples ranged from 8 $\mu$m to 20 $\mu$m. The surface roughness of the second surface of the examples ranged from 25 $\mu$m to 40 $\mu$m. The testing results on degradation performance were also similar to those of the aforementioned Examples 1 to 10.

**Examples** - **Part Three: Decellularization treatment followed** by **microwave puffing treatment (Approach III)**

Example C1

(1) Pretreatment: Longsnout catfish skin with a thickness of 0.31 mm $\pm$ 0.09 mm was repeatedly rinsed 3 times with 2 wt% sodium carbonate solution to remove mucus and grease from the inner and outer surfaces of the fish skin, and then repeatedly rinsed 3 times with deionized water.

(2) Removal of pigment and connective tissue: The fish skin obtained in step (1) was soaked in 0.05 M acetic acid solution with a pH of 2.96 and shaken on a shaker at 200 rpm for 2 min at room temperature, and then repeatedly rinsed 3 times with deionized water.

(3) Decellularization: The fish skin obtained in step (2) was soaked in 2% Triton X-100 solution and shaken on a shaker at 200 rpm for 6 h at room temperature, and then repeatedly rinsed 3 times with deionized water.

(4) Microwave treatment: The decellularized fish skin in step (3) was soaked in castor oil, taken out and placed in a microwave generator for microwave puffing. The microwave power was 900 W and the time period of microwave treatment was 5 s.

(5) Washing and drying: The fish skin treated with microwaves in step (4) was repeatedly rinsed 3 times with deionized water, and then freeze-dried at -56 °C and a vacuum degree of 28 Pa for 8 h to obtain a decellularized fish skin matrix.

For Examples C2 to C9, the preparation method was substantially the same as that in Example C1, except that the microwave treatment of step (4) was performed with different parameters, as shown in Table 11.

For Comparative Examples C1, the preparation method was substantially the same as that in Example C1, except that the microwave treatment of step (4) was not performed, as shown in Table 12.

The parameters of microwave treatment in the preparation methods of Examples C1 to C9 and Comparative Example C1 are shown in Table 10.

Table 10

| Group | Power of microwave treatment (W) | Time period of microwave treatment (s) | Thickness of decellularized fish skin matrix (mm) |
|---|---|---|---|
| Example C1 | 900 | 5 | 0.57±0.07 |
| Example C2 | 900 | 30 | 0.89±0.17 |
| Example C3 | 900 | 60 | 0.49±0.05 |
| Example C4 | 700 | 5 | 0.40±0.03 |
| Example C5 | 700 | 30 | 0.52±0.04 |
| Example C6 | 700 | 60 | 0.75±0.14 |
| Example C7 | 500 | 5 | 0.32±0.08 |
| Example C8 | 500 | 30 | 0.42±0.10 |
| Example C9 | 500 | 60 | 0.68±0.13 |
| Comparative Example C1 | 0 | 0 | 0.22±0.06 |

Test 1 - Decellularized fish skin matrix thickness testing

The thickness of the decellularized fish skin matrices obtained with microwave treatment in Examples C1 to C9 and that without microwave treatment in Comparative Example C1 was measured using a vernier caliper. The results are shown in Table 10. According to the test results, the thickness of the decellularized fish skin matrix obtained without microwave treatment in Comparative Example C1 was only 0.22 mm $\pm$ 0.06 mm, significantly less than the thickness of normal human skin (0.5 mm to 1.7 mm). In contrast, the thickness of the decellularized fish skin matrices obtained with microwave treatment in Examples C1 to C9 of the present disclosure was significantly greater than that of Comparative Example C1, and substantially the same as the thickness of normal human skin.

Test 2

The mechanical performance of the decellularized fish skin matrices in dry and wet states prepared with microwave treatment in Examples C1 to C9 and without microwave treatment in Comparative Example C1 were tested in accordance with the national standard "GB/T 1040.3-2006 Plastic - Determination of tensile properties - Part 3: Test conditions for films and sheets". The test results are shown in Table 11.

Table 11

| Group | Dry | | | Wet | | |
|---|---|---|---|---|---|---|
| | Load (N) | Tensile strength (MPa) | Elastic modulus (MPa) | Load (N) | Tensile strength (MPa) | Elastic modulus (MPa) |
| Example C1 | 46.33±2.16 | 18.87±2.18 | 148.27±11.08 | 11.58±2.25 | 7.21±0.54 | 3.71±0.28 |
| Example C2 | 76.33±5.58 | 17.75±2.56 | 87.07±7.78 | 19.08±1.39 | 4.44±1.39 | 2.18±0.19 |
| Example C3 | 63.80±2.94 | 9.25±1.12 | 76.66±6.17 | 15.95±0.73 | 15.95±0.74 | 2.92±0.15 |
| Example C4 | 66.24±4.60 | 11.48±4.10 | 175.11±14.25 | 16.56±1.15 | 16.7±1.15 | 4.37±0.36 |
| Example C5 | 49.56±2.01 | 16.14±3.46 | 151.81±12.51 | 12.39±0.51 | 12.39±0.98 | 3.79±0.31 |
| Example C6 | 54.21±3.60 | 10.22±2.10 | 85.68±8.04 | 13.55±0.90 | 13.55±0.52 | 2.14±0.20 |
| Example C7 | 49.87±2.80 | 10.18±2.18 | 181.58±13.36 | 12.47±1.87 | 12.47±1.81 | 4.54±0.33 |
| Example C8 | 58.22±3.46 | 12.10±4.11 | 171.34±9.43 | 14.56±0.98 | 14.56±4.21 | 4.28±0.24 |
| Example C9 | 72.36±7.02 | 15.39±4.06 | 165.74±6.77 | 18.09±1.74 | 18.09±2.64 | 4.14±0.17 |
| Comparative Example C1 | 59.76±5.33 | 8.02±1.63 | 197.55±10.11 | 14.94±1.35 | 14.94±3.16 | 4.94±0.25 |

As can be seen from Table 11, the decellularized fish skin matrix prepared after microwave treatment exhibited higher tensile strength than the decellularized fish skin matrix without microwave treatment. The matrix can quickly absorb water when immersed in water, and the water-absorbed matrix was soft and docile while retaining strong elasticity, and thus would not break when implanted at a skin lesion site.

Test 3

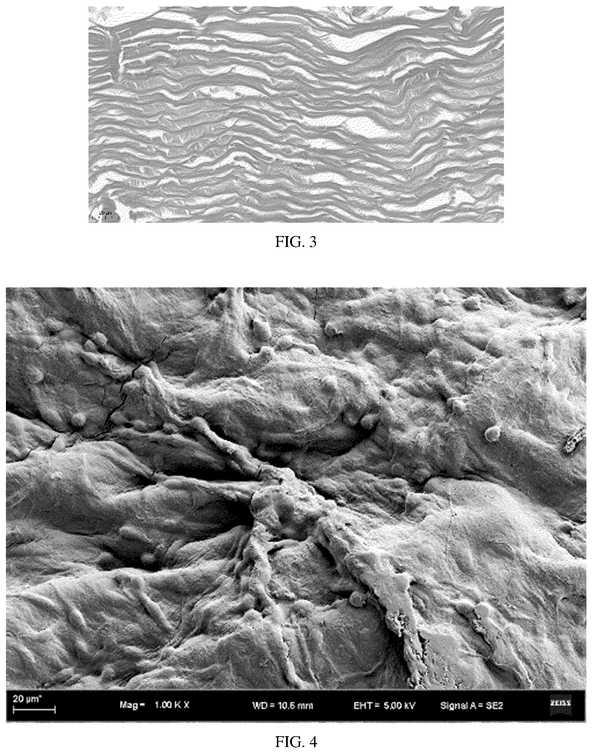

The decellularized fish skin matrix prepared in Example C1 was fixed in a 4 wt% paraformaldehyde solution for 48 h, then washed, dehydrated, paraffin-embedded, sliced, dewaxed, and H&E stained. The tissue morphology of each sample was observed under an optical microscope. The results are shown in FIG. 3. After H&E staining, collagen was red and the cell nucleus was blue-purple. As shown in FIG. 3, the decellularized fish skin matrix sample prepared in Example C1 contained a large number of collagen fibers, and no blue-purple cell nuclei were observed, indicating complete decellularization and substantially complete removal of immunogenic components from the fish skin. The tissue morphology of the matrices prepared in Examples C2 to C9 and Comparative Example C1 was similar to that in Example C1, and thus will not be repeated further.

Test 4

The decellularized fish skin matrix sample prepared in Example C1 was placed in a 24-well culture plate, inoculated with a single-cell suspension of fibroblasts, and incubated in an incubator at 37 °C with carbon dioxide in a volume concentration of 5% for 72 h. Then the sample was fixed with a polyformaldehyde solution with a mass concentration of 4%, and gradient dehydrated with 50%, 75%, 90% and 100% ethanol in sequence, vacuum dried, sputtered with gold, and finally observed under a scanning electron microscope, as shown in FIG. 4. Fibroblasts, one of the most important cells in the skin, were cultured on the decellularized fish skin matrix. As shown in FIG. 4, the long spindle-shaped protrusions are fibroblasts, which are uniformly attached to the surface of the decellularized fish skin matrix, indicating that the decellularized fish skin matrix has excellent cell compatibility and can be used as a carrier for skin cell growth. The scanning electron microscopic images of the matrices prepared in Examples C2 to C9 and Comparative Example C1 were similar to the above image for Example C1, and thus will not be repeated further.

Test 5

The decellularized fish skin matrix samples prepared in Example C1 and Comparative Example C1 were cut into sheets with uniform shape and size, which were weighed and recorded. The cut matrix samples were soaked in a phosphate-buffered saline solution containing 5 mg/L lysozyme. At day 7, day 14, day 21, and day 28, the samples were taken out respectively, freeze-dried, and weighed. The mass loss was obtained by comparing the masses before and after degradation, and the in vitro degradation rate of the matrix was calculated. The results are shown in FIG. 5, wherein Series 1 represents the decellularized fish skin matrix prepared in Comparative Example C1, and Series 2 represents the decellularized fish skin matrix prepared in Example C1. Before the in vitro degradation test, the decellularized fish skin matrix samples were not degraded, with a residual rate of 100%. A higher residual rate indicates slower degradation, and a lower residual rate value indicates faster degradation. As shown in FIG. 5, the decellularized fish skin matrix obtained in Comparative Example C1 degraded by about 10% in 21 days, and the decellularized fish skin matrix obtained in Example C1 degraded by about 40% in 21 days, indicating that both matrix materials can be degraded and have potential as wound dressings. However, the degradation rate of the decellularized fish skin matrix prepared with microwave treatment in Example C1 was much greater than that without microwave treatment in Comparative Example C1, indicating that microwave treatment can accelerate the degradation of the decellularized fish skin matrix.

In addition, for Examples B1 to B5, based on supplementary testing, the contents of the fatty acids were generally consistent with the ranges shown in Tables 4 and 5, the fiber diameters ranged approximately from 2 μm to 30 μm, the pore sizes ranged approximately from 2 μm to 5 μm, and the porosity ranged approximately from 55% to 75%.

**Examples - Part Five (Approach II)**

Example E2

Fish skin was taken out from a -20 °C freezer and placed into a -4 °C refrigerator for slow overnight thawing. The yellow grease visible on the skin surface of the thawed fish skin was removed by being flushed with running tap water. During flushing, the water was maintained at a certain flow rate, and the flushing was performed for 10 min.

The fish skin was washed with a 3% starch solution three times to remove surface fat. The undamaged degreased fish skin was soaked in a 15 wt% sucrose solution as the cryoprotectant for 30 min, the surface moisture was removed, and then the fish skin was repeatedly frozen and thawed in a -20 °C freezer, with the freeze-thaw process repeated three times, to obtain a pretreated fish skin.

The pretreated fish skin was soaked and stirred in an aqueous solution containing sodium dodecyl sulfate (SDS) for depigmentation. After soaking and stirring for 2 h, the fish skin was taken out, washed, and the solution was replaced. The aqueous solution treatment continues for a total of 4 h to remove surface melanin, obtaining depigmented fish skin. The mass proportion of SDS in the aqueous solution was 0.5%.

The depigmented fish skin was placed in a Triton X-100 solution for decellularization. After 24 h, the fish skin was washed with distilled water to completely remove the Triton X-100 remaining on the skin surface. The fish skin was freeze-dried to obtain a fish skin-based biological patch. The mass proportion of Triton X-100 in the Triton X-100 solution was 0.5%.

In Examples E14, E15, and E16, fish skin-based biological patches were prepared by a method similar to the method for preparing the fish skin-based biological patch in Example E1, with the same pretreatment steps for the fish skin. The main difference was that at least one of the types and amounts of solutions involved in the pigment removal treatment (also called depigmentation treatment) and decellularization treatment was different, as shown in Table 12.

Comparative Example E1

Comparative Example E1 was different from Example E1 in that the fish skin was only subjected to degreasing pretreatment to obtain a pretreated fish skin, without pigment removal treatment and decellularization treatment.

Comparative Example E2 (Manual depigmentation)

Comparative Example E2 was different from Example E1 in that the pretreated fish skin was manually depigmented, while other parts remained the same. The manual depigmentation process was as follows:

The pretreated fish skin was soaked in a dilute acetic acid solution with a concentration of 0.05 wt% for 2 min, allowing the fish skin to swell slightly. The fish skin was then taken out, and the pigment layer was removed from the inner and outer surfaces of the fish skin with a scraper to obtain a depigmented fish skin.

The parameter settings in the above examples are shown in Table 12.

Table 12.

| Group | Depigmentation treatment | | | Decellularization treatment |
| | Liquid replacement cycle | Penetration enhancer | Acid-alkali regulator | |
|---|---|---|---|---|
| Example E2 | 2 h, 4 h in total | 0.5% SDS | / | 0.5% Triton X-100 aqueous solution |
| Example E13 | 2 h, 4 h in total | / | 0.1% citric acid aqueous solution | 0.5% Triton X-100 aqueous solution |
| Example E14 | 2 h, 4 h in total | / | 0.1% acetic acid | 0.5% Triton X-100 aqueous solution |
| Example E15 | 2 h, 4 h in total | 0.5% SDS | 0.1% citric acid aqueous solution | 0.5% Triton X-100 aqueous solution |
| Example E16 | 2 h, 4 h in total | 0.5% SDS | 0.1% acetic acid aqueous solution | 0.5% Triton X-100 aqueous solution |
| Comparative Example E1 | / | / | / | / |
| Comparative Example E2 | After soaking in 0.05% dilute acetic acid solution for 2 min, the pigment layer was removed from the inner and outer surfaces of the fish skin with a scraper | | | 0.5% Triton X-100 |

In Table 12, SDS represents sodium dodecyl sulfate.

Test 1 - Depigmentation effect

The biological patches from the examples in Part IV had uniform surfaces without defects such as holes or scratches, exhibiting better depigmentation effect than that of Comparative Examples E1 and E2. The thawed fish skin was subjected to degreasing pretreatment to obtain a pretreated fish skin. The fish skin-based biological patch obtained by subjecting the pretreated fish skin to depigmentation using the method in Example E1, and further depigmentation using the method in Example E16, and then decellularization exhibited better depigmentation effect and reduced fat content and DNA residue.

The depigmentation states of Examples E13 to E16 can be found in Table 13. After the same treatment time, the simultaneous addition of the penetration enhancer and acid can significantly promote the precipitation of melanin. Additionally, the depigmentation effect of citric acid was better than that of acetic acid at the same concentration, but neither achieved complete depigmentation. Acetic acid caused less damage to the stability of the fish skin.

Table 13

| Group | Example E13 Citric acid | Example E15 Citric acid+SDS | Example E14 Acetic acid | Example E16 Acetic acid+SDS |
|---|---|---|---|---|
| Description | Partially faded | Basically faded | Partially faded | Basically faded |
| Effect | Moderate | Good | Moderate | Good |

Test 2 - Comparison of degradation performance of fish skin

Table 14

|  | Treatment | Degradation time | Degradation state | Indication |
|---|---|---|---|---|
| Example E14 | Acetic acid pigment removal | 2 months | Complete degradation, fragments | Oral membrane |
| Example E13 | Citric acid pigment removal | 3 months | Complete degradation, fragments | Oral membrane |

From the results shown in Table 14, it can be seen that the fish skin-based biological patches obtained with different depigmentation methods exhibited different degradation rates and degradation times, and thus can be applied to the repair of different tissues, indicating that the degradation performance of the samples can be controlled through different processes, thereby expanding the application of fish skin.

Test 3 - Fat content of fish skin

Table 15. Fat content of fish skin with different treatments

| Parameter | Fat content (%) |
|---|---|
| Comparative Example E1 | 24.1±1.2 |
| Comparative Example E2 | 20.2±0.6 |
| Example E15 | 4.1±0.3 |
| Example E16 | 3.6±0.8 |

The fat content of the fish skin with different treatments was significantly different from that of the original fish skin. After different treatments, the fat content of the fish skin gradually decreased. In addition, compared with the comparative examples, the fat content of the fish skin-based biological patches obtained from Examples E15 and E16 was significantly reduced.

Test 4 - DNA residue

Table 16. DNA residue and cytotoxicity after different treatments

| Parameter | DNA content (ng/mg) | Cell viability |
|---|---|---|
| Comparative Example E1 | 382.5±40.2 |  |
| Comparative Example E2 | 166.7±21.2 | 0.98±0.08 |
| Blank control group | / | 1 |
| Example E16 | 8.2±1.5 | 1.03±0.31 |

Table 16 shows the DNA residues in fish skin after different treatments. The DNA content in untreated fish skin was greater than 50 ng/mg. After the initial treatment, the DNA residue of fish skin decreased. With further processing, the DNA residue in the fish skin was reduced to less than 50 ng/mg, which meets the specified requirements.

Test 5 - Cytotoxicity

The test results can be found in Table 16. The cell viability of the treated sample in Example E16 was greater than 0.70, indicating the effect of promoting cell proliferation with no cytotoxicity.

The technical features of the above-mentioned embodiments and examples can be combined arbitrarily. In order to make the description concise, not all possible combinations of the technical features are described in the embodiments and examples. However, as long as there is no contradiction in the combination of these technical features, the combinations should be considered as in the scope of the present disclosure.

The above-described embodiments are only several implementations of the present disclosure, which facilitate specific and detailed understanding of the technical solutions of the present application, but they should not be understood as limiting the patent protection scope of the present disclosure. It should be noted that various modifications and improvements can be made by those skilled in the art without departing from the concept of the present disclosure, which are all within the protection scope of the present disclosure. In addition, it should be understood that after reading the above teachings of the present disclosure, those skilled in the art can make various changes or modifications to the present disclosure, and equivalent forms obtained also fall within the protection scope of the present disclosure. It should also be understood that the technical solutions obtained by those skilled in the art through logical analysis, reasoning or limited experiments on the basis of the technical solutions provided by the present disclosure are all within the protection scope of the appended claims of the present disclosure. Accordingly, the patent protection scope of the present disclosure shall be defined by the appended claims, and the description and the accompany drawings may be used to interpret the content of the claims.

**Claims**

1.   A biological patch comprising a sheet-shaped body, wherein the sheet-shaped body comprises a collagen matrix;

the biological patch contains a first fatty acid and a second fatty acid, wherein the first fatty acid comprises at least two selected from the group consisting of butyric acid, caprylic acid, lauric acid, myristic acid, pentadecanoic acid, palmitic acid, stearic acid, arachidic acid, palmitoleic acid, oleic acid, $\gamma$-linolenic acid, linolenic acid ALA, eicosapentaenoic acid EPA, erucic acid, docosahexaenoic acid DHA, and tetracosenoic acid; the second fatty acid comprises an $\omega$-6 unsaturated fatty acid;
wherein based on a dry weight of the biological patch, a mass percentage of the first fatty acid in the biological patch is greater than or equal to 1.4%, and a mass percentage of the second fatty acid in the biological patch is less than or equal to 2.0%; the dry weight of the biological patch refers to a weight of the biological patch with a water content less than or equal to 5 wt% by mass;
the biological patch comprises collagen fibers with a diameter in a range from 2 $\mu$m to 30 $\mu$m; the biological patch comprises micropores with a pore size in a range from 2 $\mu$m to 5 $\mu$m; and a porosity of the biological patch is in a range from 55% to 75%.

2.   The biological patch according to claim 1, satisfying at least one of following characteristics:

based on the dry weight of the biological patch, the mass percentage of the first fatty acid in the biological patch is in a range from 1.4% to 8%, optionally in a range from 2% to 5%;
based on the dry weight of the biological patch, the mass percentage of the second fatty acid in the biological patch is less than or equal to 1.5%, optionally less than or equal to 1%;
a mass percentage of the first fatty acid in fatty acids contained in the biological patch is greater than or equal to 50%, optionally greater than or equal to 65%;
a mass percentage of the second fatty acid in fatty acids contained in the biological patch is less than or equal to 40%, optionally less than or equal to 26%;
a mass ratio of the first fatty acid to the second fatty acid in the biological patch is greater than 1, optionally greater than or equal to 2, further optionally greater than or equal to 2.5, and even further optionally in a range from 2.6 to 5; and
the porosity of the biological patch is in a range from 55% to 70%.

3.   The biological patch according to claim 1 or 2, wherein the first fatty acid satisfies one or more of following characteristics:

based on the dry weight of the biological patch, a mass percentage of butyric acid in the biological patch is in a range from 0% to 0.1%, optionally a range from 0.064% to 0.1%;
based on the dry weight of the biological patch, a mass proportion of caprylic acid in the biological patch is in a range from 0 ppm to 40 ppm, optionally in a range from 0 ppm to 20 ppm;
based on the dry weight of the biological patch, a mass proportion of lauric acid in the biological patch is in a range from 0 ppm to 55 ppm, optionally in a range from 20 ppm to 50 ppm;
based on the dry weight of the biological patch, a mass percentage of myristic acid in the biological patch is in a range from 0% to 0.06%, optionally in a range from 0.01% to 0.05%;
based on the dry weight of the biological patch, a mass proportion of pentadecanoic acid in the biological patch is in a range from 0 ppm to 120 ppm, optionally in a range from 0 ppm to 50 ppm, further optionally 0 ppm to 40 ppm;

based on the dry weight of the biological patch, a mass percentage of palmitic acid in the biological patch is in a range from 0% to 1.45%, optionally in a range from 0.28% to 1.45%, further optionally in a range from 0.4% to 0.8%;

based on the dry weight of the biological patch, a mass percentage of stearic acid in the biological patch is in a range from 0% to 0.35%, optionally in a range from 0.05% to 0.35%, further optionally in a range from 0.05% to 0.1%;

based on the dry weight of the biological patch, a mass proportion of arachidic acid in the biological patch is in a range from 0 ppm to 240 ppm, optionally in a range from 30 ppm to 240 ppm, further optionally in a range from 100 ppm to 240 ppm;

based on the dry weight of the biological patch, a mass percentage of palmitoleic acid in the biological patch is in a range from 0% to 0.15%, optionally in a range from 0.1% to 0.15%, further optionally in a range from 0.01% to 0.05%;

based on the dry weight of the biological patch, a mass percentage of oleic acid in the biological patch is in a range from 0% to 5%, optionally in a range from 0.5% to 3%, further optionally in a range from 1% to 2%;

based on the dry weight of the biological patch, a mass percentage of γ-linolenic acid in the biological patch is in a range from 0% to 0.085%, optionally in a range from 0.01% to 0.085%, further optionally in a range from 0.01% to 0.05%;

based on the dry weight of the biological patch, a mass percentage of linolenic acid ALA in the biological patch is in a range from 0% to 0.28%, optionally in a range from 0.04% to 0.28%, further optionally in a range from 0.04% to 0.1%;

based on the dry weight of the biological patch, a mass proportion of eicosapentaenoic acid EPA in the biological patch is greater than or equal to 80 ppm, optionally greater than or equal to 150 ppm, further optionally greater than or equal to 300 ppm, even further optionally greater than or equal to 400 ppm, or still optionally in a range from 300 ppm to 600 ppm;

based on the dry weight of the biological patch, a mass percentage of erucic acid in the biological patch is in a range from 0% to 0.05%, optionally in a range from 0% to 0.04%, further optionally in a range from 0.01% to 0.04%;

based on the dry weight of the biological patch, a mass percentage of docosahexaenoic acid DHA in the biological patch is in a range from 0% to 0.08%, optionally in a range from 0.01% to 0.07%, further optionally in a range from 0.02% to 0.06%; and

based on the dry weight of the biological patch, a mass proportion of tetracosenoic acid in the biological patch is greater than or equal to 60 ppm, optionally greater than or equal to 70 ppm, further optionally greater than or equal to 80 ppm, even further optionally in a range from 80 ppm to 100 ppm.

4. The biological patch according to any one of claims 1 to 3, wherein the second fatty acid comprises linoleic acid, arachidonic acid, or a combination thereof;

the second fatty acid satisfies one or more of following characteristics:

based on the dry weight of the biological patch, a mass percentage of linoleic acid in the biological patch is in a range from 0% to 2.5%, optionally in a range from 0% to 1.5%, further optionally in a range from 0% to 1%, even further optionally in a range from 0.5% to 1%; and

based on the dry weight of the biological patch, a mass percentage of arachidonic acid in the biological patch is in a range from 0% to 0.090%, optionally in a range from 0% to 0.06%, further optionally in a range from 0.01% to 0.05%.

5. The biological patch according to any one of claims 1 to 4, comprising a first surface and a second surface that are opposite to each other, wherein the biological patch satisfies one or more of following characteristics:

a surface roughness Ra of the first surface satisfies Ra ≤20 μm, optionally is in a range from 8 μm to 20 μm; and

a surface roughness Ra of the second surface satisfies Ra ≥25 μm, optionally is in a range from 25 μm to 40 μm.

6. The biological patch according to any one of claims 1 to 5, comprising a first surface and a second surface that are opposite to each other, wherein the biological patch satisfies one or more of following characteristics:

a water contact angle of the first surface is larger than or equal to 50°, optionally in a range from 50° to 90°, and further optionally in a range from 51° to 90°;

the biological patch is capable of absorbing water weighing at least 2.5 times the dry weight of the biological patch, optionally 3 to 5 times the dry weight of the biological patch; and

a water content of the biological patch is less than or equal to 12 wt%, optionally less than or equal to 6 wt%, and further optionally in a range from 3 wt% to 6 wt%, by mass percentage.

7. The biological patch according to any one of claims 1 to 6, wherein based on an in vitro degradation testing method, the biological patch satisfies one or more of following characteristics:

a degradation period of the biological patch is in a range from 7 days to 180 days, and optionally in a range from 7 days to 84 days; and

after 7 days of degradation, a degradation ratio of the biological patch is in a range from 5.7% to 97%.

8. The biological patch according to any one of claims 1 to 7, wherein a mass proportion of DNA in the biological patch is less than or equal to 50 ng/mg, optionally less than or equal to 30 ng/mg, and further optionally less than or equal to 20 ng/mg;

a total fat content of the biological patch is in a range from 1% to 19%, optionally in a range from 3% to 19%.

9. The biological patch according to any one of claims 1 to 8, wherein the biological patch is a single-layer biological patch or a multi-layer biological patch, the multi-layer biological patch is formed by stacking and combining a plurality of single-layer biological patches;

optionally, the biological patch satisfies one or more of following characteristics:

the biological patch is a fish skin-based biological patch;

the single-layer biological patch has a thickness in a range from 0.1 mm to 1 mm;

the multi-layer biological patch has a thickness in a range from 0.5 mm to 2 mm;

a quantity of the single-layer biological patches stacked in the multi-layer biological patch is 2, 3 or 4; and

in the multi-layer biological patch, adjacent single-layer biological patches are fixed together by an adhesive layer or by a thread that penetrates the adjacent single-layer biological patches in a thickness direction.

10. A method for preparing a biological patch, comprising: performing a degreasing treatment, a pigment removal treatment, and a decellularization treatment on fish skin, and performing a freeze-drying treatment to obtain the biological patch according to any one of claims 1 to 9, wherein the fish skin has undergone a surface mucus removal treatment and a blood removal treatment;

the degreasing treatment is performed by using a method comprising: washing the fish skin with first treatment liquids to remove grease impurities from inner and outer surfaces of the fish skin, the washing comprising flushing and rinsing;

the pigment removal treatment is performed by using a method comprising: soaking the degreased fish skin in a second treatment liquid, taking the degreased fish skin out from the second treatment liquid, removing pigment, and washing the degreased fish skin with a second washing liquid to obtain a depigmented fish skin matrix;

the decellularization treatment is performed by using a method comprising: soaking the depigmented fish skin matrix in a third treatment liquid, taking the depigmented fish skin matrix out from the third treatment liquid, and washing the depigmented fish skin matrix with a third washing liquid to obtain a decellularized fish skin matrix; wherein:

the first treatment liquids comprise a first running washing liquid and a second washing liquid, and optionally comprise a surfactant solution; the running washing liquid is water; the second washing liquid is a sodium carbonate aqueous solution or a starch aqueous solution, wherein a pH value of the sodium carbonate aqueous solution is in a range from 8.4 to 11.5, a mass percentage of starch in the starch aqueous solution is in a range from 1% to 5%; the surfactant solution is a sodium dodecyl sulfate aqueous solution, and a mass percentage of sodium dodecyl sulfate in the sodium dodecyl sulfate aqueous solution is in a range from 0.3% to 0.5%;

the second treatment liquid is an aqueous solution containing a penetration enhancer, or an aqueous solution containing an acidic reagent, or an aqueous solution containing an acidic regulator;

the penetration enhancer comprises sodium dodecyl sulfate, and a mass percentage of the penetration enhancer in the aqueous solution containing the penetration enhancer is in a range from 0.1% to 0.5%;

the acidic reagent is selected from the group consisting of acetic acid, citric acid, hydrochloric acid, formic acid, sulfuric acid, nitric acid, and any combinations thereof, and a concentration of the acidic reagent in the aqueous solution containing the acidic reagent is in a range from 1 mmol/L to 500 mmol/L, a pH value of the acidic solution is in a range from 1 to 5; on a condition that the aqueous solution containing the acidic reagent is an acetic acid aqueous solution, a mass percentage of the acetic acid in the acetic acid aqueous solution is in a range from 0.3 wt% to 3 wt%;

the acidic regulator comprises citric acid, acetic acid, or a combination thereof, and a mass proportion of the acidic regulator in the aqueous solution containing the acidic regulator is in a range from 0.1% to 0.3%;

the third treatment liquid is a Triton X-100 aqueous solution or an aqueous solution containing an alkaline reagent; the third washing liquid is water;

a mass percentage of Triton X-100 in the Triton X-100 aqueous solution is in a range from 0.1% to 2%;

the alkaline reagent is selected from the group consisting of sodium carbonate, sodium bicarbonate, trisodium phosphate, sodium monohydrogen phosphate, disodium hydrogen phosphate, sodium hydroxide, potassium carbonate, potassium bicarbonate, tripotassium phosphate, potassium monohydrogen phosphate, dipotassium hydrogen phosphate, potassium hydroxide, and any combinations thereof; a concentration of the alkaline reagent in the aqueous solution containing the alkaline reagent is in a range from 0.01 mol/L to 2 mol/L, and a pH value of the alkaline solution is in a range from 9 to 13.

11. The method according to claim 10, satisfying one or more of following characteristics:

the mass percentage of starch in the starch aqueous solution is in a range from 3% to 5%;

the aqueous solution containing the acidic reagent is the acetic acid aqueous solution, and the mass percentage of acetic acid in the acetic acid aqueous solution is in a range from 1 wt% to 3 wt%;

the mass percentage of Triton X-100 in the Triton X-100 aqueous solution is in a range from 0.5% to 2%;

the penetration enhancer is sodium dodecyl sulfate, and the mass percentage of the penetration enhancer in the aqueous solution containing the penetration enhancer is in a range from 0.3% to 0.5%.

12. The method according to claim 10 or 11, adopting an approach selected from following approaches:

approach I: the first treatment liquids comprise a first running washing liquid and a second washing liquid, and comprise or without a surfactant solution; the second treatment liquid is an aqueous solution containing an acidic reagent; the third treatment liquid is an aqueous solution containing an alkaline reagent;

approach II: the first treatment liquids comprise a first running washing liquid and a second washing liquid, the second washing liquid is a starch aqueous solution; the second treatment liquid is an aqueous solution containing a penetration enhancer, or an aqueous solution containing an acidic regulator; the third treatment liquid is a Triton X-100 aqueous solution; and

approach III: the first treatment liquids comprise a first running washing liquid and a second washing liquid, the second washing liquid is a sodium carbonate aqueous solution; the second treatment liquid is an aqueous solution containing an acidic regulator; the third treatment liquid is a Triton X- 100 aqueous solution.

13. The method according to claim 10 or 11, satisfying one or more of following characteristics:

in soaking the degreased fish skin in the second treatment liquid, a soaking temperature is in a range from 0 °C to 60 °C;

the second treatment liquid is not replaced or replaced at least once, and each replacement cycle is in a range from 1 hour to 4 hours, optionally, from 2 hours to 3 hours; and

in soaking the degreased fish skin in the second treatment liquid, a total soaking duration is in a range from 2 hours to 8 hours, optionally from 2 hours to 4 hours.

14. The method according to claim 10 or 11, wherein the freeze-drying treatment is performed according to any one of following methods:

method F1: freeze-drying under vacuum at a temperature of -80 °C to -50 °C and a pressure of 15 Pa to 60 Pa for a freeze-drying period of 6 hours to 24 hours, and optionally pre-freezing at a temperature of -80 °C to -76 °C for a time period of 20 hours to 24 hours before freeze-drying under vacuum;

method F2: pre-freezing at a temperature of -80 °C to -76 °C for a time period of 20 hours to 24 hours, and drying at a temperature of 20° C to 30 °C and a pressure of 38 Pa to 42 Pa for a time period 45 hours to 50 hours.

15. The method according to claim 10 or 11, wherein after the decellularization treatment and before the freeze-drying treatment, the method further comprises:

wetting the decellularized fish skin matrix obtained from the decellularization treatment with a moisture-retaining agent; and

performing a microwave treatment on the wetted decellularized fish skin matrix to puff the decellularized fish skin matrix;

wherein the moisture-retaining agent is vegetable oil;

a power of the microwave treatment is in a range from 500 W to 900 W, optionally in a range from 700 W to 900 W;

a time period of the microwave treatment is in a range from 5 seconds to 60 seconds, optionally in a range from 30 seconds to 60 seconds.

16. The method according to claim 15, wherein before performing the decellularization treatment on the fish skin, the method further comprises: removing mucus and oil from the surfaces of the fish skin, and removing pigment cells and connective tissue from the inner and outer surfaces of the fish skin;

removing mucus and oil from the surfaces of the fish skin comprises: rinsing the fish skin in an alkali solution with a pH value of 8.4 to 11.5;

removing pigment cells and connective tissue from the inner and outer surfaces of the fish skin comprises: soaking the mucus and oil removed fish skin in an acid solution with a pH value of 2.0 to 4.5 at a temperature of 20°C to 37°C;

optionally, after the microwave treatment, the method further comprises freeze-drying the decellularized fish skin matrix, wherein the freeze-drying is performed at a temperature of -80 °C to -50 °C and a vacuum degree of 15 Pa to 60 Pa for a time period of 6 hours to 24 hours;

optionally, the fish skin is selected from scaleless fish skin, and the scaleless fish skin is selected from the group consisting of longsnout catfish skin, catfish skin, eel skin, and rice eel skin.

17. A method for preparing a single-layer biological patch, comprising:

performing an acid treatment on fish skin in an acidic solution, removing residual acidic solution, removing pigment to obtain a pigment-removed material, and washing the pigment-removed material to obtain a depigmented tissue material, wherein the fish skin has undergone a blood removal treatment and a degreasing treatment, and the acidic solution is an aqueous solution containing an acidic reagent;

performing an alkali treatment on the depigmented tissue material in an alkaline solution to obtain an alkali-treated material, and washing the alkali-treated material to obtain a decellularized tissue material, wherein the alkaline solution is an aqueous solution containing an alkaline reagent;

pre-freezing the decellularized tissue material and freeze-drying under vacuum to obtain the single-layer biological patch;

optionally, the method satisfies one or more of following characteristics:

the acidic reagent is selected from the group consisting of acetic acid, citric acid, hydrochloric acid, formic acid, sulfuric acid, nitric acid, and any combinations thereof;

a concentration of the acidic reagent in the acidic solution is in a range from 1 mmol/L to 500 mmol/L;

a pH value of the acidic solution is in a range from 1 to 5;

the acid treatment is performed for 1 minute to 60 minutes;

the pigment is removed by scraping, stirring, shaking, or any combinations thereof; and

the pigment-removed material is washed with water under shaking, wherein for every 1 g of the pigment-removed material, a volume of water used is in a range from 10 mL to 30 mL; the washing is performed once or multiple times, with each washing lasting 5 minutes to 15 minutes;

optionally, the method satisfies one or more of following characteristics:

the alkaline reagent is selected from the group consisting of sodium carbonate, sodium bicarbonate, trisodium phosphate, sodium monohydrogen phosphate, disodium hydrogen phosphate, sodium hydroxide, potassium carbonate, potassium bicarbonate, tripotassium phosphate, potassium monohydrogen phosphate, dipotassium hydrogen phosphate, potassium hydroxide; and any combinations thereof;

a concentration of the alkaline reagent in the alkaline solution is in a range from 0.01 mol/L to 2 mol/L;

a pH value of the alkaline solution is in a range from 9 to 13;

the alkali treatment is performed at a temperature in a range from 20°C to 30°C under shaking at a speed in a range from 100 rpm to 200 rpm;

the alkali treatment is performed for 6 hours to 30 hours; and

the alkali-treated material is washed with water under shaking, wherein for every 1 g of the alkali-treated material, a volume of water used is in a range from 10 mL to 50 mL; the washing is performed once or multiple times, with each washing lasting 1 minute to 12 minutes;
optionally, the method satisfies one or more of following characteristics:

the pre-freezing is performed at a temperature in a range from -80 °C to -76 °C;
a time period of the pre-freezing is in a range from 20 hours to 24 hours;
the freeze-drying is performed at a temperature in a range from 20 °C to 30 °C;
the freeze-drying is performed at a pressure in a range from 38 Pa to 42 Pa; and
a time period of the freeze-drying is in a range from 45 hours to 50 hours.

18. A fish skin-based biological patch comprising a sheet-shaped body, wherein the sheet-shaped body comprises a collagen matrix; the fish skin-based biological patch is prepared from fish skin through treatments comprising depigmentation and decellularization; the fish skin-based biological patch has a degradation period in a range from 7 days to 180 days, a fat content in a range from 1% to 19%, and a residual DNA content less than 50 ng/mg; optionally, the fish skin-based biological patch is prepared by a method comprising:

performing a degreasing pretreatment on the fish skin to prepare pretreated fish skin;
placing the pretreated fish skin in an aqueous solution, performing a depigmentation treatment, and optionally a partial decellularization treatment, on the pretreated fish skin to obtain a depigmented fish skin;
performing a decellularization treatment on the depigmented fish skin to obtain the fish skin-based biological patch;
wherein in the depigmentation treatment, the aqueous solution is replaced every 1 hours to 4 hours, and optionally 2 hours to 3 hours;
optionally, the depigmentation treatment satisfies at least one of following characteristics:

the depigmentation treatment is performed at a temperature in a range from 0 °C to 60 °C,
the aqueous solution further comprises one or more additives selected from a penetration enhancer and an acid or alkali regulator; the penetration enhancer comprises sodium dodecyl sulfate, and a mass percentage of the penetration enhancer in the aqueous solution is in a range from 0.3% to 0.5%; the acid or alkali regulator comprises an acidic regulator, and the acidic regulator comprises citric acid, acetic acid, or a combination thereof; and
performing the decellularization treatment on the depigmented fish skin comprises: decellularizing the depigmented fish skin with a Triton solution containing 0.1% to 10% by mass of Triton X-100.

19. Use of the biological patch according to any one of claims 1 to 9, or the pbiological patch prepared by the method according to any one of claims 10 to 16, or the single-layer biological patch prepared by the method according to claim 17, or the fish skin-based biological patch according to claim 18 in preparation of a soft tissue repair implant material or skin repair dressing;
optionally, the soft tissue repair for which the soft tissue repair implant material is applicable comprises one or more selected from the group consisting of dura mater defect repair, sports tendon tear repair, hernia and abdominal wall defect repair, burn plastic surgery repair, and oral membrane defect repair.

FIG. 1

FIG. 2

FIG. 3

FIG. 4

FIG. 5

HE staining
Week 2

Comparative Example 4

Comparative Example 5

Example 5

FIG. 6

## INTERNATIONAL SEARCH REPORT

| International application No. |
|---|
| **PCT/CN2023/132986** |

### A. CLASSIFICATION OF SUBJECT MATTER

A61L27/36(2006.01)i; A61L15/40(2006.01)i; A61L17/08(2006.01)i; A61L26/00(2006.01)i; A61L31/00(2006.01)i; A61L27/58(2006.01)i; A61L27/60(2006.01)i; A61L15/42(2006.01)i; A61L27/56(2006.01)i; A61L27/50(2006.01)i

According to International Patent Classification (IPC) or to both national classification and IPC

### B. FIELDS SEARCHED

Minimum documentation searched (classification system followed by classification symbols)

IPC: A61

Documentation searched other than minimum documentation to the extent that such documents are included in the fields searched

Electronic data base consulted during the international search (name of data base and, where practicable, search terms used)

CNTXT, ENTXTC, USTXT, JPTXT, WPABS, ENTXT, DWPI, patentics, 中国期刊网全文数据库, CJFD, ISI WEB OF SCIENCE: 申请人, 发明人, 脱细胞基质, ECM, 补片, 软组织, 支架, 鱼皮, 脂肪酸, omega-3, omega-6, 胶原, 纤维, 去脂, 脱脂, 碳酸钠, 淀粉, 去细胞, 脱细胞, 曲拉通, triton, 碱, 色素, 脱色, 十二烷基硫酸钠, SDS, 酸, 微波, decellularized matrix, patch, soft tissue, scaffold, stent, fish skin, fatty acids, omega-3, omega-6, collagen, fiber, degreas+, defat+, sodium carbonate, starch, decellula+, triton, base, pigment, decolor+, sodium dodecyl sulfate, SDS, acid, microwave

### C. DOCUMENTS CONSIDERED TO BE RELEVANT

| Category* | Citation of document, with indication, where appropriate, of the relevant passages | Relevant to claim No. |
|---|---|---|
| X | CN 102781485 A (KERECIS EHF) 14 November 2012 (2012-11-14) description, paragraphs 8-14, 27-31, and 35-36 | 1-9, 18-19 |
| X | CN 106075579 A (BODY ORGAN BIOMEDICAL CORP.) 09 November 2016 (2016-11-09) description, paragraphs 45, 52-54, and 102 | 17, 19 |
| PX | CN 116650723 A (SUZHOU MICROPORT REGENERATIVE MEDICINE TECHNOLOGY CO., LTD.) 29 August 2023 (2023-08-29) claims 1-11 | 18 |
| X | WO 2013144727 A2 (KERECIS EHF) 03 October 2013 (2013-10-03) description, page 2, line 22-page 4, line 19, and page 8, line 30-page 11, line 5 | 1-9, 18-19 |
| A | CN 102781485 A (KERECIS EHF) 14 November 2012 (2012-11-14) description, paragraphs 8-14, 27-31, and 35-36 | 10-17 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **26 January 2024** | **06 February 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 609 885 A1**

## INTERNATIONAL SEARCH REPORT

| International application No. |
| --- |
| **PCT/CN2023/132986** |

### C. DOCUMENTS CONSIDERED TO BE RELEVANT

| Category* | Citation of document, with indication, where appropriate, of the relevant passages | Relevant to claim No. |
| --- | --- | --- |
| A | CN 106075579 A (BODY ORGAN BIOMEDICAL CORP.) 09 November 2016 (2016-11-09) description, paragraphs 45, 52-54, and 102 | 1-16, 18 |
| A | WO 2013144727 A2 (KERECIS EHF) 03 October 2013 (2013-10-03) description, page 2, line 22-page 4, line 19, and page 8, line 30-page 11, line 5 | 10-17 |
| A | CN 108187140 A (OCEAN UNIVERSITY OF CHINA) 22 June 2018 (2018-06-22) entire document | 1-19 |
| A | CN 111714707 A (SHAANXI BIO REGENERATION MEDICINE CO., LTD.) 29 September 2020 (2020-09-29) entire document | 1-19 |
| A | CN 113476667 A (MARINE BIOMEDICAL RESEARCH INSTITUTE OF QINGDAO et al.) 08 October 2021 (2021-10-08) entire document | 1-19 |
| A | CN 113827774 A (SHENYANG JINGYING WEILAN MEDICAL TECHNOLOGY CO., LTD.) 24 December 2021 (2021-12-24) entire document | 1-19 |
| A | US 2015037436 A1 (MUSCULOSKELETAL TRANSPLANT FOUNDATION) 05 February 2015 (2015-02-05) entire document | 1-19 |

Form PCT/ISA/210 (second sheet) (July 2022)

**INTERNATIONAL SEARCH REPORT**
Information on patent family members

International application No.

**PCT/CN2023/132986**

| Patent document cited in search report | | | Publication date (day/month/year) | Patent family member(s) | | | Publication date (day/month/year) |
|---|---|---|---|---|---|---|---|
| CN | 102781485 | A | 14 November 2012 | JP | 2013507164 | A | 04 March 2013 |
| | | | | JP | 5893563 | B2 | 23 March 2016 |
| | | | | US | 2011244054 | A1 | 06 October 2011 |
| | | | | US | 8613957 | B2 | 24 December 2013 |
| | | | | EP | 2485779 | A2 | 15 August 2012 |
| | | | | EP | 2485779 | B1 | 21 February 2018 |
| | | | | KR | 20120134096 | A | 11 December 2012 |
| | | | | KR | 101755740 | B1 | 07 July 2017 |
| | | | | WO | 2011042794 | A2 | 14 April 2011 |
| CN | 106075579 | A | 09 November 2016 | JP | 2016193891 | A | 17 November 2016 |
| | | | | JP | 6190911 | B2 | 30 August 2017 |
| | | | | US | 2019070224 | A1 | 07 March 2019 |
| | | | | US | 10695381 | B2 | 30 June 2020 |
| | | | | EP | 3075399 | A1 | 05 October 2016 |
| | | | | EP | 3075399 | B1 | 16 October 2019 |
| | | | | KR | 20160117341 | A | 10 October 2016 |
| | | | | KR | 101822266 | B1 | 26 January 2018 |
| | | | | KR | 20180000708 | A | 03 January 2018 |
| | | | | KR | 101885487 | B1 | 10 September 2018 |
| | | | | US | 2016287643 | A1 | 06 October 2016 |
| | | | | US | 10172891 | B2 | 08 January 2019 |
| | | | | CN | 106075579 | B | 25 October 2019 |
| CN | 116650723 | A | 29 August 2023 | None | | | |
| WO | 2013144727 | A2 | 03 October 2013 | None | | | |
| CN | 108187140 | A | 22 June 2018 | None | | | |
| CN | 111714707 | A | 29 September 2020 | None | | | |
| CN | 113476667 | A | 08 October 2021 | None | | | |
| CN | 113827774 | A | 24 December 2021 | None | | | |
| US | 2015037436 | A1 | 05 February 2015 | US | 2019008903 | A1 | 10 January 2019 |
| | | | | US | 10596201 | B2 | 24 March 2020 |
| | | | | WO | 2015017500 | A1 | 05 February 2015 |
| | | | | US | 2017119826 | A1 | 04 May 2017 |
| | | | | US | 10092600 | B2 | 09 October 2018 |
| | | | | US | 2023270794 | A1 | 31 August 2023 |
| | | | | EP | 3027235 | A1 | 08 June 2016 |
| | | | | US | 2022079994 | A1 | 17 March 2022 |
| | | | | US | 11779610 | B2 | 10 October 2023 |
| | | | | US | 2020171094 | A1 | 04 June 2020 |
| | | | | US | 11191788 | B2 | 07 December 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2022114640402
- CN 2022115709087
- CN 2023108267378

### Non-patent literature cited in the description

- *CHEMICAL ABSTRACTS*, 623-42-7
- *CHEMICAL ABSTRACTS*, 106-70-7
- *CHEMICAL ABSTRACTS*, 111-11-5
- *CHEMICAL ABSTRACTS*, 110-42-9
- *CHEMICAL ABSTRACTS*, 1731-86-8
- *CHEMICAL ABSTRACTS*, 111-82-0
- *CHEMICAL ABSTRACTS*, 1731-88-0
- *CHEMICAL ABSTRACTS*, 124-10-7
- *CHEMICAL ABSTRACTS*, 56219-06-8
- *CHEMICAL ABSTRACTS*, 7132-64-1
- *CHEMICAL ABSTRACTS*, 90176-52-6
- *CHEMICAL ABSTRACTS*, 112-39-0
- *CHEMICAL ABSTRACTS*, 1120-25-8
- *CHEMICAL ABSTRACTS*, 1731-92-6
- *CHEMICAL ABSTRACTS*, 75190-82-8
- *CHEMICAL ABSTRACTS*, 112-61-8
- *CHEMICAL ABSTRACTS*, 112-62-9
- *CHEMICAL ABSTRACTS*, 2566-97-4
- *CHEMICAL ABSTRACTS*, 112-63-0
- *CHEMICAL ABSTRACTS*, 1120-28-1
- *CHEMICAL ABSTRACTS*, 16326-32-2
- *CHEMICAL ABSTRACTS*, 301-00-8
- *CHEMICAL ABSTRACTS*, 6064-90-0
- *CHEMICAL ABSTRACTS*, 2463-02-7
- *CHEMICAL ABSTRACTS*, 929-77-1
- *CHEMICAL ABSTRACTS*, 21061-10-9
- *CHEMICAL ABSTRACTS*, 1120-34-9
- *CHEMICAL ABSTRACTS*, 55682-88-7
- *CHEMICAL ABSTRACTS*, 2433-97-8
- *CHEMICAL ABSTRACTS*, 2566-89-4
- *CHEMICAL ABSTRACTS*, 61012-47-3
- *CHEMICAL ABSTRACTS*, 2442-49-1
- *CHEMICAL ABSTRACTS*, 2734-47-6
- *CHEMICAL ABSTRACTS*, 2733-88-2
- *CHEMICAL ABSTRACTS*, 301-01-9